# EUROPEAN PATENT SPECIFICATION

(11) **EP 1 940 466 B1**
(45) Date of publication and mention of the grant of the patent: **28.11.2012**
(21) Application number: 06836346.4
(22) Date of filing: 17.10.2006
(51) Int. Cl.: A61K 39/395

(54) **ANTI-ADDL MONOCLONAL ANTIBODIES AND USE THEREOF**
MONOKLONALE ANTIKÖRPER GEGEN ADDL UND ANWENDUNG DAVON
ANTICORPS MONOCLONAUX ANTI-ADDL ET LEUR UTILISATION

(30) Priority: 21.10.2005 US 38125
(43) Date of publication of application: 09.07.2008
(73) Proprietor: Merck Sharp & Dohme Corp., Rahway, NJ 07065-0907 (US)
(72) Inventor: KINNEY, Gene, Collegeville, PA 19426 (US); STROHL, William, R., Bridgewater, NJ 08807 (US); AN, Zhiqiang, Ambler, PA 19002 (US)
(74) Representative: O'Connell, Maura
(86) International application number: PCT/US2006/040508
(87) International publication number: WO 2007/050359

(56) References cited:
- WO-A1-98/44955
- WO-A2-03/104437
- WO-A2-2004/031400
- WO-A2-2004/108895
- WO-A2-2006/055178
- US-A- 5 750 349
- US-A- 5 786 180
- HAES ET AL: "Detection of a Biomarker for Alzheimer's Disease from synthetic anc clinical samples using a nanoscale optical biosensor." JOURNAL OF THE AMERICAN CHEMICAL SOCIETY 23 FEB 2005, vol. 127, no. 7, 23 February 2005 (2005-02-23), pages 2264-2271, XP002568084 ISSN: 0002-7863
- BOUTAUD OLIVIER ET AL: "PGH2-derived levuglandin adducts increase the neurotoxicity of amyloid beta1-42." JOURNAL OF NEUROCHEMISTRY FEB 2006, vol. 96, no. 4, February 2006 (2006-02), pages 917-923, XP002568085 ISSN: 0022-3042
- FRENKEL ET AL.: 'N-terminal EFRH sequence of Alzheimer's beta-amyloid peptide represents the epitope of its anti-aggregating antibodies' J. NEUROIMMUNOL. vol. 88, 1998, pages 85 - 90, XP001001343

### SEQUENCE LISTING

<110> Kinney, Gene Strohl, William R. An, Zhiqiang
<120> ANTI-ADDL MONOCLONAL ANTIBODY AND USE THEREOF
<130> MRK0003WO
<150> PCT/US2005/038125
   <151> 2005-10-21
<160> 153
<170> Patent In version 3.3
<210> 1
   <211> 6
   <212> PRT
   <213> Homo sapiens
<400> 1
   Glu Phe Arg His Asp Ser
   1               5
<210> 2
   <211> 9
   <212> PRT
   <213> Artificial Sequence
<220>
   <223> Synthetic CDR3 peptide
<220>
   <221> MISC_FEATURE
   <222> (1)..(1)
   <223> Xaa denotes Phe or Leu
<220>
   <221> MISC_FEATURE
   <222> (3)..(3)
   <223> Xaa denotes Ala or Thr
<400> 2
   Xaa Gln Xaa Thr Arg Val Pro Leu Thr
   1               5
<210> 3
   <211> 13
   <212> PRT
   <213> Artificial Sequence
<220>
   <223> Synthetic CDR3 peptide
<400> 3
   Arg Gln Leu Gly Thr Arg Gly Thr Asp Ala Met Asp Tyr
   1 5 10
<210> 4
   <211> 13
   <212> PRT
   <213> Artificial Sequence
<220>
   <223> Synthetic CDR3 peptide
<400> 4
   Arg Ala Leu Ser Pro Arg Ser Ile Asp Ala Met Asp Tyr
   1 5 10
<210> 5
   <211> 13
   <212> PRT
   <213> Artificial Sequence
<220>
   <223> Synthetic CDR3 peptide
<400> 5
   Arg Gln Leu Gly Ala Arg Lys Thr Asp Ala Met Asp Tyr
   1 5 10
<210> 6
   <211> 13
   <212> PRT
   <213> Artificial Sequence
<220>
   <223> Synthetic CDR3 peptide
<400> 6
   Arg Gln Leu Gly Pro Arg Lys Arg Asp Ala Met Asp Tyr
   1 5 10
<210> 7
   <211> 13
   <212> PRT
   <213> Artificial Sequence
<220>
   <223> Synthetic CDR3 peptide
<400> 7
   Arg Gln Leu Gly Lys Leu Lys Thr Asp Ala Met Asp Tyr
   1 5 10
<210> 8
   <211> 13
   <212> PRT
   <213> Artificial Sequence
<220>
   <223> Synthetic CDR3 peptide
<400> 8
   Arg Gln Leu Gly Arg Arg Ser Val Asp Ala Met Asp Tyr
   1 5 10
<210> 9
   <211> 13
   <212> PRT
   <213> Artificial Sequence
<220>
   <223> Synthetic CDR3 peptide
<220>
   <221> MISC_FEATURE
   <222> (2)..(2)
   <223> Xaa denotes Gln or Ala
<220>
   <221> MISC_FEATURE
   <222> (4)..(4)
   <223> Xaa denotes Ser or Gly
<220>
   <221> MISC_FEATURE
   <222> (5)..(5)
   <223> Xaa denotes Pro, Ala, Lys, Arg, or Thr
<220>
   <221> MISC_FEATURE
   <222> (6)..(6)
   <223> Xaa denotes Lys or Arg
<220>
   <221> MISC_FEATURE
   <222> (7)..(7)
   <223> Xaa denotes Gly, Ser, or Lys
<220>
   <221> MISC_FEATURE
   <222> (8)..(8)
   <223> Xaa denotes Val, Thr, Ile or Arg
<400> 9
   Arg Xaa Leu Xaa Xaa Xaa Xaa Xaa Asp Ala Met Asp Tyr
   1 5 10
<210> 10
   <211> 10
   <212> PRT
   <213> Artificial Sequence
<220>
   <223> Synthetic peptide
<400> 10
   Leu Pro Val Thr Pro Gly Glu Pro Ala Ser
   1               5                   10
<210> 11
   <211> 40
   <212> DNA
   <213> Artificial Sequence
<220>
   <223> Synthetic oligonucleotide
<400> 11
   gatctctaga tgaagattgc ctgttaggct gttggtgctg 40
<210> 12
   <211> 39
   <212> DNA
   <213> Artificial Sequence
<220>
   <223> Synthetic oligonucleotide
<400> 12
   gatctctaga tggagwcaga cacactcctg ytatgggtg 39
<210> 13
   <211> 39
   <212> DNA
   <213> Artificial Sequence
<220>
   <223> Synthetic oligonucleotide
<400> 13
   gatctctaga tgagtgtgct cactcaggtc ctggsgttg 39
<210> 14
   <211> 42
   <212> DNA
<213> Artificial Sequence
<220>
   <223> Synthetic oligonucleotide
<400> 14
   gatctctaga tgaggrcccc tgctcagwtt yttggmwtct tg 42
<210> 15
   <211> 39
   <212> DNA
   <213> Artificial Sequence
<220>
   <223> Synthetic oligonucleotide
<400> 15
   gatctctaga tggatttwca ggtgcagatt wtcagcttc 39
<210> 16
   <211> 38
   <212> DNA
   <213> Artificial Sequence
<220>
   <223> Synthetic oligonucleotide
<400> 16
   gatctctaga tgaggtkcyy tgytsaycty ctctgrgg 38
<210> 17
   <211> 41
   <212> DNA
   <213> Artificial Sequence
<220>
   <223> Synthetic oligonucleotide
<400> 17
   gatctctaga tgggcwtcaa agatggagtc acakwyycwg g 41
<210> 18
   <211> 39
   <212> DNA
   <213> Artificial Sequence
<220>
   <223> Synthetic oligonucleotide
<400> 18
   gatctctaga tgtggggayc tktttycmmt ttttcaatg 39
<210> 19
   <211> 34
   <212> DNA
   <213> Artificial Sequence
<220>
   <223> Synthetic oligonucleotide
<400> 19
   gatctctaga tggtrtccwc asctcagttc cttg 34
<210> 20
   <211> 36
   <212> DNA
   <213> Artificial Sequence
<220>
   <223> Synthetic oligonucleotide
<400> 20
   gatctctaga tgtatatatg tttgttgtct atttct 36
<210> 21
   <211> 37
   <212> DNA
   <213> Artificial Sequence
<220>
   <223> Synthetic oligonucleotide
<400> 21
   gatctctaga tggaagcccc agctcagctt ctcttcc 37
<210> 22
   <211> 30
   <212> DNA
   <213> Artificial Sequence
<220>
   <223> Synthetic oligonucleotide
<400> 22
   gatcgagctc actggatggt gggaagatgg 30
<210> 23
   <211> 36
   <212> DNA
   <213> Artificial Sequence
<220>
   <223> Synthetic oligonucleotide
<400> 23
   gatctctaga tgaaatgcag ctggggcats ttcttc 36
<210> 24
   <211> 35
   <212> DNA
   <213> Artificial Sequence
<220>
   <223> Synthetic oligonucleotide
<400> 24
   gatctctaga tgggatggag ctrtatcats ytctt 35
<210> 25
   <211> 36
   <212> DNA
   <213> Artificial Sequence
<220>
   <223> Synthetic oligonucleotide
<400> 25
   gatctctaga tgaagwtgtg gttaaactgg gttttt 36
<210> 26
   <211> 34
   <212> DNA
   <213> Artificial Sequence
<220>
   <223> Synthetic oligonucleotide
<400> 26
   gatctctaga tgractttgg gytcagcttg rttt 34
<210> 27
   <211> 41
   <212> DNA
   <213> Artificial Sequence
<220>
   <223> Synthetic oligonucleotide
<400> 27
   gatctctaga tgggactcca ggcttcaatt tagttttcct t 41
<210> 28
   <211> 38
   <212> DNA
   <213> Artificial Sequence
<220>
   <223> Synthetic oligonucleotide
<400> 28
   gatctctaga tggcttgtcy ttrgsgctrc tcttctgc 38

<210> 29
   <211> 36
   <212> DNA
   <213> Artificial Sequence
<220>
   <223> Synthetic oligonucleotide
<400> 29
   gatctctaga tggratggag ckggrgtctt tmtctt 36
<210> 30
   <211> 32
   <212> DNA
   <213> Artificial Sequence
<220>
   <223> Synthetic oligonucleotide
<400> 30
   gatctctaga tgagagtgct gattcttttg tg 32
<210> 31
   <211> 40
   <212> DNA
   <213> Artificial Sequence
<220>
   <223> Synthetic oligonucleotide
<400> 31
   gatctctaga tggmttgggt gtggamcttg cttattcctg 40
<210> 32
   <211> 37
   <212> DNA
   <213> Artificial Sequence
<220>
   <223> Synthetic oligonucleotide
<400> 32
   gatctctaga tgggcagact taccattctc attcctg 37
<210> 33
   <211> 37
   <212> DNA
   <213> Artificial Sequence
<220>
   <223> Synthetic oligonucleotide
<400> 33
   gatctctaga tggattttgg gctgattttt tttattg 37
<210> 34
   <211> 36
   <212> DNA
   <213> Artificial Sequence
<220>
   <223> Synthetic oligonucleotide
<400> 34
   gatctctaga tgatggtgtt aagtcttctg tacctg 36
<210> 35
   <211> 32
   <212> DNA
   <213> Artificial Sequence
<220>
   <223> Synthetic oligonucleotide
<400> 35
   gcatcgagct ccagtggata gacagatggg gg 32
<210> 36
   <211> 32
   <212> DNA
   <213> Artificial Sequence
<220>
   <223> Synthetic oligonucleotide
<400> 36
   gcatcgagct ccagtggata gaccgatggg gg 32
<210> 37
   <211> 32
   <212> DNA
   <213> Artificial Sequence
<220>
   <223> Synthetic oligonucleotide
<400> 37
   gcatcgagct ccagtggatg agctgatggg gg 32
<210> 38
   <211> 32
   <212> DNA
   <213> Artificial Sequence
<220>
   <223> Synthetic oligonucleotide
<400> 38
   gcatcgagct ccaagggata gacagatggg gc 32
<210> 39
   <211> 9
   <212> PRT
   <213> Artificial Sequence
<220>
   <223> Synthetic peptide
<400> 39
   Phe Gln Gly Ser Leu Val Pro Leu Thr
   1               5
<210> 40
   <211> 9
   <212> PRT
   <213> Artificial Sequence
<220>
   <223> Synthetic peptide
<220>
   <221> MISC_FEATURE
   <222> (1)..(5)
   <223> Xaa denotes any amino acid residue
<400> 40
   Xaa Xaa Xaa Xaa Xaa Val Pro Leu Thr
   1 5
<210> 41
   <211> 9
   <212> PRT
   <213> Artificial Sequence
<220>
   <223> Synthetic peptide
<220>
   <221> MISC_FEATURE
   <222> (5)..(9)
   <223> Xaa denotes any amino acid residue
<400> 41
   Phe Gln Gly Ser Xaa Xaa Xaa Xaa Xaa
   1 5
<210> 42
   <211> 9
   <212> PRT
<213> Artificial Sequence
<220>
   <223> Synthetic peptide
<400> 42
   Ala Asp Thr Thr His Val Pro Leu Thr
   1 5
<210> 43
   <211> 9
   <212> PRT
   <213> Artificial Sequence
<220>
   <223> Synthetic peptide
<400> 43
   Ala His Ser Thr Phe Val Pro Leu Thr
   1 5
<210> 44
   <211> 9
   <212> PRT
   <213> Artificial Sequence
<220>
   <223> Synthetic peptide
<400> 44
   Ala Gln Ala Ser Phe Val Pro Leu Thr
   1 5
<210> 45
   <211> 9
   <212> PRT
   <213> Artificial Sequence
<220>
   <223> Synthetic peptide
<400> 45
   Ala Gln Ala Thr Lys Val Pro Leu Thr
   1 5
<210> 46
   <211> 9
   <212> PRT
   <213> Artificial Sequence
<220>
   <223> Synthetic peptide
<400> 46
   Ala Gln Ser Ser Lys Val Pro Leu Thr
   1 5
<210> 47
   <211> 9
   <212> PRT
   <213> Artificial Sequence
<220>
   <223> Synthetic peptide
<400> 47
   Ala Gln Ser Thr Leu Val Pro Leu Thr
   1 5
<210> 48
   <211> 9
   <212> PRT
   <213> Artificial Sequence
<220>
   <223> Synthetic peptide
<400> 48
   Phe Ala Ala Ser Ser Val Pro Leu Thr
   1 5
<210> 49
   <211> 9
   <212> PRT
   <213> Artificial Sequence
<220>
   <223> Synthetic peptide
<400> 49
   Phe Glu Ser Thr Tyr Val Pro Leu Thr
   1 5
<210> 50
   <211> 9
   <212> PRT
   <213> Artificial Sequence
<220>
   <223> Synthetic peptide
<400> 50
   Phe Glu Ser Ser Arg Val Pro Leu Thr
   1 5
<210> 51
   <211> 9
   <212> PRT
   <213> Artificial Sequence
<220>
   <223> Synthetic peptide
<400> 51
   Phe Asn Ala Thr Trp Val Pro Leu Thr
   1 5
<210> 52
   <211> 9
   <212> PRT
   <213> Artificial Sequence
<220>
   <223> Synthetic peptide
<400> 52
   Phe Gln Ala Ser Arg Val Pro Leu Thr
   1 5
<210> 53
   <211> 9
   <212> PRT
   <213> Artificial Sequence
<220>
   <223> Synthetic peptide
<400> 53
   Phe Gln Ala Thr Arg Val Pro Leu Thr
   1               5
<210> 54
   <211> 9
   <212> PRT
   <213> Artificial Sequence
<220>
   <223> Synthetic peptide
<400> 54
   Phe Gln Gly Ser Phe Ile Gly Leu Ser
   1               5

<210> 55
   <211> 9
   <212> PRT
   <213> Artificial Sequence
<220>
   <223> Synthetic peptide
<400> 55
   Phe Gln Gly Ser Phe Ile Pro Gly Thr
   1               5
<210> 56
   <211> 9
   <212> PRT
   <213> Artificial Sequence
<220>
   <223> Synthetic peptide
<400> 56
   Phe Gln Gly Ser Phe Leu Pro Pro Ser
   1               5
<210> 57
   <211> 9
   <212> PRT
   <213> Artificial Sequence
<220>
   <223> Synthetic peptide
<400> 57
   Phe Gln Gly Ser Phe Leu Pro Gln Leu
   1 5
<210> 58
   <211> 9
   <212> PRT
   <213> Artificial Sequence
<220>
   <223> Synthetic peptide
<400> 58
   Phe Gln Gly Ser Leu Phe Pro Pro Val
   1 5
<210> 59
   <211> 9
   <212> PRT
   <213> Artificial Sequence
<220>
   <223> Synthetic peptide
<400> 59
   Phe Gln Gly Ser Leu Phe Ser Pro Ser
   1 5
<210> 60
   <211> 9
   <212> PRT
   <213> Artificial Sequence
<220>
   <223> Synthetic peptide
<400> 60
   Phe Gln Gly Ser Arg Ile Pro Ile Ser
   1 5
<210> 61
   <211> 9
   <212> PRT
   <213> Artificial Sequence
<220>
   <223> Synthetic peptide
<400> 61
   Phe Gln Gly Ser Arg Leu Pro Val Ser
   1 5
<210> 62
   <211> 9
   <212> PRT
   <213> Artificial Sequence
<220>
   <223> Synthetic peptide
<400> 62
   Phe Gln Gly Ser Arg Val Pro Leu Val
   1               5
<210> 63
   <211> 9
   <212> PRT
   <213> Artificial Sequence
<220>
   <223> Synthetic peptide
<400> 63
   Phe Gln Ser Ser Phe Val Pro Leu Thr
   1               5
<210> 64
   <211> 9
   <212> PRT
   <213> Artificial Sequence
<220>
   <223> Synthetic peptide
<400> 64
   Phe Gln Ser Ser Arg Val Pro Leu Thr
   1               5
<210> 65
   <211> 9
   <212> PRT
   <213> Artificial Sequence
<220>
   <223> Synthetic peptide
<400> 65
   Gly Gln Thr Thr Leu Val Pro Leu Thr
   1               5
<210> 66
   <211> 9
   <212> PRT
   <213> Artificial Sequence
<220>
   <223> Synthetic peptide
<400> 66
   His Glu Ser Thr Leu Val Pro Leu Thr
   1 5
<210> 67
   <211> 9
   <212> PRT
   <213> Artificial Sequence
<220>
   <223> Synthetic peptide

<400> 67
   His Gln Ser Ser Lys Val Pro Leu Thr
   1 5
<210> 68
   <211> 9
   <212> PRT
   <213> Artificial Sequence
<220>
   <223> Synthetic peptide
<400> 68
   Ile Gln Thr Ser Leu Val Pro Leu Thr
   1 5
<210> 69
   <211> 9
   <212> PRT
   <213> Artificial Sequence
<220>
   <223> Synthetic peptide
<400> 69
   Ile Gln Ala Ala Leu Val Pro Leu Thr
   1 5
<210> 70
   <211> 9
   <212> PRT
   <213> Artificial Sequence
<220>
   <223> Synthetic peptide
<400> 70
   Leu Gln Ser Ser Phe Val Pro Leu Thr
   1 5
<210> 71
   <211> 9
   <212> PRT
   <213> Artificial Sequence
<220>
   <223> Synthetic peptide
<400> 71
   Leu Glu Thr Ser Arg Val Pro Leu Thr
   1 5
<210> 72
   <211> 9
   <212> PRT
   <213> Artificial Sequence
<220>
   <223> Synthetic peptide
<400> 72
   Leu Ala Ser Ser His Val Pro Leu Thr
   1 5
<210> 73
   <211> 9
   <212> PRT
   <213> Artificial Sequence
<220>
   <223> Synthetic peptide
<400> 73
   Leu Asn Ser Thr Thr Val Pro Leu Thr
   1 5
<210> 74
   <211> 9
   <212> PRT
   <213> Artificial Sequence
<220>
   <223> Synthetic peptide
<400> 74
   Leu Gln Ser Lys Ser Val Pro Leu Thr
   1 5
<210> 75
   <211> 9
   <212> PRT
   <213> Artificial Sequence
<220>
   <223> Synthetic peptide
<400> 75
   Leu Gln Ser Val Arg Val Pro Leu Thr
   1 5
<210> 76
   <211> 9
   <212> PRT
   <213> Artificial sequence
<220>
   <223> Synthetic peptide
<400> 76
   Leu Gln Ser Ser Leu Val Pro Leu Thr
   1 5
<210> 77
   <211> 9
   <212> PRT
   <213> Artificial Sequence
<220>
   <223> Synthetic peptide
<400> 77
   Leu Gln Thr Gly Arg Val Pro Leu Thr
   1 5
<210> 78
   <211> 9
   <212> PRT
   <213> Artificial Sequence
<220>
   <223> Synthetic peptide
<400> 78
   Leu Gln Thr Ser Phe Val Pro Leu Thr
   1 5
<210> 79
   <211> 9
   <212> PRT
   <213> Artificial Sequence
<220>
   <223> Synthetic peptide
<400> 79
   Leu Gln Thr Ser Asn Val Pro Leu Thr
   1 5
<210> 80
   <211> 9
<212> PRT
   <213> Artificial Sequence
<220>
   <223> Synthetic peptide
<400> 80
   Leu Gln Thr Thr Arg Val Pro Leu Thr
   1 5
<210> 81
   <211> 9
   <212> PRT
   <213> Artificial Sequence
<220>
   <223> Synthetic peptide
<400> 81
   Leu Ser Ser Thr Phe Val Pro Leu Thr
   1 5
<210> 82
   <211> 9
   <212> PRT
   <213> Artificial Sequence
<220>
   <223> Synthetic peptide
<400> 82
   Leu Ser Ser Thr His Val Pro Leu Thr
   1 5
<210> 83
   <211> 9
   <212> PRT
   <213> Artificial Sequence
<220>
   <223> Synthetic peptide
<400> 83
   Leu Thr Ser Ser Ala Val Pro Leu Thr
   1 5
<210> 84
   <211> 9
   <212> PRT
   <213> Artificial Sequence
<220>
   <223> Synthetic peptide
<400> 84
   Leu Val Ser Ser Leu Val Pro Leu Thr
   1 5
<210> 85
   <211> 9
   <212> PRT
   <213> Artificial Sequence
<220>
   <223> Synthetic peptide
<400> 85
   Met Glu Thr Ala Asn Val Pro Leu Thr
   1 5
<210> 86
   <211> 9
   <212> PRT
   <213> Artificial Sequence
<220>
   <223> Synthetic peptide
<400> 86
   Met Gln Ser Ser Phe Val Pro Leu Thr
   1 5
<210> 87
   <211> 9
   <212> PRT
   <213> Artificial Sequence
<220>
   <223> Synthetic peptide
<400> 87
   Met Gln Ser Ser Leu Val Pro Leu Thr
   1 5
<210> 88
   <211> 9
   <212> PRT
   <213> Artificial Sequence
<220>
   <223> Synthetic peptide
<400> 88
   Met Gln Thr Ser Lys Val Pro Leu Thr
   1               5
<210> 89
   <211> 9
   <212> PRT
   <213> Artificial Sequence
<220>
   <223> Synthetic peptide
<400> 89
   Ser Gln Ala Arg Met Val Pro Leu Thr
   1               5
<210> 90
   <211> 9
   <212> PRT
   <213> Artificial Sequence
<220>
   <223> Synthetic peptide
<400> 90
   Ser Gln Ala Ser Arg Val Pro Leu Thr
   1               5
<210> 91
   <211> 9
   <212> PRT
   <213> Artificial Sequence
<220>
   <223> Synthetic peptide
<400> 91
   Thr Gln Ser Thr Gln Val Pro Leu Thr
   1               5
<210> 92
   <211> 9
   <212> PRT
   <213> Artificial Sequence
<220>
   <223> Synthetic peptide
<400> 92
   Val Cys Ala Thr Phe Val Pro Leu Thr
   1 5

<210> 93
   <211> 9
   <212> PRT
   <213> Artificial Sequence
<220>
   <223> Synthetic peptide
<400> 93
   Val Gln Ser Ser Ala Val Pro Leu Thr
   1 5
<210> 94
   <211> 9
   <212> PRT
   <213> Artificial Sequence
<220>
   <223> Synthetic peptide
<400> 94
   Val Gln Thr Ser Leu Val Pro Leu Thr
   1 5
<210> 95
   <211> 9
   <212> PRT
   <213> Artificial Sequence
<220>
   <223> Synthetic peptide
<400> 95
   Val Gln Thr Ser Val Val Pro Leu Thr
   1 5
<210> 96
   <211> 9
   <212> PRT
   <213> Artificial Sequence
<220>
   <223> Synthetic peptide
<400> 96
   Val Gln Thr Thr Ala Val Pro Leu Thr
   1               5
<210> 97
   <211> 9
   <212> PRT
   <213> Artificial Sequence
<220>
   <223> Synthetic peptide
<400> 97
   Leu Gln Thr Ala Arg Val Pro Leu Thr
   1               5
<210> 98
   <211> 9
   <212> PRT
   <213> Artificial Sequence
<220>
   <223> Synthetic peptide
<400> 98
   Phe Gln Gly Ser Leu Leu Pro Leu Ser
   1               5
<210> 99
   <211> 13
   <212> PRT
   <213> Artificial Sequence
<220>
   <223> Synthetic peptide
<400> 99
   Arg Gln Leu Gly Leu Arg Ser Ile Asp Ala Met Asp Tyr
   1               5                   10
<210> 100
   <211> 13
   <212> PRT
   <213> Artificial Sequence
<220>
   <223> Synthetic peptide
<220>
   <221> MISC_FEATURE
   <222> (1)..(5)
   <223> Xaa denotes any amino acid residue
<400> 100
   Xaa Xaa Xaa Xaa Xaa Arg Ser Ile Asp Ala Met Asp Tyr
                   1               5                   10

<210> 101
   <211> 13
   <212> PRT
   <213> Artificial Sequence
<220>
   <223> Synthetic peptide
<220>
   <221> MISC_FEATURE
   <222> (9)..(13)
   <223> Xaa denotes any amino acid residue
<400> 101
   Arg Gln Leu Gly Leu Arg Ser Ile Xaa Xaa Xaa Xaa Xaa
   1               5                   10
<210> 102
   <211> 13
   <212> PRT
   <213> Artificial Sequence
<220>
   <223> Synthetic peptide
<220>
   <221> MISC_FEATURE
   <222> (5)..(9)
   <223> Xaa denotes any amino acid residue
<400> 102
   Arg Gln Leu Gly Xaa Xaa Xaa Xaa Xaa Ala Met Asp Tyr
   1               5                   10
<210> 103
   <211> 459
   <212> DNA
   <213> Mus musculus
<400> 103
   tgggcagact taccattctc attcctgctg ctgattgtcc ctgcatatgt cttgtcccaa     60
   gttactctaa aagagtctgg ccctgggata ttgaagccct cacagaccct cagtctgact    120
   tgttctctct ctgggttttc actgagcact tctggtatgg gtgtaggctg gtttcgtcag    180
   ccttcaggga agggtctgga gtggctggca cacatttggt gggatgatga taagtcctat    240
   aatccatccc tgaagagccg gctcacaatc tccaagtata cctctagaaa ccaggttttc    300
   ctcacgatca ccagtgtgga cactgcagat actgccactt actattgtgc tcgaagacaa    360
   ctcggactaa gatcaattga tgctatggac tactggggtc aaggaacctc agtcaccgtc    420
   tcctcagcca aaacgacacc cccatctgtc tatccactg                           459
<210> 104
   <211> 435
   <212> DNA
   <213> Mus musculus
<400> 104
   agattgcctg ttaggctgtt ggtgctgatg ttctggattc ctgcttccac cagtgatgtt     60
   ttgatgaccc aaactcctct ctccctgcct gtcagtcttg gagatcaagc ctccatctct    120
   tgcagatcta gtcagagcat tctacatagt aatggaaaca cctatttaga gtggtacctg    180
   cagaaaccag gccagtctcc aaagctcctg atctacaaag tttccaaccg attttctggg    240
   gtcccagaca ggttcagtgg cagtggatca gggacagatt tcacactcaa gatcagcaga    300
   gtggaggctg aggatctggg agtttattac tgttttcaag gttcacttgt tccgctcacg    360
   ttcggtgctg ggaccaagct ggagctgaaa cgggctgatg ctgcaccaac tgtatccatc    420
   ttcccaccat ccagt                                                     435
<210> 105
   <211> 123
   <212> PRT
   <213> Mus musculus
<400> 105
   Gln Val Thr Leu Lys Glu Ser Gly Pro Gly Ile Leu Lys Pro Ser Gln
   1               5                   10                  15
   Thr Leu Ser Leu Thr Cys Ser Leu Ser Gly Phe Ser Leu Ser Thr Ser
               20                  25                  30
   Gly Met Gly Val Gly Trp Phe Arg Gln Pro Ser Gly Lys Gly Leu Glu
           35                  40                  45
   Trp Leu Ala His Ile Trp Trp Asp Asp Asp Lys Ser Tyr Asn Pro Ser
       50                  55                  60
   Leu Lys Ser Arg Leu Thr Ile Ser Lys Tyr Thr Ser Arg Asn Gln Val
   65                  70                  75                  80
   Phe Leu Thr Ile Thr Ser Val Asp Thr Ala Asp Thr Ala Thr Tyr Tyr
                   85                  90                  95
   Cys Ala Arg Arg Gln Leu Gly Leu Arg Ser Ile Asp Ala Met Asp Tyr
               100                 105                 110
   Trp Gly Gln Gly Thr Ser Val Thr Val Ser Ser
           115                 120
<210> 106
   <211> 120
   <212> PRT
   <213> Homo sapiens
<400> 106
   Gln Val Thr Leu Lys Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Met Arg Val Ser Trp Ile Arg Gln Pro Pro Gly Lys Ala Leu Glu
           35                  40                  45
   Trp Leu Ala Arg Ile Asp Trp Asp Asp Asp Lys Phe Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Ile Tyr Tyr Tyr Tyr Tyr Gly Met Asp Val Trp Gly Gln
               100                 105                 110
   Gly Thr Thr Val Thr Val Ser Ser
           115                 120
<210> 107
   <211> 123
   <212> PRT
   <213> Artificial Sequence
<220>
   <223> Synthetic antibody
<400> 107
   Gln Val Thr Leu Lys Glu Ser Gly Pro Ala Leu Val Lys Pro Thr Gln
   1 5 10 15
   Thr Leu Thr Leu Thr Cys Thr Leu Ser Gly Phe Ser Leu Ser Thr Ser
   20 25 30
   Gly Met Gly Val Gly Trp Ile Arg Gln Pro Pro Gly Lys Ala Leu Glu
   35 40 45
   Trp Leu Ala His Ile Trp Trp Asp Asp Asp Lys Ser Tyr Asn Pro Ser
   50 55 60
   Leu Lys Ser Arg Leu Thr Ile Ser Lys Asp Thr Ser Lys Asn Gln Val
   65 70 75 80
   Val Leu Thr Met Thr Asn Met Asp Pro Val Asp Thr Ala Thr Tyr Tyr
   85 90 95
   Cys Ala Arg Arg Gln Leu Gly Leu Arg Ser Ile Asp Ala Met Asp Tyr
   100 105 110
   Trp Gly Gln Gly Thr Thr Val Thr Val Ser Ser
   115 120
<210> 108
   <211> 123
   <212> PRT
   <213> Artificial Sequence
<220>
   <223> Synthetic antibody
<400> 108
   Gln Val Thr Leu Lys Glu Ser Gly Pro Gly Leu Leu Lys Pro Thr Gln
   1 5 10 15
   Thr Leu Thr Leu Thr Cys Thr Leu Ser Gly Phe Ser Leu Ser Thr Ser
   20 25 30
   Gly Met Gly Val Gly Trp Phe Arg Gln Pro Pro Gly Lys Gly Leu Glu
   35 40 45
   Trp Leu Ala His Ile Trp Trp Asp Asp Asp Lys Ser Tyr Asn Pro Ser
   50 55 60
   Leu Lys Ser Arg Leu Thr Ile Ser Lys Asp Thr Ser Lys Asn Gln Val
   65 70 75 80
   Val Leu Thr Ile Thr Asn Val Asp Pro Val Asp Thr Ala Thr Tyr Tyr
   85 90 95
   Cys Ala Arg Arg Gln Leu Gly Thr Arg Gly Thr Asp Ala Met Asp Tyr
   100 105 110
   Trp Gly Gln Gly Thr Thr Val Thr Val Ser Ser
   115 120

<210> 109
   <211> 112
   <212> PRT
   <213> Mus musculus
<400> 109
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Ser Ile Leu His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser Leu Val Pro Leu Thr Phe Gly Ala Gly Thr Lys Leu Glu Leu Lys
   100 105 110
<210> 110
   <211> 112
   <212> PRT
   <213> Homo sapiens
<400> 110
   Asp Ile Val Met Thr Gln Thr Pro Leu Ser Leu Ser Val Thr Pro Gly
   1 5 10 15
   Gln Pro Ala Ser Ile Ser Cys Lys Ser Ser Gln Ser Leu Leu His Ser
   20 25 30
   Asp Gly Lys Thr Tyr Leu Tyr Trp Tyr Leu Gln Lys Pro Gly Gln Ser
   35 40 45
   Pro Gln Leu Leu Ile Tyr Glu Val Ser Ser Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Gly
   85 90 95
   Ile His Leu Pro Tyr Thr Phe Gly Gln Gly Thr Lys Leu Glu Ile Lys
   100 105 110
<210> 111
   <211> 112
   <212> PRT
   <213> Artificial Sequence
<220>
   <223> Synthetic antibody
<400> 111
   Asp Val Val Met Thr Gln Ser Pro Leu Ser Leu Pro Val Thr Pro Gly
   1 5 10 15
   Glu Pro Ala Ser Ile Ser Cys Arg Ser Ser Gln Ser Ile Leu His Ser
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Leu Gln Thr
   85 90 95
   Thr Arg Val Pro Leu Thr Phe Gly Gln Gly Thr Lys Leu Glu Ile Lys
   100 105 110
<210> 112
   <211> 112
   <212> PRT
   <213> Artificial Sequence
<220>
   <223> Synthetic antibody
<400> 112
   Asp Val Val Met Thr Gln Thr Pro Leu Ser Leu Pro Val Thr Pro Gly
   1 5 10 15
   Gln Pro Ala Ser Ile Ser Cys Arg Ser Ser Gln Ser Ile Leu His Ser
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Leu Gln Thr
   85 90 95
   Thr Arg Val Pro Leu Thr Phe Gly Gln Gly Thr Lys Leu Glu Ile Lys
   100 105 110
<210> 113
   <211> 369
   <212> DNA
   <213> Artificial Sequence
<220>
   <223> Synthetic nucleic acid molecule
<400> 113
   caggtgaccc tgaaggagtc tggccctgcc ctggtgaagc ccacccagac cctgaccctg     60
   acctgcacct tctctggctt cagcctgagc acctctggca tgggcgtggg ctggatccgg    120
   cagccccctg gcaaggccct ggagtggctg gcccacatct ggtgggacga cgacaagtcc    180
   tacaacccca gcctgaagag ccggctgacc atcagcaagg acaccagcaa gaaccaggtg    240
   gtgctgacca tgaccaacat ggaccctgtg gacacagcca cctactactg tgcccggcgg    300
   cagctgggcc tgcggagcat tgatgccatg gactactggg gccagggcac cacagtgaca    360
   gtgtccagc                                                            369
<210> 114
   <211> 369
   <212> DNA
   <213> Artificial Sequence
<220>
   <223> Synthetic nucleic acid molecule
<400> 114
   caggtgaccc tgaaggagtc tggccctgcc ctggtgaagc ccacccagac cctgaccctg     60
   acctgcaccc tgtctggctt cagcctgagc acctctggca tgggcgtggg ctggatccgg    120
   cagccccctg gcaaggccct ggagtggctg gcccacatct ggtgggacga cgacaagtcc    180
   tacaacccca gcctgaagag ccggctgacc atcagcaagg acaccagcaa gaaccaggtg    240
   gtgctgacca tgaccaacat ggaccctgtg gacacagcca cctactactg tgcccggcgg    300
   cagctgggcc tgcggagcat tgatgccatg gactactggg gccagggcac cacagtgaca    360
   gtgtccagc                                                            369
<210> 115
   <211> 336
   <212> DNA
   <213> Artificial Sequence
<220>
   <223> Synthetic nucleic acid molecule
<400> 115
   gatgtggtga tgacccagag ccccctgtcc ctgcctgtga cccctggcga gcctgccagc     60
   atctcctgcc ggagctccca gagcatcctg cactccaatg gcaacaccta cctggagtgg    120
   tacctgcaga agcctggcca gagcccccag ctgctgatct acaaggtgtc caaccggttc    180
   tccggcgtgc ctgaccggtt cagcggctcc ggcagcggca cagacttcac cctgaagatc    240
   agccgggtgg aggctgagga tgtgggcgtc tactactgct tccagggcag cctggtgccc    300
   ctgacctttg gccagggcac caagctggag atcaag                              336
<210> 116
   <211> 482
   <212> PRT
   <213> Artificial Sequence
<220>
   <223> Synthetic polypeptide
<400> 116
   Met Lys Lys Thr Ala Ile Ala Ile Ala Val Ala Leu Ala Gly Phe Ala
   1               5                   10                  15
   Thr Val Ala Gln Ala Ala Leu Glu Gln Val Thr Leu Lys Glu Ser Gly
               20                  25                  30
   Pro Ala Leu Val Lys Pro Thr Gln Thr Leu Thr Leu Thr Cys Thr Phe
           35                  40                  45
   Ser Gly Phe Ser Leu Ser Thr Ser Gly Met Gly Val Gly Trp Ile Arg
       50                  55                  60
   Gln Pro Pro Gly Lys Ala Leu Glu Trp Leu Ala His Ile Trp Trp Asp
   65                  70                  75                  80
   Asp Asp Lys Ser Tyr Asn Pro Ser Leu Lys Ser Arg Leu Thr Ile Ser
                   85                  90                  95
   Lys Asp Thr Ser Lys Asn Gln Val Val Leu Thr Met Thr Asn Met Asp
               100                 105                 110
   Pro Val Asp Thr Ala Thr Tyr Tyr Cys Ala Arg Arg Gln Leu Gly Leu
           115                 120                 125
   Arg Ser Ile Asp Ala Met Asp Tyr Trp Gly Gln Gly Thr Thr Val Thr
       130                 135                 140
   Val Ser Ser Ala Ser Thr Lys Gly Pro Ser Val Phe Pro Leu Ala Pro
   145                 150                 155                 160
   Ser Ser Lys Ser Thr Ser Gly Gly Thr Ala Ala Leu Gly Cys Leu Val
                   165                 170                 175
   Lys Asp Tyr Phe Pro Glu Pro Val Thr Val Ser Trp Asn Ser Gly Ala
               180                 185                 190
   Leu Thr Ser Gly Val His Thr Phe Pro Ala Val Leu Gln Ser Ser Gly
           195                 200                 205
   Leu Tyr Ser Leu Ser Ser Val Val Thr Val Pro Ser Ser Ser Leu Gly
       210                 215                 220
   Thr Gln Thr Tyr Ile Cys Asn Val Asn His Lys Pro Ser Asn Thr Lys
   225                 230                 235                 240
   Val Asp Lys Arg Val Glu Pro Lys Ser Cys Thr Ser Gly His His His
                   245                 250                 255
   His His His Gly Gly Glu Gln Lys Leu Ile Ser Glu Glu Asp Leu Gly
               260                 265                 270
   Gly Pro Phe Val Cys Glu Tyr Gln Gly Gln Ser Ser Asp Leu Pro Gln
           275                 280                 285
   Pro Pro Val Asn Ala Gly Gly Gly Ser Gly Gly Gly Ser Gly Gly Gly
       290                 295                 300
   Ser Glu Gly Gly Gly Ser Glu Gly Gly Gly Ser Glu Gly Gly Gly Ser
   305                 310                 315                 320
   Glu Gly Gly Gly Ser Gly Gly Gly Ser Gly Ser Gly Asp Phe Asp Tyr
                   325                 330                 335
   Glu Lys Met Ala Asn Ala Asn Lys Gly Ala Met Thr Glu Asn Ala Asp
               340                 345                 350
   Glu Asn Ala Leu Gln Ser Asp Ala Lys Gly Lys Leu Asp Ser Val Ala
           355                 360                 365
   Thr Asp Tyr Gly Ala Ala Ile Asp Gly Phe Ile Gly Asp Val Ser Gly
       370                 375                 380
   Leu Ala Asn Gly Asn Gly Ala Thr Gly Asp Phe Ala Gly Ser Asn Ser
   385                 390                 395                 400
   Gln Met Ala Gln Val Gly Asp Gly Asp Asn Ser Pro Leu Met Asn Asn
                   405                 410                 415
   Phe Arg Gln Tyr Leu Pro Ser Leu Pro Gln Ser Val Glu Cys Arg Pro
               420                 425                 430
   Tyr Val Phe Gly Ala Gly Lys Pro Tyr Glu Phe Ser Ile Asp Cys Asp
           435                 440                 445
   Lys Ile Asn Leu Phe Arg Gly Val Phe Ala Phe Leu Leu Tyr Val Ala
   450 455 460
   Thr Phe Met Tyr Val Phe Ser Thr Phe Ala Asn Ile Leu Arg Asn Lys
   465 470 475 480
   Glu Ser

<210> 117
   <211> 481
   <212> PRT
   <213> Artificial Sequence
<220>
   <223> Synthetic polypeptide
<400> 117
   Met Lys Lys Thr Ala Ile Ala Ile Ala Val Ala Leu Ala Gly Phe Ala
   1 5 10 15
   Thr Val Ala Gln Ala Ala Leu Glu Gln Val Thr Leu Lys Glu Ser Gly
   20 25 30
   Pro Ala Leu Val Lys Pro Thr Gln Thr Leu Thr Leu Thr Cys Thr Leu
   35 40 45
   Ser Gly Phe Ser Leu Ser Thr Ser Gly Met Gly Val Gly Trp Ile Arg
   50 55 60
   Gln Pro Pro Gly Lys Ala Leu Glu Trp Leu Ala His Ile Trp Trp Asp
   65 70 75 80
   Asp Asp Lys Ser Tyr Asn Pro Ser Leu Lys Ser Arg Leu Thr Ile Ser
   85 90 95
   Lys Asp Thr Ser Lys Asn Gln Val Val Leu Thr Met Thr Asn Met Asp
   100 105 110
   Pro Val Asp Thr Ala Thr Tyr Tyr Cys Ala Arg Arg Gln Leu Gly Leu
   115 120 125
   Arg Ser Ile Asp Ala Met Asp Tyr Trp Gly Gln Gly Thr Thr Val Thr
   130 135 140
   Val Ser Ala Ser Thr Lys Gly Pro Ser Val Phe Pro Leu Ala Pro Ser
   145 150 155 160
   Ser Lys Ser Thr Ser Gly Gly Thr Ala Ala Leu Gly Cys Leu Val Lys
                   165                 170                 175
   Asp Tyr Phe Pro Glu Pro Val Thr Val Ser Trp Asn Ser Gly Ala Leu
               180                 185                 190
   Thr Ser Gly Val His Thr Phe Pro Ala Val Leu Gln Ser Ser Gly Leu
           195                 200                 205
   Tyr Ser Leu Ser Ser Val Val Thr Val Pro Ser Ser Ser Leu Gly Thr
       210                 215                 220
   Gln Thr Tyr Ile Cys Asn Val Asn His Lys Pro Ser Asn Thr Lys Val
   225                 230                 235                 240
   Asp Lys Arg Val Glu Pro Lys Ser Cys Thr Ser Gly His His His His
                   245                 250                 255
   His His Gly Gly Glu Gln Lys Leu Ile Ser Glu Glu Asp Leu Gly Gly
               260                 265                 270
   Pro Phe Val Cys Glu Tyr Gln Gly Gln Ser Ser Asp Leu Pro Gln Pro
           275                 280                 285
   Pro Val Asn Ala Gly Gly Gly Ser Gly Gly Gly Ser Gly Gly Gly Ser
       290                 295                 300
   Glu Gly Gly Gly Ser Glu Gly Gly Gly Ser Glu Gly Gly Gly Ser Glu
   305                 310                 315                 320
   Gly Gly Gly Ser Gly Gly Gly Ser Gly Ser Gly Asp Phe Asp Tyr Glu
                   325                 330                 335
   Lys Met Ala Asn Ala Asn Lys Gly Ala Met Thr Glu Asn Ala Asp Glu
               340                 345                 350
   Asn Ala Leu Gln Ser Asp Ala Lys Gly Lys Leu Asp Ser Val Ala Thr
           355                 360                 365
   Asp Tyr Gly Ala Ala Ile Asp Gly Phe Ile Gly Asp Val Ser Gly Leu
       370                 375                 380
   Ala Asn Gly Asn Gly Ala Thr Gly Asp Phe Ala Gly Ser Asn Ser Gln
   385                 390                 395                 400
   Met Ala Gln Val Gly Asp Gly Asp Asn Ser Pro Leu Met Asn Asn Phe
                   405                 410                 415
   Arg Gln Tyr Leu Pro Ser Leu Pro Gln Ser Val Glu Cys Arg Pro Tyr
               420                 425                 430
   Val Phe Gly Ala Gly Lys Pro Tyr Glu Phe Ser Ile Asp Cys Asp Lys
           435                 440                 445
   Ile Asn Leu Phe Arg Gly Val Phe Ala Phe Leu Leu Tyr Val Ala Thr
       450                 455                 460
   Phe Met Tyr Val Phe Ser Thr Phe Ala Asn Ile Leu Arg Asn Lys Glu
   465                 470                 475                 480
   Ser
<210> 118
   <211> 243
   <212> PRT
   <213> Artificial Sequence
<220>
   <223> Synthetic polypeptide
<400> 118
   Met Lys Tyr Leu Leu Pro Thr Ala Ala Ala Gly Leu Leu Leu Leu Ala
   1               5                   10                  15
   Ala Gln Pro Ala Met Ala Ser Arg Asp Val Val Met Thr Gln Ser Pro
               20                  25                  30
   Leu Ser Leu Pro Val Thr Pro Gly Glu Pro Ala Ser Ile Ser Cys Arg
           35                  40                  45
   Ser Ser Gln Ser Ile Leu His Ser Asn Gly Asn Thr Tyr Leu Glu Trp
       50                  55                  60
   Tyr Leu Gln Lys Pro Gly Gln Ser Pro Gln Leu Leu Ile Tyr Lys Val
   65                  70                  75                  80
   Ser Asn Arg Phe Ser Gly Val Pro Asp Arg Phe Ser Gly Ser Gly Ser
                   85                  90                  95
   Gly Thr Asp Phe Thr Leu Lys Ile Ser Arg Val Glu Ala Glu Asp Val
               100                 105                 110
   Gly Val Tyr Tyr Cys Phe Gln Gly Ser Leu Val Pro Leu Thr Phe Gly
           115                 120                 125
   Gln Gly Thr Lys Leu Glu Ile Lys Arg Thr Val Ala Ala Pro Ser Val
       130                 135                 140
   Phe Ile Phe Pro Pro Ser Asp Glu Gln Leu Lys Ser Gly Thr Ala Ser
   145                 150                 155                 160
   Val Val Cys Leu Leu Asn Asn Phe Tyr Pro Arg Glu Ala Lys Val Gln
                   165                 170                 175
   Trp Lys Val Asp Asn Ala Leu Gln Ser Gly Asn Ser Gln Glu Ser Val
               180                 185                 190
   Thr Glu Gln Asp Ser Lys Asp Ser Thr Tyr Ser Leu Ser Ser Thr Leu
           195                 200                 205
   Thr Leu Ser Lys Ala Asp Tyr Glu Lys His Lys Val Tyr Ala Cys Glu
       210                 215                 220
   Val Thr His Gln Gly Leu Ser Ser Pro Val Thr Lys Ser Phe Asn Arg
   225                 230                 235                 240
   Gly Glu Cys
<210> 119
   <211> 5458
   <212> DNA
   <213> Artificial Sequence
<220>
   <223> Synthetic nucleic acid molecule
<400> 119
   gcccaatacg caaaccgcct ctccccgcgc gttggccgat tcattaatgc agctggcacg     60
   acaggtttcc cgactggaaa gcgggcagtg agcgcaacgc aattaatgtg agttagctca    120
   ctcattaggc accccaggct ttacacttta tgcttccggc tcgtatgttg tgtggaattg    180
   tgagcggata acaatttcac acaagatcta gctattccag agattacgca aattctattt    240
   caaggagaca gtcataatga aatacctgct gccgactgca gctgctggtc tgctgctgct 300
   ggcggcccag ccggctatgg cttctagaga tgtggtgatg acccagagcc ccctgtccct 360
   gcctgtgacc cctggcgagc ctgccagcat ctcctgccgg agctcccaga gcatcctgca 420
   ctccaatggc aacacctacc tggagtggta cctgcagaag cctggccaga gcccccagct 480
   gctgatctac aaggtgtcca accggttctc cggcgtgcct gaccggttca gcggctccgg 540
   cagcggcaca gacttcaccc tgaagatcag ccgggtggag gctgaggatg tgggcgtcta 600
   ctactgcttc cagggcagcc tggtgcccct gacctttggc cagggcacca agctggagat 660
   caagcgtacg gtggctgcac catctgtctt catcttcccg ccatctgatg agcagttgaa 720
   atctggaact gcctctgttg tgtgcctgct gaataacttc tatcccagag aggccaaagt 780
   acagtggaag gtggataacg ccctccaatc gggtaactcc caggagagtg tcacagagca 840
   ggacagcaag gacagcacct acagcctcag cagcaccctg acgctgagca aagcagacta 900
   cgagaaacac aaagtctacg cctgcgaagt cacccatcag ggcctgagct cgcccgtcac 960
   aaagagcttc aacaggggag agtgttaaca attgctagaa ttgtgagcgg ataacaattt 1020
   cagcaggtcg agttcttgat aacgaggcgt aaaaaatgaa aaagacagct atcgcgattg 1080
   cagtggcact ggctggtttc gctaccgtgg cccaggcggc cctcgagcag gtgaccctga 1140
   aggagtctgg ccctgccctg gtgaagccca cccagaccct gaccctgacc tgcaccttct 1200
   ctggcttcag cctgagcacc tctggcatgg gcgtgggctg gatccggcag ccccctggca 1260
   aggccctgga gtggctggcc cacatctggt gggacgacga caagtcctac aaccccagcc 1320
   tgaagagccg gctgaccatc agcaaggaca ccagcaagaa ccaggtggtg ctgaccatga 1380
   ccaacatgga ccctgtggac acagccacct actactgtgc ccggcggcag ctgggcctgc 1440
   ggagcattga tgccatggac tactggggcc agggcaccac agtgacagtg tccagcgcct 1500
   ccaccaaggg cccatcggtc ttccccctgg caccctcctc caagagcacc tctgggggca 1560
   cagcggccct gggctgcctg gtcaaggact acttccccga accggtgacg gtgtcgtgga 1620
   actcaggcgc cctgaccagc ggcgtgcaca ccttcccggc tgtcctacag tcctcaggac 1680
   tctactccct cagcagcgtg gtgaccgtgc cctccagcag cttgggcacc cagacctaca 1740
   tctgcaacgt gaatcacaag cccagcaaca ccaaggtgga caagagagtt gagcccaaat 1800
   cttgtactag tggccaccac caccatcacc atggcggtga acaaaaactc atctcagaag 1860
   aggatctggg tggttagcca ttcgtttgtg aatatcaagg ccaatcgtct gacctgcctc 1920
   aacctcctgt caatgctggc ggcggctctg gtggtggttc tggtggcggc tctgagggtg 1980
   gcggctctga gggtggcggt tctgagggtg gcggctctga gggtggcggt tccggtggcg 2040
   gctccggttc cggtgatttt gattatgaaa aaatggcaaa cgctaataag ggggctatga 2100
   ccgaaaatgc cgatgaaaac gcgctacagt ctgacgctaa aggcaaactt gattctgtcg 2160
   ctactgatta cggtgctgct atcgatggtt tcattggtga cgtttccggc cttgctaatg 2220
   gtaatggtgc tactggtgat tttgctggct ctaattccca aatggctcaa gtcggtgacg 2280
   gtgataattc acctttaatg aataatttcc gtcaatattt accttctttg cctcagtcgg 2340
   ttgaatgtcg cccttatgtc tttggcgctg gtaaaccata tgaattttct attgattgtg 2400
   acaaaataaa cttattccgt ggtgtctttg cgtttctttt atatgttgcc acctttatgt 2460
   atgtattttc gacgtttgct aacatactgc gtaataagga gtcttaagct agctggccgt 2520
   cgttttacaa cgtcgtgact gggaaaaccc tggcgttacc caacttaatc gccttgcagc 2580
   acatccccct ttcgccagct ggcgtaatag cgaagaggcc cgcaccgatc gcccttccca 2640
   acagttgcgc agcctgaatg gcgaatggcg attccgttgc aatggctggc ggtaatattg 2700
   ttctggatat taccagcaag gccgatagtt tgagttcttc tactcaggca agtgatgtta 2760
   ttactaatca aagaagtatt gcgacaacgg ttaatttgcg tgatggacag actcttttac 2820
   tcggtggcct cactgattat aaaaacactt ctcaggattc tggcgtaccg ttcctgtcta 2880
   aaatcccttt aatcggcctc ctgtttagct cccgctctga ttctaacgag gaaagcacgt 2940
   tatacgtgct cgtcaaagca accatagtac gcgccctgta gcggcgcatt aagcgcggcg 3000
   ggtgtggtgg ttacgcgcag cgtgaccgct acacttgcca gcgccctagc gcccgctcct 3060
   ttcgctttct tcccttcctt tctcgccacg ttcgccggct ttccccgtca agctctaaat 3120
   cgggggctcc ctttagggtt ccgatttagt gctttacggc acctcgaccc caaaaaactt 3180
   gattagggtg atggttcacg tagtgggcca tcgccctgat agacggtttt tcgccctttg 3240
   acgttggagt ccacgttctt taatagtgga ctcttgttcc aaactggaac aacactcaac 3300
   cctatctcgg tctattcttt tgatttataa gggattttgc cgatttcggc ctattggtta 3360
   aaaaatgagc tgatttaaca aaaatttaac gcgaatttta acaaaatatt aacgtttaca 3420
   atttaaatat ttgcttatac aatcttcctg tttttggggc ttttctgatt atcaaccggg 3480
   gtacatatga ttgacatgct agttttacga ttaccgttca tcgcaggtgg cacttttcgg 3540
   ggaaatgtgc gcggaacccc tatttgttta tttttctaaa tacattcaaa tatgtatccg 3600
   ctcatgagac aataaccctg ataaatgctt caataatatt gaaaaaggaa gagtatgagt 3660
   attcaacatt tccgtgtcgc ccttattccc ttttttgcgg cattttgcct tcctgttttt 3720
   gctcacccag aaacgctggt gaaagtaaaa gatgctgaag atcagttggg tgcacgagtg 3780
   ggttacatcg aactggatct caacagcggt aagatccttg agagttttcg ccccgaagaa 3840
   cgttttccaa tgatgagcac ttttaaagtt ctgctatgtg gcgcggtatt atcccgtatt 3900
   gacgccgggc aagagcaact cggtcgccgc atacactatt ctcagaatga cttggttgag 3960
   tactcaccag tcacagaaaa gcatcttacg gatggcatga cagtaagaga attatgcagt 4020
   gctgccataa ccatgagtga taacactgcg gccaacttac ttctgacaac gatcggagga 4080
   ccgaaggagc taaccgcttt tttgcacaac atgggggatc atgtaactcg ccttgatcgt 4140
   tgggaaccgg agctgaatga agccatacca aacgacgagc gtgacaccac gatgcctgta 4200
   gcaatggcaa caacgttgcg caaactatta actggcgaac tacttactct agcttcccgg 4260
   caacaattaa tagactggat ggaggcggat aaagttgcag gaccacttct gcgctcggcc 4320
   cttccggctg gctggtttat tgctgataaa tctggagccg gtgagcgtgg gtctcgcggt 4380
   atcattgcag cactggggcc agatggtaag ccctcccgta tcgtagttat ctacacgacg 4440
   gggagtcagg caactatgga tgaacgaaat agacagatcg ctgagatagg tgcctcactg 4500
   attaagcatt ggtaactgtc agaccaagtt tactcatata tactttagat tgatttaaaa 4560
   cttcattttt aatttaaaag gatctaggtg aagatccttt ttgataatct catgaccaaa 4620
   atcccttaac gtgagttttc gttccactga gcgtcagacc ccgtagaaaa gatcaaagga 4680
   tcttcttgag atcctttttt tctgcgcgta atctgctgct tgcaaacaaa aaaaccaccg 4740
   ctaccagcgg tggtttgttt gccggatcaa gagctaccaa ctctttttcc gaaggtaact 4800
   ggcttcagca gagcgcagat accaaatact gtccttctag tgtagccgta gttaggccac 4860
   cacttcaaga actctgtagc accgcctaca tacctcgctc tgctaatcct gttaccagtg 4920
   gctgctgcca gtggcgataa gtcgtgtctt accgggttgg actcaagacg atagttaccg 4980
   gataaggcgc agcggtcggg ctgaacgggg ggttcgtgca cacagcccag cttggagcga 5040
   acgacctaca ccgaactgag atacctacag cgtgagctat gagaaagcgc cacgcttccc 5100
   gaagggagaa aggcggacag gtatccggta agcggcaggg tcggaacagg agagcgcacg 5160
   agggagcttc cagggggaaa cgcctggtat ctttatagtc ctgtcgggtt tcgccacctc 5220
   tgacttgagc gtcgattttt gtgatgctcg tcaggggggc ggagcctatg gaaaaacgcc 5280
   agcaacgcgg cctttttacg gttcctggcc ttttgctggc cttttgctca catgttcttt 5340
   cctgcgttat cccctgattc tgtggataac cgtattaccg cctttgagtg agctgatacc 5400
   gctcgccgca gccgaacgac cgagcgcagc gagtcagtga gcgaggaagc ggaagagc 5458
<210> 120
   <211> 26
   <212> DNA
   <213> Artificial Sequence
<220>
   <223> Synthetic oligonucleotide
<400> 120
   ctatggcttc tagagatgtg gtgatg 26
<210> 121
   <211> 398
   <212> DNA
   <213> Artificial Sequence
<220>
   <223> Synthetic nucleic acid molecule
<400> 121
   agctgctggt ctgctgctgc tggcggccca gccggctatg gcttctagag atgtggtgat     60
   gacccagagc cccctgtccc tgcctgtgac ccctggcgag cctgccagca tctcctgccg    120
   gagctcccag agcatcctgc actccaatgg caacacctac ctggagtggt acctgcagaa    180
   gcctggccag agcccccagc tgctgatcta caaggtgtcc aaccggttct ccggcgtgcc    240
   tgaccggttc agcggctccg gcagcggcac agacttcacc ctgaagatca gccgggtgga    300
   ggctgaggat gtgggcgtct actactgctt ccagggcagc ctggtgcccc tgacctttgg    360
   ccagggcacc aagctggaga tcaagcgtac ggtggctg                            398
<210> 122
   <211> 398
   <212> DNA
   <213> Artificial Sequence
<220>
   <223> Synthetic nucleic acid molecule
<220>
   <221> misc_feature
   <222> (329)..(330)
   <223> n is a, c, g, or t
<220>
   <221> misc_feature
   <222> (332)..(333)
   <223> n is a, c, g, or t
<220>
   <221> misc_feature
   <222> (335)..(336)
   <223> n is a, c, g, or t
<220>
   <221> misc_feature
   <222> (338)..(339)
   <223> n is a, c, g, or t
<220>
   <221> misc_feature
   <222> (341)..(342)
   <223> n is a, c, g, or t
<400> 122
   agctgctggt ctgctgctgc tggcggccca gccggctatg gcttctagag atgtggtgat      60
   gacccagagc cccctgtccc tgcctgtgac ccctggcgag cctgccagca tctcctgccg     120
   gagctcccag agcatcctgc actccaatgg caacacctac ctggagtggt acctgcagaa     180
   gcctggccag agcccccagc tgctgatcta caaggtgtcc aaccggttct ccggcgtgcc     240
   tgaccggttc agcggctccg gcagcggcac agacttcacc ctgaagatca gccgggtgga     300
   ggctgaggat gtgggcgtct actactgcnn knnknnknnk nnkgtgcccc tgacctttgg     360
   ccagggcacc aagctggaga tcaagcgtac ggtggctg                             398
<210> 123
   <211> 82
   <212> DNA
   <213> Artificial Sequence
<220>
   <223> Synthetic oligonucleotide
<220>
   <221> misc_feature
   <222> (57)..(58)
   <223> n is a, c, g, or t
<220>
   <221> misc_feature
   <222> (60)..(61)
   <223> n is a, c, g, or t
<220>
   <221> misc_feature
   <222> (63)..(64)
   <223> n is a, c, g, or t
<220>
   <221> misc_feature
   <222> (66)..(67)
   <223> n is a, c, g, or t
<220>
   <221> misc_feature
   <222> (69)..(70)
   <223> n is a, c, g, or t

<400> 123
   cagccaccgt acgcttgatc tccagcttgg tgccctggcc aaaggtcagg ggcacmnnmn      60
   nmnnmnnmnn gcagtagtag ac                                               82
<210> 124
   <211> 398
   <212> DNA
   <213> Artificial Sequence
<220>
   <223> Synthetic nucleic acid molecule
<220>
   <221> misc feature
   <222> (341)..(342)
   <223> n is a, c, g, or t
<220>
   <221> misc_feature
   <222> (344)..(345)
   <223> n is a, c, g, or t
<220>
   <221> misc_feature
   <222> (347)..(348)
   <223> n is a, c, g, or t
<220>
   <221> misc_feature
   <222> (350)..(351)
   <223> n is a, c, g, or t
<220>
   <221> misc_feature
   <222> (353)..(354)
   <223> n is a, c, g, or t
<400> 124
   agctgctggt ctgctgctgc tggcggccca gccggctatg gcttctagag atgtggtgat      60
   gacccagagc cccctgtccc tgcctgtgac ccctggcgag cctgccagca tctcctgccg      120
   gagctcccag agcatcctgc actccaatgg caacacctac ctggagtggt acctgcagaa     180
   gcctggccag agcccccagc tgctgatcta caaggtgtcc aaccggttct ccggcgtgcc     240
   tgaccggttc agcggctccg gcagcggcac agacttcacc ctgaagatca gccgggtgga     300
   ggctgaggat gtgggcgtct actactgctt ccagggcagc nnknnknnkn nknnktttgg     360
   ccagggcacc aagctggaga tcaagcgtac ggtggctg                             398
<210> 125
   <211> 82
   <212> DNA
   <213> Artificial Sequence
<220>
   <223> Synthetic oligonucleotide
<220>
   <221> misc_feature
   <222> (45)..(46)
   <223> n is a, c, g, or t
<220>
   <221> misc feature
   <222> (48)..(49)
   <223> n is a, c, g, or t
<220>
   <221> misc_feature
   <222> (51)..(52)
   <223> n is a, c, g, or t
<220>
   <221> misc_feature
   <222> (54)..(55)
   <223> n is a, c, g, or t
<220>
   <221> misc_feature
   <222> (57)..(58)
   <223> n is a, c, g, or t
<400> 125
   cagccaccgt acgcttgatc tccagcttgg tgccctggcc aaamnnmnnm nnmnnmnngc 60
   tgccctggaa gcagtagtag ac 82
<210> 126
   <211> 27
   <212> DNA
   <213> Artificial Sequence
<220>
   <223> Synthetic oligonucleotide
<400> 126
   gtttatctcg agcaggtgac cctgaag 27
<210> 127
   <211> 399
   <212> DNA
   <213> Artificial Sequence
<220>
   <223> Synthetic nucleic acid molecule
<400> 127
   ccgtggccca ggcggccctc gagcaggtga ccctgaagga gtctggccct gccctggtga 60
   agcccaccca gaccctgacc ctgacctgca ccttctctgg cttcagcctg agcacctctg 120
   gcatgggcgt gggctggatc cggcagcccc ctggcaaggc cctggagtgg ctggcccaca 180
   tctggtggga cgacgacaag tcctacaacc ccagcctgaa gagccggctg accatcagca 240
   aggacaccag caagaaccag gtggtgctga ccatgaccaa catggaccct gtggacagtg 300
   cccggcggca gctgggcctg cggagcattg atgccatgga ctactggggc cagggcacca 360
   cagtgacagt gtccagcgcc tccaccaagg gcccatcgg 399

<210> 128
   <211> 399
   <212> DNA
   <213> Artificial Sequence
<220>
   <223> Synthetic nucleic acid molecule
<220>
   <221> misc_feature
   <222> (306)..(307)
   <223> n is a, c, g, or t
<220>
   <221> misc_feature
   <222> (309)..(310)
   <223> n is a, c, g, or t
<220>
   <221> misc_feature
   <222> (312)..(313)
   <223> n is a, c, g, or t
<220>
   <221> misc_feature
   <222> (315)..(316)
   <223> n is a, c, g, or t
<220>
   <221> misc_feature
   <222> (318)..(319)
   <223> n is a, c, g, or t
<400> 128
   ccgtggccca ggcggccctc gagcaggtga ccctgaagga gtctggccct gccctggtga 60
   agcccaccca gaccctgacc ctgacctgca ccttctctgg cttcagcctg agcacctctg 120
   gcatgggcgt gggctggatc cggcagcccc ctggcaaggc cctggagtgg ctggcccaca 180
   tctggtggga cgacgacaag tcctacaacc ccagcctgaa gagccggctg accatcagca 240
   aggacaccag caagaaccag gtggtgctga ccatgaccaa catggaccct gtggacagtg 300
   cccggnnknn knnknnknnk cggagcattg atgccatgga ctactggggc cagggcacca    360
   cagtgacagt gtccagcgcc tccaccaagg gcccatcgg                           399
<210> 129
   <211> 102
   <212> DNA
   <213> Artificial Sequence
<220>
   <223> Synthetic oligonucleotide
<220>
   <221> misc_feature
   <222> (81)..(82)
   <223> n is a, c, g, or t
<220>
   <221> misc feature
   <222> (84)..(85)
   <223> n is a, c, g, or t
<220>
   <221> misc_feature
   <222> (87)..(88)
   <223> n is a, c, g, or t
<220>
   <221> misc feature
   <222> (90)..(91)
   <223> n is a, c, g, or t
<220>
   <221> misc_feature
   <222> (93)..(94)
   <223> n is a, c, g, or t
<400> 129
   ccgatgggcc cttggtggag gcgctggaca ctgtcactgt ggtgccctgg ccccagtagt     60
   ccatggcatc aatgctccgm nnmnnmnnmn nmnnccgggc ac                       102
<210> 130
   <211> 399
   <212> DNA
   <213> Artificial Sequence
<220>
   <223> Synthetic nucleic acid molecule
<220>
   <221> misc_feature
   <222> (330)..(331)
   <223> n is a, c, g, or t

<220>
   <221> misc_feature
   <222> (333)..(334)
   <223> n is a, c, g, or t
<220>
   <221> misc_feature
   <222> (336)..(337)
   <223> n is a, c, g, or t
<220>
   <221> misc_feature
   <222> (339)..(340)
   <223> n is a, c, g, or t
<220>
   <221> misc_feature
   <222> (392)..(343)
   <223> n is a, c, g, or t
<400> 130
   ccgtggccca ggcggccctc gagcaggtga ccctgaagga gtctggccct gccctggtga      60
   agcccaccca gaccctgacc ctgacctgca ccttctctgg cttcagcctg agcacctctg     120
   gcatgggcgt gggctggatc cggcagcccc ctggcaaggc cctggagtgg ctggcccaca     180
   tctggtggga cgacgacaag tcctacaacc ccagcctgaa gagccggctg accatcagca     240
   aggacaccag caagaaccag gtggtgctga ccatgaccaa catggaccct gtggacagtg     300
   cccggcggca gctgggcctg cggagcattn nknnknnknn knnktggggc cagggcacca     360
   cagtgacagt gtccagcgcc tccaccaagg gcccatcgg                            399
<210> 131
   <211> 91
   <212> DNA
   <213> Artificial Sequence
<220>
   <223> Synthetic oligonucleotide
<220>
   <221> misc_feature
   <222> (57)..(58)
   <223> n is a, c, g, or t
<220>
   <221> misc_feature
   <222> (60)..(61)
   <223> n is a, c, g, or t
<220>
   <221> misc_feature
   <222> (63)..(64)
   <223> n is a, c, g, or t
<220>
   <221> misc_feature
   <222> (66)..(67)
   <223> n is a, c, g, or t
<220>
   <221> misc_feature
   <222> (69)..(70)
   <223> n is a, c, g, or t
<400> 131
   ccgatgggcc cttggtggag gcgctggaca ctgtcactgt ggtgccctgg ccccamnnmn     60
   nmnnmnnmnn aatgctccgc aggcccagct g                                    91
<210> 132
   <211> 399
   <212> DNA
   <213> Artificial Sequence
<220>
   <223> Synthetic nucleic acid molecule
<220>
   <221> misc_feature
   <222> (318)..(319)
   <223> n is a, c, g, or t
<220>
   <221> misc_feature
   <222> (321)..(322)
   <223> n is a, c, g, or t
<220>
   <221> misc_feature
   <222> (324)..(325)
   <223> n is a, c, g, or t
<220>
   <221> misc_feature
   <222> (327)..(328)
   <223> n is a, c, g, or t
<220>
   <221> misc_feature
   <222> (330)..(331)
   <223> n is a, c, g, or t
<400> 132
   ccgtggccca ggcggccctc gagcaggtga ccctgaagga gtctggccct gccctggtga     60
   agcccaccca gaccctgacc ctgacctgca ccttctctgg cttcagcctg agcacctctg    120
   gcatgggcgt gggctggatc cggcagcccc ctggcaaggc cctggagtgg ctggcccaca    180
   tctggtggga cgacgacaag tcctacaacc ccagcctgaa gagccggctg accatcagca    240
   aggacaccag caagaaccag gtggtgctga ccatgaccaa catggaccct gtggacagtg    300
   cccggcggca gctgggcnnk nnknnknnkn nkgccatgga ctactggggc cagggcacca    360
   cagtgacagt gtccagcgcc tccaccaagg gcccatcgg                           399
<210> 133
   <211> 91
   <212> DNA
   <213> Artificial Sequence
<220>
   <223> Synthetic oligonucleotide
<220>
   <221> misc_feature
   <222> (69)..(70)
   <223> n is a, c, g, or t
<220>
   <221> misc_feature
   <222> (72)..(73)
   <223> n is a, c, g, or t
<220>
   <221> misc_feature
   <222> (75)..(76)
   <223> n is a, c, g, or t
<220>
   <221> misc_feature
   <222> (78)..(79)
   <223> n is a, c, g, or t
<220>
   <221> misc_feature
   <222> (81)..(82)
   <223> n is a, c, g, or t
<400> 133
   ccgatgggcc cttggtggag gcgctggaca ctgtcactgt ggtgccctgg ccccagtagt     60
   ccatggcmnn mnnmnnmnnm nngcccagct g                                    91
<210> 134
   <211> 330
   <212> PRT
   <213> Homo sapiens
<400> 134
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Ala Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330

<210> 135
   <211> 326
   <212> PRT
   <213> Homo sapiens
<400> 135
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Thr Ser Ser Asn Phe Gly Thr Gln Thr
   65                  70                  75                  80
   Tyr Thr Cys Asn Val Asp His Lys Pro Ser Asn Thr Lys Val Asp Lys
                   85                  90                  95
   Thr Val Glu Arg Lys Cys Cys Val Glu Cys Pro Pro Cys Pro Ala Pro
               100                 105                 110
   Pro Val Ala Gly Pro Ser Val Phe Leu Phe Pro Pro Lys Pro Lys Asp
           115                 120                 125
   Thr Leu Met Ile Ser Arg Thr Pro Glu Val Thr Cys Val Val Val Asp
   130 135 140
   Val Ser His Glu Asp Pro Glu Val Gln Phe Asn Trp Tyr Val Asp Gly
   145 150 155 160
   Val Glu Val His Asn Ala Lys Thr Lys Pro Arg Glu Glu Gln Phe Asn
   165 170 175
   Ser Thr Phe Arg Val Val Ser Val Leu Thr Val Val His Gln Asp Trp
   180 185 190
   Leu Asn Gly Lys Glu Tyr Lys Cys Lys Val Ser Asn Lys Gly Leu Pro
   195 200 205
   Ala Pro Ile Glu Lys Thr Ile Ser Lys Thr Lys Gly Gln Pro Arg Glu
   210 215 220
   Pro Gln Val Tyr Thr Leu Pro Pro Ser Arg Glu Glu Met Thr Lys Asn
   225 230 235 240
   Gln Val Ser Leu Thr Cys Leu Val Lys Gly Phe Tyr Pro Ser Asp Ile
   245 250 255
   Ala Val Glu Trp Glu Ser Asn Gly Gln Pro Glu Asn Asn Tyr Lys Thr
   260 265 270
   Thr Pro Pro Met Leu Asp Ser Asp Gly Ser Phe Phe Leu Tyr Ser Lys
   275 280 285
   Leu Thr Val Asp Lys Ser Arg Trp Gln Gln Gly Asn Val Phe Ser Cys
   290 295 300
   Ser Val Met His Glu Ala Leu His Asn His Tyr Thr Gln Lys Ser Leu
   305 310 315 320
   Ser Leu Ser Pro Gly Lys
   325
<210> 136
   <211> 327
   <212> PRT
   <213> Homo sapiens
<400> 136
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly Lys
   325
<210> 137
   <211> 326
   <212> PRT
   <213> Artificial Sequence
<220>
   <223> Synthetic polypeptide
<400> 137
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Thr Ser Ser Asn Phe Gly Thr Gln Thr
   65 70 75 80
   Tyr Thr Cys Asn Val Asp His Lys Pro Ser Asn Thr Lys Val Asp Lys
   85 90 95
   Thr Val Glu Arg Lys Cys Cys Val Glu Cys Pro Pro Cys Pro Ala Pro
               100                 105                 110
   Pro Val Ala Gly Pro Ser Val Phe Leu Phe Pro Pro Lys Pro Lys Asp
           115                 120                 125
   Thr Leu Met Ile Ser Arg Thr Pro Glu Val Thr Cys Val Val Val Asp
       130                 135                 140
   Val Ser Gln Glu Asp Pro Glu Val Gln Phe Asn Trp Tyr Val Asp Gly
   145                 150                 155                 160
   Val Glu Val His Asn Ala Lys Thr Lys Pro Arg Glu Glu Gln Phe Asn
                   165                 170                 175
   Ser Thr Phe Arg Val Val Ser Val Leu Thr Val Leu His Gln Asp Trp
               180                 185                 190
   Leu Asn Gly Lys Glu Tyr Lys Cys Lys Val Ser Asn Lys Gly Leu Pro
           195                 200                 205
   Ser Ser Ile Glu Lys Thr Ile Ser Lys Thr Lys Gly Gln Pro Arg Glu
       210                 215                 220
   Pro Gln Val Tyr Thr Leu Pro Pro Ser Arg Glu Glu Met Thr Lys Asn
   225                 230                 235                 240
   Gln Val Ser Leu Thr Cys Leu Val Lys Gly Phe Tyr Pro Ser Asp Ile
                   245                 250                 255
   Ala Val Glu Trp Glu Ser Asn Gly Gln Pro Glu Asn Asn Tyr Lys Thr
               260                 265                 270
   Thr Pro Pro Met Leu Asp Ser Asp Gly Ser Phe Phe Leu Tyr Ser Lys
           275                 280                 285
   Leu Thr Val Asp Lys Ser Arg Trp Gln Gln Gly Asn Val Phe Ser Cys
       290                 295                 300
   Ser Val Met His Glu Ala Leu His Asn His Tyr Thr Gln Lys Ser Leu
   305                 310                 315                 320
   Ser Leu Ser Pro Gly Lys
                   325
<210> 138
   <211> 449
   <212> PRT
   <213> Artificial Sequence
<220>
   <223> Synthetic polypeptide
<400> 138
   Gln Val Thr Leu Lys Glu Ser Gly Pro Gly Leu Leu Lys Pro Thr Gln
   1               5                   10                  15
   Thr Leu Thr Leu Thr Cys Thr Leu Ser Gly Phe Ser Leu Ser Thr Ser
               20                  25                  30
   Gly Met Gly Val Gly Trp Phe Arg Gln Pro Pro Gly Lys Gly Leu Glu
           35                  40                  45
   Trp Leu Ala His Ile Trp Trp Asp Asp Asp Lys Ser Tyr Asn Pro Ser
       50                  55                  60
   Leu Lys Ser Arg Leu Thr Ile Ser Lys Asp Thr Ser Lys Asn Gln Val
   65                  70                  75                  80
   Val Leu Thr Ile Thr Asn Val Asp Pro Val Asp Thr Ala Thr Tyr Tyr
                   85                  90                  95
   Cys Ala Arg Arg Gln Leu Gly Thr Arg Gly Thr Asp Ala Met Asp Tyr
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Thr Ser Ser Asn Phe Gly Thr Gln Thr Tyr Thr Cys Asn Val
           195                 200                 205
   Asp His Lys Pro Ser Asn Thr Lys Val Asp Lys Thr Val Glu Arg Lys
       210                 215                 220
   Cys Cys Val Glu Cys Pro Pro Cys Pro Ala Pro Pro Val Ala Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser Gln Glu Asp
               260                 265                 270
   Pro Glu Val Gln Phe Asn Trp Tyr Val Asp Gly Val Glu Val His Asn
           275                 280                 285
   Ala Lys Thr Lys Pro Arg Glu Glu Gln Phe Asn Ser Thr Phe Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Gly Leu Pro Ser Ser Ile Glu Lys
                   325                 330                 335
   Thr Ile Ser Lys Thr Lys Gly Gln Pro Arg Glu Pro Gln Val Tyr Thr
               340                 345                 350
   Leu Pro Pro Ser Arg Glu Glu Met Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Met Leu
   385                 390                 395                 400
   Asp Ser Asp Gly Ser Phe Phe Leu Tyr Ser Lys Leu Thr Val Asp Lys
                   405                 410                 415
   Ser Arg Trp Gln Gln Gly Asn Val Phe Ser Cys Ser Val Met His Glu
               420                 425                 430
   Ala Leu His Asn His Tyr Thr Gln Lys Ser Leu Ser Leu Ser Pro Gly
           435                 440                 445
   Lys
<210> 139
   <211> 1407
   <212> DNA
   <213> Artificial Sequence
<220>
   <223> Synthetic nucleic acid molecule
<400> 139
   atggaatgga gctgggtctt tctcttcttc ctgtcagtaa ctacaggtgt ccactcgcag      60
   gtgaccctga aggagtctgg ccctggcctg ctgaagccca cccagaccct gaccctgacc     120
   tgcaccctgt ctggcttcag cctgagcacc tctggcatgg gcgtgggctg gttccggcag     180
   ccccctggca agggcctgga gtggctggcc cacatctggt gggacgacga caagtcctac     240
   aaccccagcc tgaagagccg gctgaccatc agcaaggaca ccagcaagaa ccaggtggtg     300
   ctgaccatca ccaacgtgga ccctgtggac acagccacct actactgtgc ccggcggcag     360
   ctgggcacta gggggacgga tgccatggac tactggggcc agggcaccac agtgacagtg     420
   tccagcgcat ccaccaaggg cccatccgtc ttccccctgg cgccctgctc caggagcacc     480
   tccgagagca cagccgccct gggctgcctg gtcaaggact acttccccga accggtgacg     540
   gtgtcgtgga actcaggcgc cctgaccagc ggcgtgcaca ccttcccggc tgtcctacag     600
   tcctcaggac tctactccct cagcagcgtg gtgaccgtga cctccagcaa ctttggcacg     660
   cagacctaca cctgcaacgt agatcacaag cccagcaaca ccaaggtgga caagacagtt     720
   gagcggaaat gctgcgtgga gtgcccacca tgcccagcac ctccagtggc cggaccatca     780
   gtcttcctgt tccccccaaa acccaaggac actctcatga tctcccggac ccctgaggtc      840
   acgtgcgtgg tggtggacgt gagccaggaa gaccccgagg tccagttcaa ctggtacgtg     900
   gatggcgtgg aggtgcataa tgccaagaca aagccgcggg aggagcagtt caacagcacg     960
   ttccgtgtgg tcagcgtcct caccgtcctg caccaggact ggctgaacgg caaggagtac    1020
   aagtgcaagg tctccaacaa aggcctcccg tcctccatcg agaaaaccat ctccaaaacc    1080
   aaagggcagc cccgagagcc acaggtgtac accctgcccc catcccggga ggagatgacc    1140
   aagaaccagg tcagcctgac ctgcctggtc aaaggcttct accccagcga catcgccgtg    1200
   gagtgggaga gcaatgggca gccggagaac aactacaaga ccacgcctcc catgctggac   1260
   tccgacggct ccttcttcct ctacagcaag ctaaccgtgg acaagagcag gtggcagcag   1320
   gggaatgtct tctcatgctc cgtgatgcat gaggctctgc acaaccacta cacacagaag   1380
   agcctctccc tgtctcctgg taaatga                                       1407
<210> 140
   <211> 219
   <212> PRT
   <213> Artificial Sequence
<220>
   <223> Synthetic polypeptide
<400> 140
   Asp Val Val Met Thr Gln Thr Pro Leu Ser Leu Pro Val Thr Pro Gly
   1               5                   10                  15
   Gln Pro Ala Ser Ile Ser Cys Arg Ser Ser Gln Ser Ile Leu His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Leu Gln Thr
                   85                  90                  95
   Thr Arg Val Pro Leu Thr Phe Gly Gln Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 141
   <211> 720
   <212> DNA
   <213> Artificial Sequence
<220>
   <223> Synthetic oligonucleotide
<400> 141
   atgagtgtgc ccactcaggt cctggggttg ctgctgctgt ggcttacaga tgccagatgc     60
   gatgtggtga tgacccagac ccccctgtcc ctgcctgtga cccctggcca gcctgccagc    120
   atctcctgcc ggagctccca gagcatcctg cactccaatg gcaacaccta cctggagtgg    180
   tacctgcaga agcctggcca gagcccccag ctgctgatct acaaggtgtc caaccggttc    240
   tccggcgtgc ctgaccggtt cagcggctcc ggcagcggca cagacttcac cctgaagatc    300
   agccgggtgg aggctgagga tgtgggcgtc tactactgcc ttcagactac tcgtgtgccc    360
   ctgacctttg gccagggcac caagctggag atcaagcgta cggtggctgc accatctgtc    420
   ttcatcttcc cgccatctga tgagcagttg aaatctggaa ctgcctctgt tgtgtgcctg    480
   ctgaataact tctatcccag agaggccaaa gtacagtgga aggtggataa cgccctccaa    540
   tcgggtaact cccaggagag tgtcacagag caggacagca aggacagcac ctacagcctc    600
   agcagcaccc tgacgctgag caaagcagac tacgagaaac acaaagtcta cgcctgcgaa    660
   gtcacccatc agggcctgag ctcgcccgtc acaaagagct tcaacagggg agagtgttag    720
<210> 142
   <211> 13
   <212> PRT
   <213> Artificial Sequence
<220>
   <223> Synthetic peptide
<400> 142
   Arg Gln Leu Gly Lys Leu Ala Leu Asp Ala Met Asp Tyr
   1 5 10
<210> 143
   <211> 13
   <212> PRT
   <213> Artificial Sequence
<220>
   <223> Synthetic peptide
<400> 143
   Arg Gln Leu Gly Leu Arg Ser Ile Asp Ala Met Asp Tyr
   1 5 10
<210> 144
   <211> 13
   <212> PRT
   <213> Artificial Sequence
<220>
   <223> Synthetic peptide
<400> 144
   Arg Gln Leu Gly Gln Arg Gln Thr Asp Ala Met Asp Tyr
   1 5 10
<210> 145
   <211> 13
   <212> PRT
   <213> Artificial Sequence
<220>
   <223> Synthetic peptide
<400> 145
   Arg Ala Ile Gln Pro Arg Ser Ile Asp Ala Met Asp Tyr
   1 5 10
<210> 146
   <211> 13
   <212> PRT
   <213> Artificial Sequence
<220>
   <223> Synthetic peptide
<400> 146
   Arg Gln Leu Gly Leu Arg Ser Ile Asp Ala His Thr Arg
   1 5 10

<210> 147
   <211> 13
   <212> PRT
   <213> Artificial Sequence
<220>
   <223> Synthetic peptide
<400> 147
   Arg Gln Leu Gly Gln Pro Ser Val Asp Ala Met Asp Tyr
   1 5 10
<210> 148
   <211> 13
   <212> PRT
   <213> Artificial Sequence
<220>
   <223> Synthetic peptide
<400> 148
   Arg Gln Leu Gly Phe Gln Ser Thr Asp Ala Met Asp Tyr
   1 5 10
<210> 149
   <211> 13
   <212> PRT
   <213> Artificial Sequence
<220>
   <223> Synthetic peptide
<400> 149
   Arg Gln Leu Gly Gln Ala Gly His Asp Ala Met Asp Tyr
   1 5 10
<210> 150
   <211> 13
   <212> PRT
   <213> Artificial Sequence
<220>
   <223> Synthetic peptide
<400> 150
   Arg Gln Leu Gly Asp Asn Val Ala Asp Ala Met Asp Tyr
   1 5 10
<210> 151
   <211> 13
   <212> PRT
   <213> Artificial Sequence
<220>
   <223> Synthetic peptide
<400> 151
   Arg Gln Leu Gly Met Ala Thr Pro Asp Ala Met Asp Tyr
   1               5                   10
<210> 152
   <211> 13
   <212> PRT
   <213> Artificial Sequence
<220>
   <223> Synthetic peptide
<400> 152
   Arg Gln Leu Gly Ala His Trp Leu Asp Ala Met Asp Tyr
   1               5                   10
<210> 153
   <211> 13
   <212> PRT
   <213> Artificial Sequence
<220>
   <223> Synthetic peptide
<400> 153
   Arg Gln Leu Gly Pro Glu Pro Gln Asp Ala Met Asp Tyr
   1               5                   10

## Description

### Background of the Invention

Alzheimer's Disease is a progressive and degenerative dementia (Terry, et al. (1991) Ann. Neurol. 30:572-580; Coyle (1987) In: Encyclopedia of Neuroscience, Adelman (ed.), Birkhäuser, Boston-Basel-Stuttgart, pp 29-31,). In its early stages, Alzheimer's Disease manifests primarily as a profound inability to form new memories (Selkoe (2002) Science 298:789-791), reportedly due to neurotoxins derived from amyloid beta (Aβ). Aβ is an amphipathic peptide whose abundance is increased by mutations and risk factors linked to Alzheimer's Disease. Fibrils formed from Aβ constitute the core of amyloid plaques, which are hallmarks of an Alzheimer's Disease brain. Analogous fibrils generated *in vitro* are lethal to cultured brain neurons. These findings indicate that memory loss is a consequence of neuron death caused by fibrillar Aβ.

Despite strong experimental support for fibrillar Aβ and memory loss, a poor correlation exists between dementia and amyloid plaque burden (Katzman (1988) Ann. Neurol. 23:138-144). Moreover, transgenic hAPP mice (Dodart, et al. (2002) Nat. Neurosci. 5:452-457; Kotilinek, et al. (2002) J. Neurosci. 22:6331-6335), which develop age-dependent amyloid plaques and, most importantly, age-dependent memory dysfunction, show that within 24 hours of vaccination with monoclonal antibodies against. Aβ memory loss can be reversed with no change in plaque levels. Such findings are not consistent with a mechanism for memory loss dependent on neuron death caused by amyloid fibrils.

Additional neurologically active molecules formed by Aβ self-assembly have been suggested. These molecules include soluble Aβ oligomers, also referred to as Aβ-derived diffusible ligands or ADDLs. Oligomers are amyloid plaques and, most importantly, age-dependent memory dysfunction, show that within 24 hours of vaccination with monoclonal, antibodies against Aβ memory loss can be reversed with no change in plaque levels. Such findings are not consistent with a mechanism for memory loss dependent on neuron death caused by amyloid fibrils.

Additional neurologically active molecules formed by Aβ self-assembly have been suggested. These molecules include soluble Aβ oligomers, also referred to as Aβ-derived diffusible ligands or ADDLs. Oligomers are metastable and form at low concentrations of Aβ1-42 (Lambert, et al. (1998) Proc. Natl. Acad. Sci. USA 95:6448-6453). Aβ oligomers rapidly inhibit long-term potentiation (LTP), a classic experimental paradigm for memory and synaptic plasticity. As such, memory loss stems from synapse failure, prior to neuron death and synapse failure by Aβ oligomers, not fibrils (Hardy & Selkoe (2002) Science 297:353-356). Soluble oligomers have been found in brain tissue and are strikingly elevated in Alzheimer's Disease (Kayed, et al. (2003) Science 300:486-489; Gong, et al. (2003) Proc. Natl. Acad. Sci. USA 100:10417-10422) and in hAPP transgenic mice Alzheimer's Disease models (Kotilinek, et al. (2002) J. Neurosci. 22:6331-6335; Chang, et al. (2003) J. Mol. Neurosci . 20:305-313).

WO 03104437 comprises antibodies that bind to amyloid beta-derived diffusible ligands (ADDLs). ADDLs comprise amyloid ß protein assembled into soluble, globular, non-fibrillar, oligomeric structures capable of activating specific cellular processes. WO 03104437 also comprises methods of using ADDL-specific antibodies for assaying the formation, presence, receptor protein binding and cellular activity of ADDLs, as well as using such antibodies to detect compounds that block the formation or activity of ADDLs, and methods of identifying such compounds. WO 03104437 further provides methods of using ADDL-specific antibodies in modulating ADDL formation and/or activity, *inter alia* in the treatment of learning and/or memory disorders.

A variety of Alzheimer's Disease treatment options have been suggested. Vaccine clinical trials have revealed that persons mounting a vigorous immune response to the vaccine exhibit cognitive benefit (Hock, et al. (2003) Neuron 38:547-554); however, frequency of CNS inflammation caused early termination of part of the trial (Birmingham & Frantz (2002) Nat. Med. 8:199-200) . As an alternative to a vaccine, therapeutic antibodies that target ADDLs without binding monomers or fibrils have been suggested (Klein (2002) Neurochem. Int. 41:345-352) . ADDLS are highly antigenic, generating oligomer-selective polyclonal antibodies in rabbits at concentration of ~50 µg/mL (Lambert, et al. (2001) J. Neurochem. 79 : 595-605) . Results from transgenic mice models also suggest that antibodies can be successful in reversing memory decline (Dodart, et al. (2002) Nat. Neurosci. 5:452-457; U.S. Patent Application Serial No. 11/194,989). Accordingly, there is a need in the art for ADDL-selective therapeutic antibodies for the prevention and treatment of Alzheimer's Disease . The present invention meets this need.

### Summary of the Invention

The present invention is an isolated antibody, or fragment thereof, capable of differentially recognizing a multi-dimensional conformation of one or more Aβ-derived diffusible ligands. In particular, the antibody of the instant invention has a complementary determining region (CDRs) of Arg-Xaa₁-Leu-Xaa₂-Xaa₃-Xaa₄ Xaa₅-Xaa₆-Asp-Ala-Met-Asp-Tyr (SEQ ID NO:9), wherein Xaa₁ is Gln or Ala; Xaa₂ is Ser or Gly; Xaa₃ is Pro, Ala, Lys, Arg, or Thr; Xaa₄ is Lys or Arg; Xaa₅ is Gly, Ser, or Lys; Xaa₆ is Val, Thr, Ile or Arg. In particular embodiments, the antibody of the present invention is in admixture with a pharmaceutically acceptable carrier. In other embodiments, the antibody of the present invention is in a kit. Still other embodiments embrace an antibody having heavy and light chain variable region sequences as set forth in SEQ ID NO:108 and SEQ ID NO:112. An antibody having heavy and light chain sequences as set forth in SEQ ID NO:138 and SEQ ID NO:140 is also provided.

Methods for preventing binding of Aβ-derived diffusible ligands to a neuron and inhibiting assembly of Aβ-derived diffusible ligands employing an antibody or antibody fragment which binds a multi-dimensional conformation of one or more Aβ-derived diffusible ligands are also provided.

The present invention further embraces a method for prophylactically or therapeutically treating a disease associated with Aβ-derived diffusible ligands using an antibody of the instant invention. Administration of an antibody of the invention can prevent binding of Aβ-derived diffusible ligands to a neuron thereby preventing or treating the disease associated with Aβ-derived diffusible ligands.

The present invention is also a method for identifying a therapeutic agent that prevents the binding of Aβ-derived diffusible ligands to a neuron. This method of the invention involves contacting a neuron with Aβ-derived diffusible ligands in the presence of an agent and using an antibody of the present invention to determine binding of the Aβ-derived diffusible ligands to the neuron in the presence of the agent.

The present invention also embraces a method for detecting Aβ-derived diffusible ligands in a sample and a method for diagnosing a disease associated with Aβ-derived diffusible ligands. Such methods involve contacting a sample with an antibody of the instant invention so that the Aβ-derived diffusible ligands can be detected and a disease associated with Aβ-derived diffusible ligands can be diagnosed.

### Brief Description of the Drawings

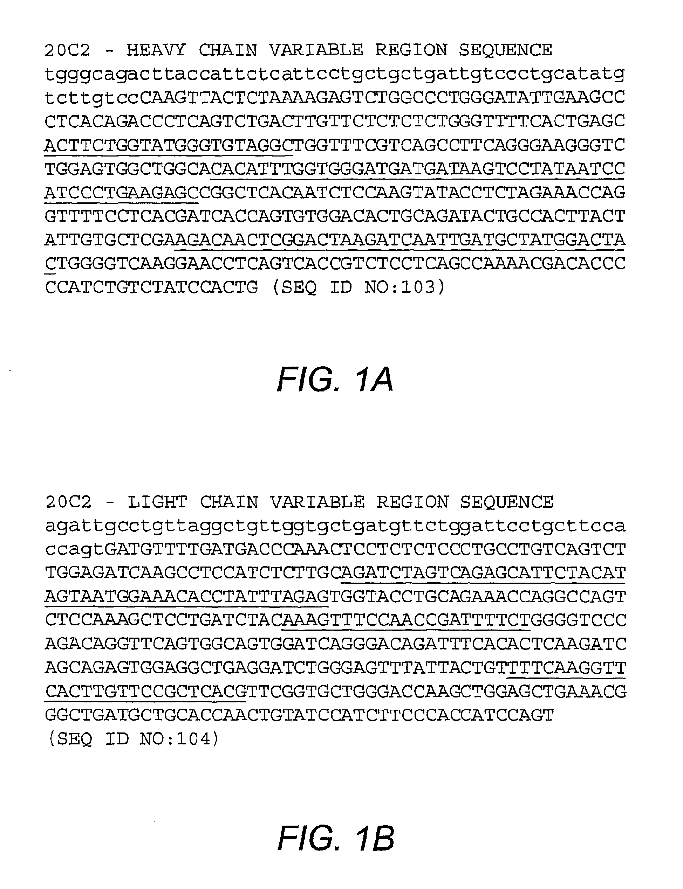

Figure 1 shows the nucleic acid sequences for the heavy (Figure 1A) and light (Figure 1B) chain variable regions for murine anti-ADDL antibody 20C2. Lower case letters indicate the antibody leader sequences and uppercase letters indicate antibody variable region sequences, The nucleotides coding for the complementary determining regions (CDRs) are underlined.

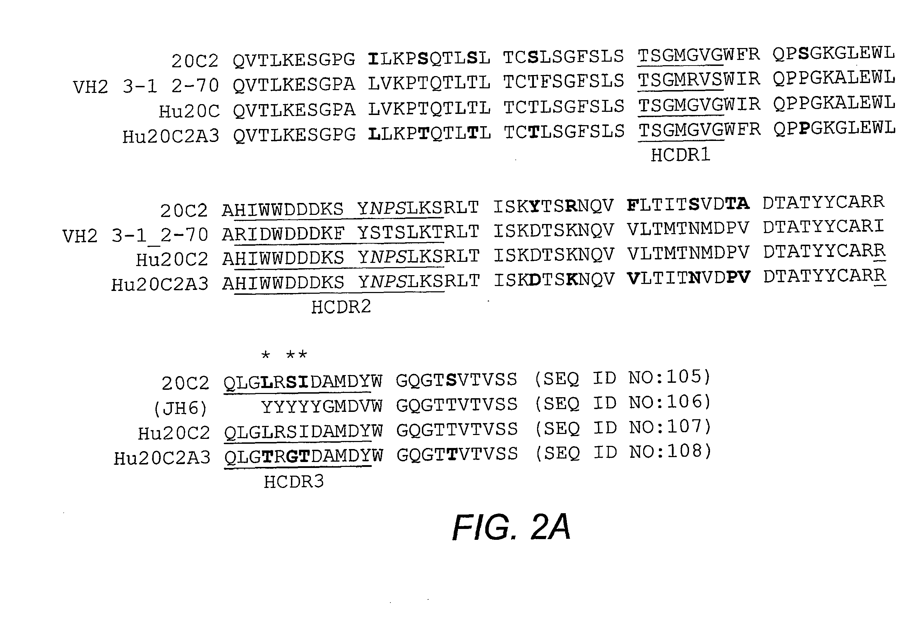

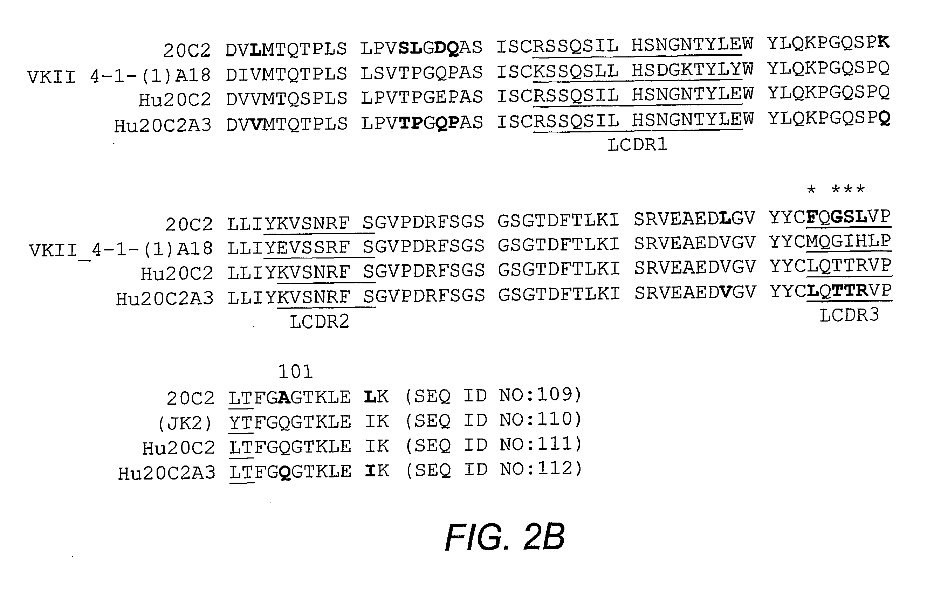

Figure 2 shows comparisons of heavy (Figure 2A) and light (Figure 2B) chain variable region amino acid sequences of murine antibody 20C2 and humanized antibodies, Hu20C2 (CDR grafted) and Hu20C2A3 (veneered). Sequences are presented as comparisons between the 20C2 mouse sequence, the most homologous human genomic sequence and the humanized sequences. Sequence differences in the frame regions between murine 20C2 and humanized Hu20C2A3 are in bold. Sequence differences in the underlined CDR regions between humanized Hu20C2A3 and murine 20C2 are in bold and indicated with an *. CDRs are underlined.

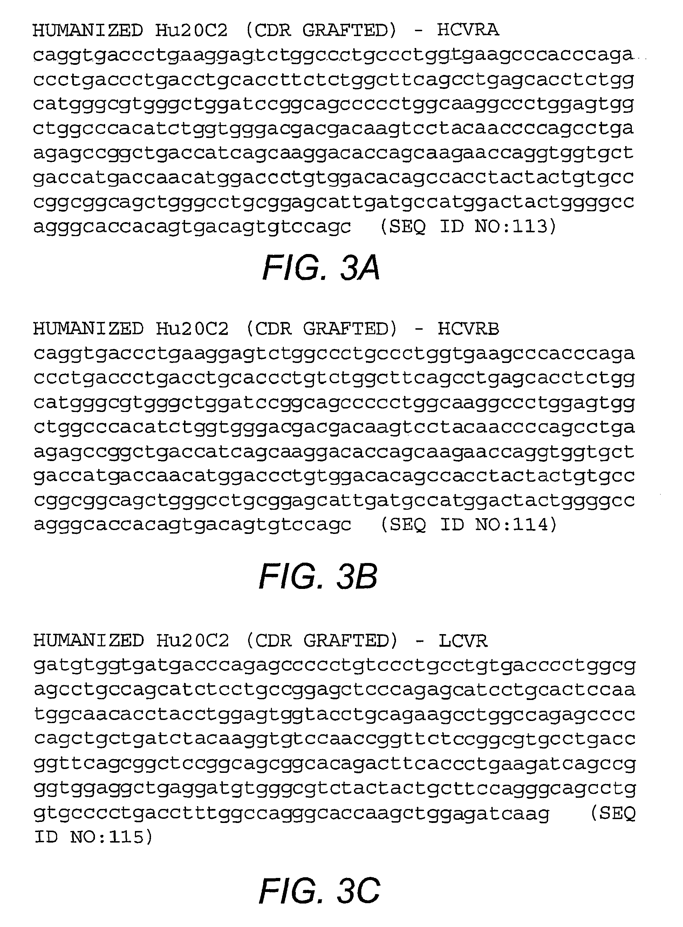

Figure 3 shows nucleic acid sequences for the heavy (Figures 3A and 3B) and light (Figure 3C) chain variable regions (HCVRs and LCVRs, respectively) for humanized anti-ADDL antibody Hu20C2 (CDR grafted). Two humanized versions of the Hu20C2 heavy chain were generated (HCVRA and HCVRB) that differ by one amino acid at position 24. In Hu20C2 HCVRA the human amino acid was used and in Hu20C2 HCVRB the mouse amino acid was used. Variable region sequences were cloned into full heavy and light chain antibody expression vectors.

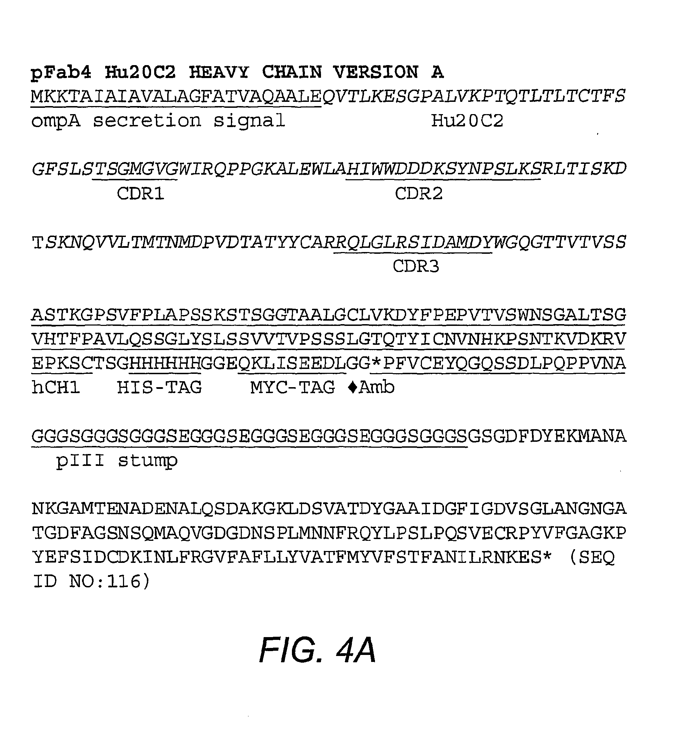

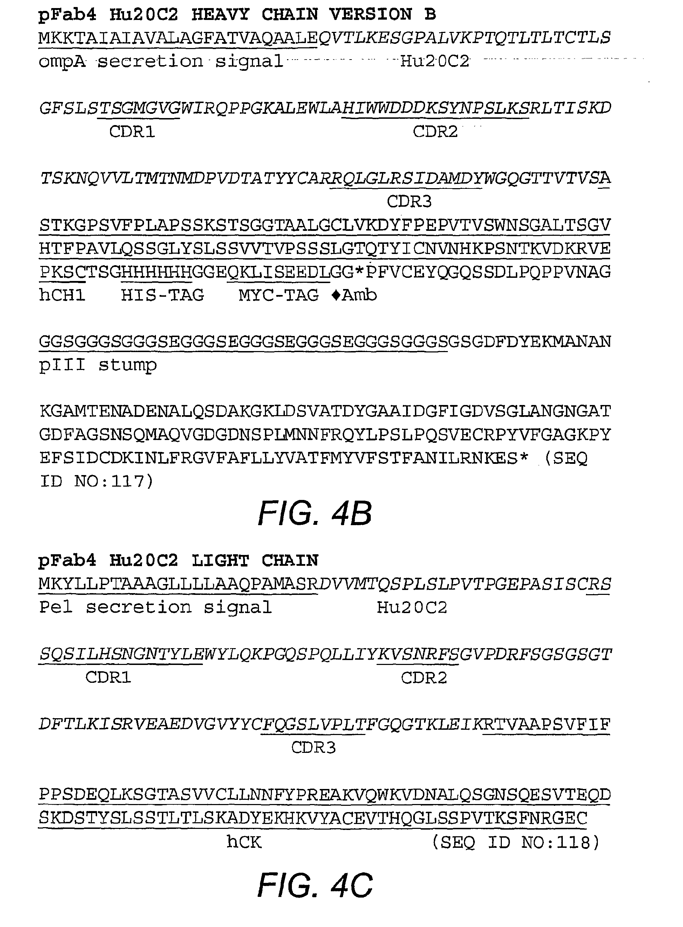

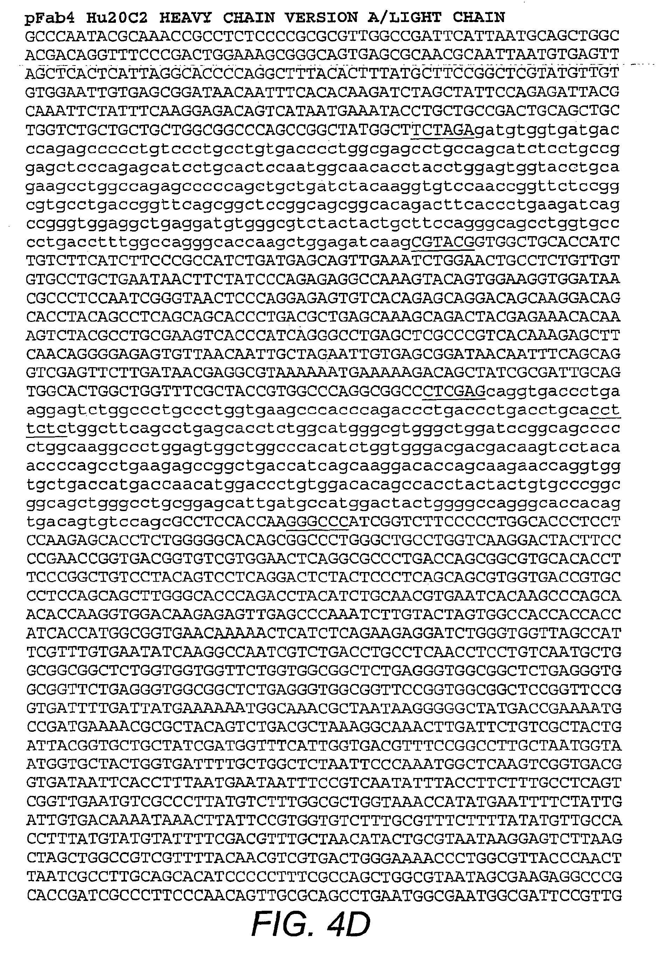

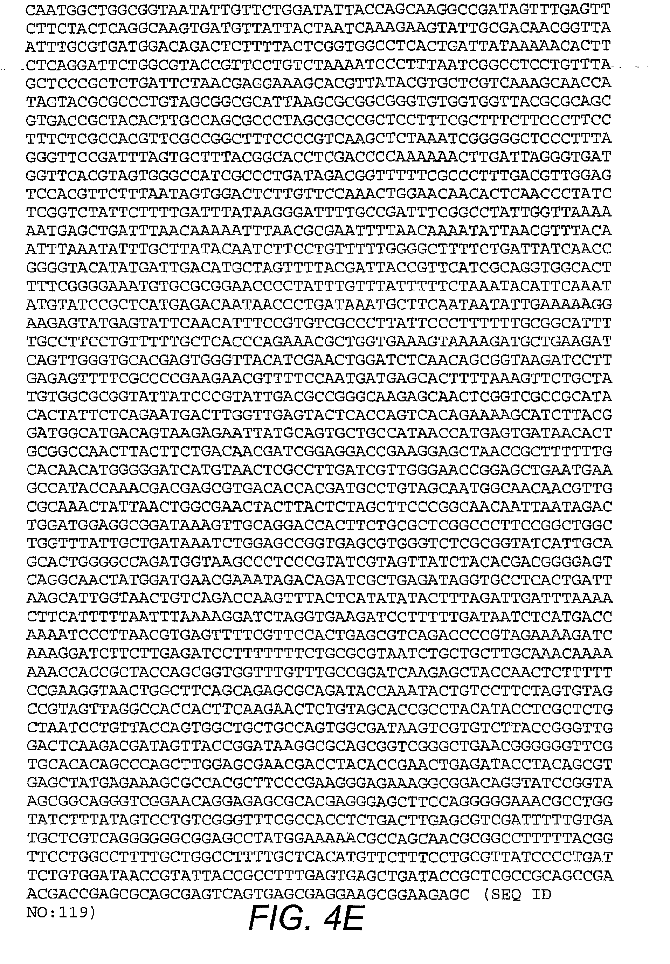

Figure 4 shows the annotated amino acid sequences and nucleotide sequences of Hu20C2 humanized antibody in Fab phage-display vector pFab4. Amino acid sequence for heavy chain version A (Figure 4A), heavy chain version B (Figure 4B), and the light chain (Figure 4C) of Hu20C2 humanized antibody in Fab phage-display vector pFab4 are in italic and underlined regions are as indicated. Nucleotide sequence of heavy chain version A fused with the light chain of Hu20C2 in pFab4 vector is shown in Figure 4D-4E with sequences encoding the Hu20C2 antibody sequences shown in lowercase.

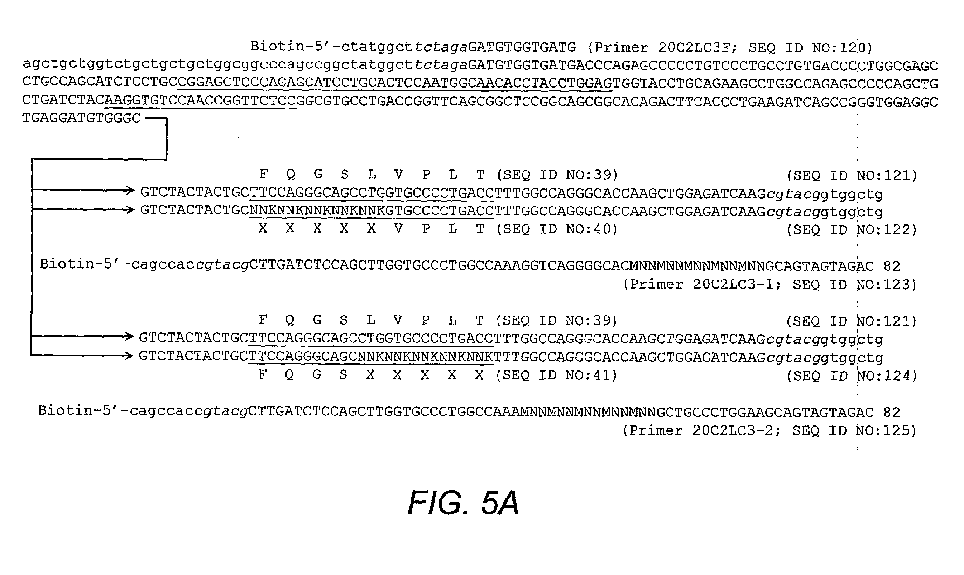

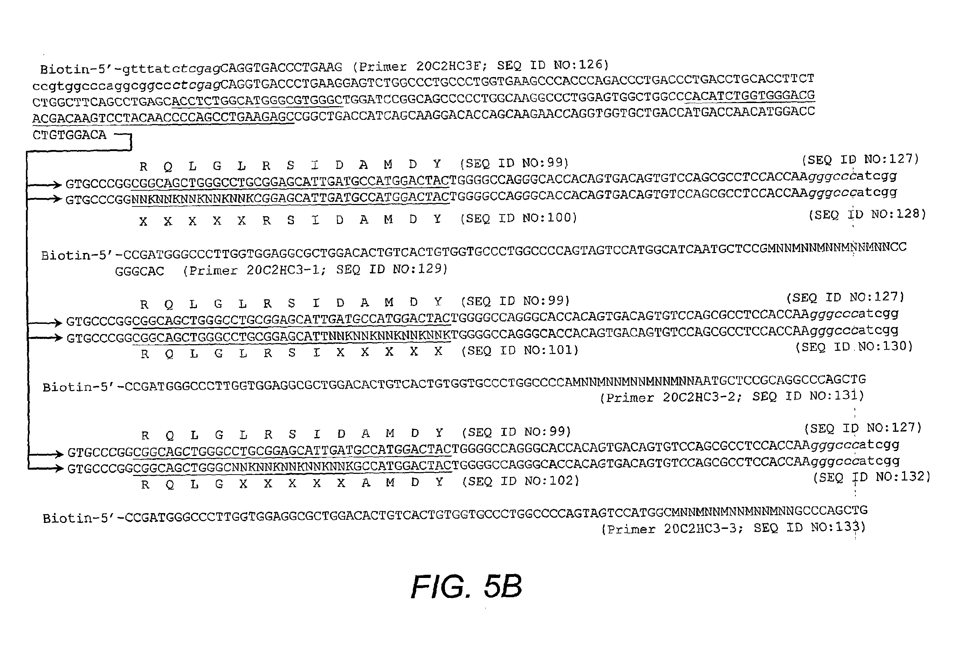

Figure 5 depicts the design and primers employed in preparing two light chain CDR3 libraries, namely LC3-1 and LC3-2 (Figure 5A), and three heavy chain CDR3 libraries, namely 20C2B-39HC₃-1, 20C2B-39HC₃-2, and 20C2B-39HC₃-3 (Figure 5B), for respectively generating affinity matured Hu20C2 light and heavy chain CDR3s. Restriction endonuclease recognition sites used for cloning are indicated in italic. Uppercase indicates nucleic acids encoding antibody variable region sequences. Nucleic acids encoding CDRs are underlined. Biotin-labeled primers are indicated.

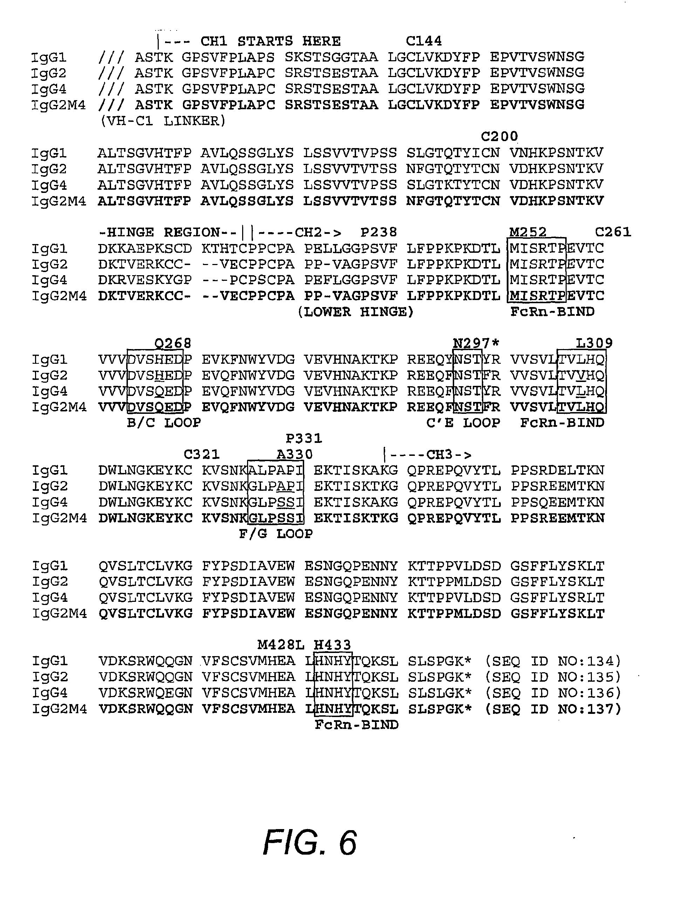

Figure 6 shows a comparison of the amino acid sequence of human antibody constant regions and the sequence of IgG2m4. The asterisk indicates a glycosylation site at Asn297. Regions of FcRn binding are indicated. Sequences in which IgG2m4 is different from IgG2 are underlined.

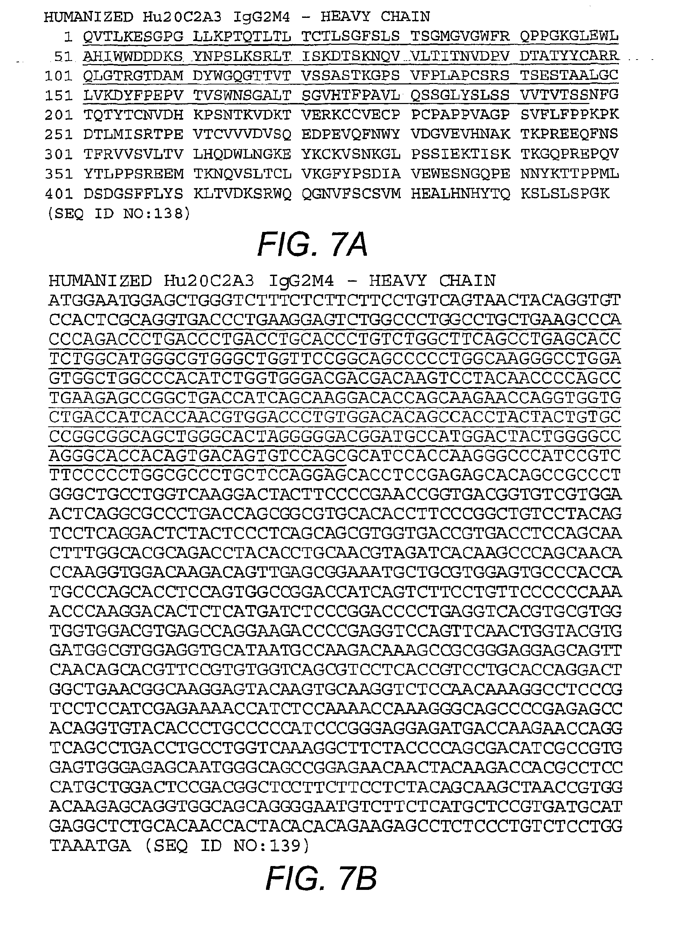

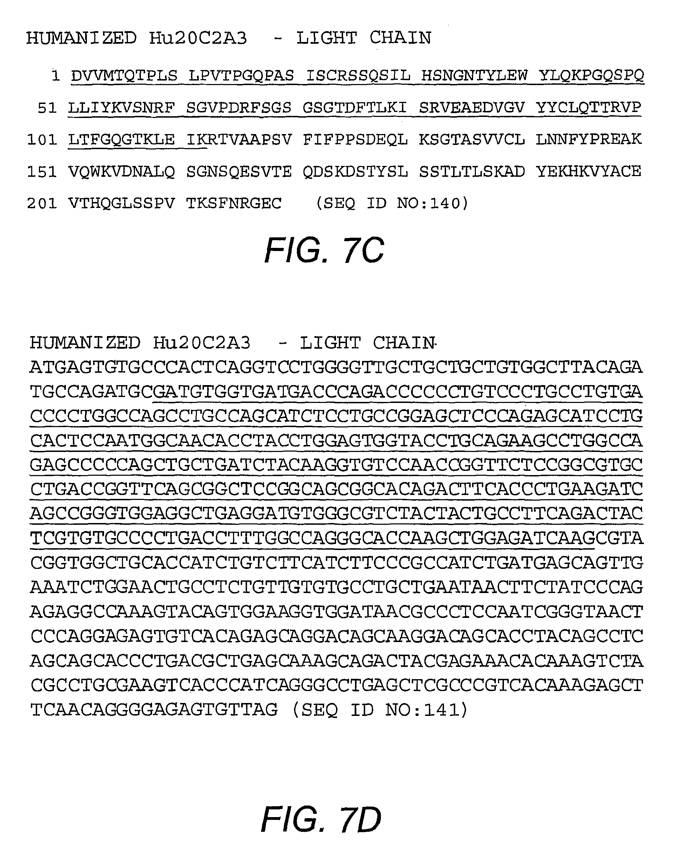

Figure 7 shows the amino acid (Figures 7A and 7C) and nucleotide (Figures 7B and 7D) sequences for the full IgG2m4 humanized heavy chain (Figures 7A and 7B) and humanized Kappa light chain (Figures 7C and 7D) for anti-ADDL antibody Hu20C2A3. Underlining indicates variable region sequences. The remaining sequences are constant region sequences.

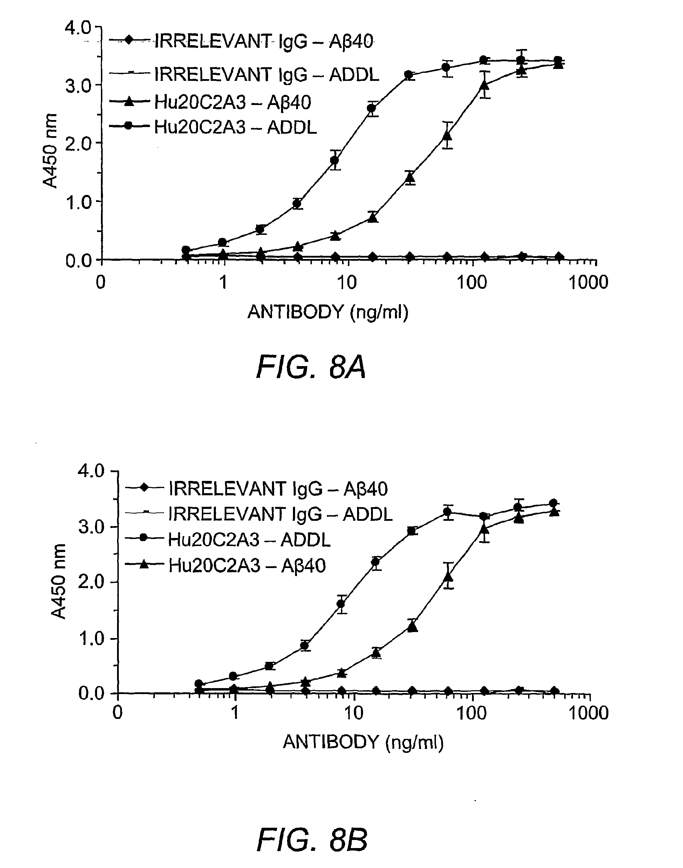

Figure 8 shows interactions between Aβ40 monomer or ADDLs with Hu20C2A3 produced by two different systems, CHO (Figure 8A) or *Pichia* (Figure 8B), as determined by ELISA.

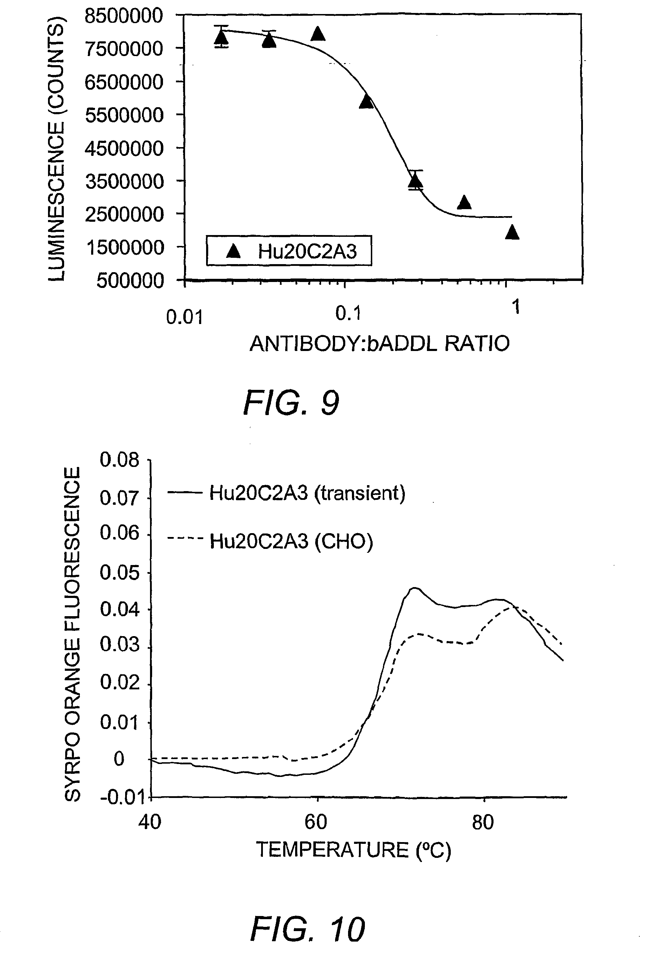

Figure 9 shows Hu20C2A3 inhibition of bADDL binding to primary hippocampal neurons.
Figure 10 shows fluorescent thermal melt analysis of Hu20C2A3.

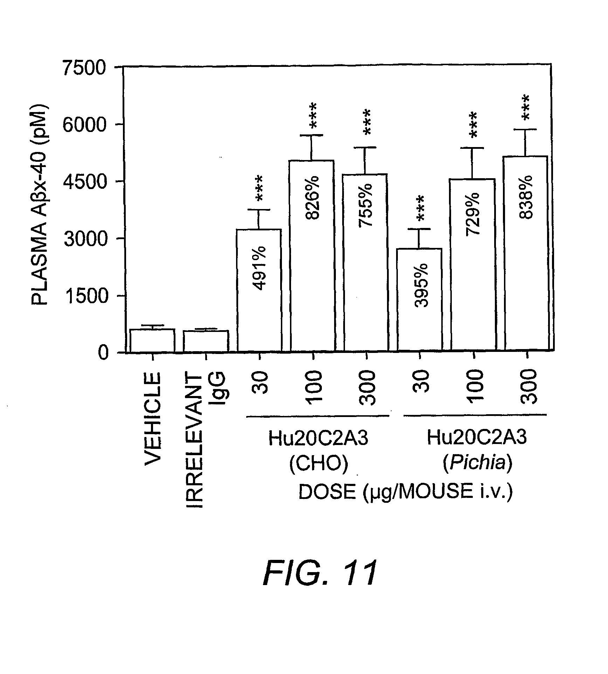

Figure 11 shows plasma Aβx-40 levels (pM) of APP-YAC mice following intravenous injection of Hu20C2A3, irrelevant control or vehicle. Hu20C2A3 was prepared by stable transfection of CHO cells or *Pichia.* Aβx-40 was determined using a 4G8/G2-10 ELISA. ***, *p*<0.001 by Tukey-Kramer HSD post-hoc testing. Error bars = SEM; N=6/group.

### Detailed Description of the Invention

Monoclonal antibodies, which differentially recognize multi-dimensional conformations of Aβ-derived diffusible ligands (i.e., ADDLs), have now been generated. Antibodies of this invention are derived from the murine monoclonal antibody 20C2. Murine 20C2 is known in the art for exhibiting the following characteristics. Murine 20C2 is an IgG1 antibody which binds to both synthetic and endogenous ADDLs bound to cultured hippocampal cells. Furthermore, this antibody can block both endogenous and synthetic ADDL binding to cultured cells, abate the binding of biotinylated ADDLs (bADDLs) to neurons, and prevent tau phosphorylation. The core linear epitope for 20C2 is Glu-Phe-Arg-His-Asp-Ser (SEQ ID NO:1), corresponding to amino acid residues 3-8 of Aβ1-42, with a conformational epitope that is dependent upon elements from within residues 17-42 of Aβ, but only when assembled.

The instant antibodies are humanized and, in some embodiments affinity-matured derivatives of murine 20C2. Like the murine 20C2 antibody, the antibodies disclosed herein exhibit a high degree of selectivity for multi-dimensional conformations of ADDLs, with minimal detection of monomer Aβ peptides. Advantageously, the instant antibodies identify endogenous oligomers in Alzheimer's Disease brain slices and inhibit binding of bADDLs to neurons. Moreover, the instant antibodies provide a significant and robust increase in plasma Aβx-40 levels, an increase in which is known to be associated with an ultimate lowering of brain Aβ. Accordingly, the antibodies of this invention find use in the prevention of ADDL binding to neurons and assembly of ADDLs and the treatment of ADDL-related diseases including Alzheimer's Disease.

Accordingly, the present invention is an isolated antibody that differentially recognizes one or more multi-dimensional conformations of ADDLs. An antibody of the instant invention is said to be isolated when it is present in the substantial absence of other biological macromolecules of the same type. Thus, an "isolated antibody" refers to an antibody which is substantially free of other antibodies; however, the molecule may include some additional agents or moieties which do not deleteriously affect the basic characteristics of the antibody (e.g., binding specificity, neutralizing activity, etc.).

An antibody which is capable of specifically binding one or more multi-dimensional conformations of ADDLs, binds particular ADDLs derived from the oligomerization of Aβ1-42, but like murine 20C2 does not cross-react with other Aβ peptides, namely Aβ1-12, Aβ1**-**28, Aβ1-40, and Aβ12-28 as determined by western blot analyses; and preferentially bind ADDLs in solution. Specific binding between two entities generally refers to an affinity of at least 10⁶, 10⁷, 10⁸, 10⁹, or 10¹⁰ M⁻¹. Affinities greater than 10⁸ M⁻¹ are desired to achieve specific binding.

In particular embodiments, an antibody that is capable of specifically binding a multi-dimensional conformation of one or more ADDLs is also raised against (i.e., an animal is immunized with) multi-dimensional conformations of ADDLs. In other embodiments, an antibody that is capable of specifically binding a multi-dimensional conformation of one or more ADDLs is raised against a low n-mer-forming peptide such as Aβ1-42[Nle35-Dpro37].

The term "epitope" refers to a site on an antigen to which B and/or T cells respond or a site on a molecule against which an antibody will be produced and/or to which an antibody will bind. For example, an epitope can be recognized by an antibody defining the epitope.

A linear epitope is an epitope wherein an amino acid primary sequence comprises the epitope recognized. A linear epitope typically includes at least 3, and more usually, at least 5, for example, about 6 to about 10 amino acids in a unique sequence.

A conformational epitope, in contrast to a linear epitope, is an epitope wherein the primary sequence of the amino acids comprising the epitope is not the sole defining component of the epitope recognized (e.g., an epitope wherein the primary sequence of amino acids is not necessarily recognized by the antibody defining the epitope). Typically a conformational epitope encompasses an increased number of amino acids relative to a linear epitope. With regard to recognition of conformational epitopes, the antibody recognizes a three-dimensional structure of the peptide or protein. For example, when a protein molecule folds to form a three-dimensional structure, certain amino acids and/or the polypeptide backbone forming the conformational epitope become juxtaposed enabling the antibody to recognize the epitope. Methods of determining conformation of epitopes include but are not limited to, for example, x-ray crystallography, two-dimensional nuclear magnetic resonance spectroscopy and site-directed spin labeling and electron paramagnetic resonance spectroscopy. See, for example, Epitope Mapping Protocols in Methods in Molecular Biology (1996) Vol. 66, Morris (Ed.).

Aβ**-**derived diffusible ligands or ADDLs refer to soluble oligomers of amyloid β1-42 which are desirably composed of aggregates of less than eight or nine amyloid β1-42 peptides and are found associated with Alzheimer's Disease. This is in contrast to high molecular weight aggregation intermediates, which form stings of micelles leading to fibril formation.

As exemplified herein, the instant antibody binds or recognizes at least one multi-dimensional conformation of an ADDL. In particular embodiments, the instant antibody binds at least two, at least three, or at least four multi-dimensional conformations of an ADDL. Multi-dimensional conformations of ADDLs are intended to encompass dimers, trimers, tetramers pentamers, hexamers, heptamers, octamers, nonamers, decamers, etc. as defined by analysis via SDS-PAGE. Because trimer, tetramer, etc. designations can vary with the assay method employed (see, e.g., Bitan, et al. (2005) Amyloid 12:88-95) the definition of trimer, tetramer, and the like, as used herein, is according to SDS-PAGE analysis. As such, the antibody of the instant invention has oligomer-specific characteristics. In particular embodiments, a multi-dimensional conformation of an ADDL is associated with a specific polypeptide structure which results in a conformational epitope that is recognized by an antibody of the present invention. In other embodiments, an antibody of the invention specifically binds a multi-dimensional conformation ADDL having a size range of approximately a trimer or tetramer, which have molecular weights in excess of >50 kDa.

In certain embodiments, in addition to binding to a multi-dimensional conformation, the instant antibody binds to a selected linear epitope of amyloid β1-42. A linear epitope of an ADDLs is intended as a four, five, six or more amino acid residue peptide located in the N-terminal 10, 11, 12, 15 or 20 amino acid residues of amyloid β1-42. In particular embodiments, an antibody of the invention specifically binds to a linear epitope within residues 1-10, 1-8, 3-10, or 3-8 of amyloid β1-42. An exemplary linear epitope of amyloid β1-42 which is bound by a humanized antibody of the invention is amino acid residues Glu-Phe-Arg-His-Asp-Ser (SEQ ID NO:1).

While antibodies of the instant invention may have similar linear epitopes, such linear epitopes are not wholly indicative of the binding characteristics of the instant antibodies (i.e., ability to block ADDL binding to neurons, prevent tau phosphorylation and inhibit ADDL assembly) because, as is well-known to the skilled artisan, the linear epitope may only correspond to a portion of the antigen's epitope (see, e.g., Breitling and Dübel (1999) In: Recombinant Antibodies, John Wiley & Sons, Inc., NY, pg. 115). For example, murine 20C2 is known to bind assemblies of charge-inverted, truncated Aβ7-42 peptide, which lack the linear epitope for 20C2 (i.e., amino acid residues 3-8) and contain a very different sequence corresponding to residues 7-16 of Aβ. Therefore, 20C2, as well as humanized derivatives thereof, bind to conformational epitopes that depend upon elements from within residues 17-42 of Aβ, but only when in a multidimensional conformation. The antibody of the instant invention can be distinguished from those of the art as being capable of differentially recognizing multi-dimensional ADDLs and accordingly differentially blocking ADDL binding to neurons, differentially preventing tau phosphorylation and differentially inhibiting ADDL assembly.

An antibody, as used in accordance with the instant invention includes, but is not be limited to, monoclonal antibodies, and chimeric, human (e.g. isolated from B cells), humanized, neutralizing, bispecific or single chain antibodies thereof. In one embodiment, an antibody of the instant invention is monoclonal. For the production of antibodies, various hosts including goats, rabbits, chickens, rats, mice, humans, and others, can be immunized by injection with synthetic or natural ADDLs. Methods for producing antibodies are well-known in the art. See, e.g., Kohler and Milstein ((1975) Nature 256:495-497) and Harlow and Lane (Antibodies: A Laboratory Manual (Cold Spring Harbor Laboratory, New York (1988)).

Depending on the host species, various adjuvants can be used to increase the immunological response. Adjuvants used in accordance with the instant invention desirably augment the intrinsic response to ADDLs without causing conformational changes in the immunogen that affect the qualitative form of the response. Particularly suitable adjuvants include 3 De-O-acylated monophosphoryl lipid A (MPL™; RIBI ImmunoChem Research Inc., Hamilton, MT; see GB 2220211) and oil-in-water emulsions, such as squalene or peanut oil, optionally in combination with immune stimulants, such as monophosphoryl lipid A (see Stoute, et al. (1997) N. Engl. J. Med. 336:86-91), muramyl peptides (e.g., N-acetylmuramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'dipalmitoyl-sn-glycero-3-hydroxyphosphoryloxy)-ethylamine (E-PE), N-acetylglucsaminyl-N-acetylmuramyl-L-Al-D-isoglu-L-Ala-dipalmitoxy propylamide (DTP-DPP)), or other bacterial cell wall components. Specific examples of oil-in-water emulsions include MF59 (WO 90/14837), containing 5% Squalene, 0.5% TWEEN™ 80, and 0.5% SPAN 85 (optionally containing various amounts of MTP-PE) formulated into submicron particles using a microfluidizer such as Model 110Y microfluidizer (Microfluidics, Newton, MA); SAF containing 10% Squalene, 0.4% TWEEN™ 80, 5% PLURONIC^{®}-blocked polymer L121, and thr-MDP, either microfluidized into a submicron emulsion or vortexed to generate a larger particle size emulsion; and RIBI™ adjuvant system (RAS) (Ribi ImmunoChem, Hamilton, MT) containing 2% squalene, 0.2% TWEEN™ 80, and one or more bacterial cell wall components such as monophosphoryllipid A, trehalose dimycolate (TDM), and cell wall skeleton (CWS).

Another class of adjuvants is saponin adjuvants, including ISCOMs (immunostimulating complexes) and ISCOMATRIX^{®} (CSL Ltd., Parkville, Australia). Other suitable adjuvants include Complete Freund's Adjuvant (CFA), Incomplete Freund's Adjuvant (IFA), mineral gels such as aluminum hydroxide, and surface-active substances such as lysolecithin, PLURONIC^{®} polyols, polyanions, peptides, CpG (WO 98/40100), keyhole limpet hemocyanin, dinitrophenol, and cytokines such as interleukins (IL-1, IL-2, and IL-12), macrophage colony stimulating factor (M-CSF), and tumor necrosis factor (TNF). Among adjuvants used in humans, BCG (bacilli Calmette-Guerin) and *Corynebacterium parvum* are particularly suitable.

An antibody to a multi-dimensional conformation ADDL is generated by immunizing an animal with ADDLs. Generally, ADDLs can be generated synthetically or by recombinant fragment expression and purification. Synthetic ADDLs can be prepared as disclosed herein or in accordance with the methods disclosed in U.S. Patent No. 6,218,506 or in copending applications US Serial Nos. 60/621,776, 60/652,538, 60/695,528 and 60/695,526. Further, ADDLs can be fused with another protein, such as keyhole limpest hemocyanin to generate an antibody against the chimeric molecule. The ADDLs can be conformationally constrained to form an epitope useful as described herein and furthermore can be associated with a surface for example, physically attached or chemically bonded to a surface in such a manner so as to allow for the production of a conformation which is recognized by the antibodies of the present invention.

Monoclonal antibodies to multi-dimensional conformations of ADDLs can be prepared using any technique which provides for the production of antibody molecules by continuous cell lines in culture. These include, but are not limited to, the hybridoma technique, the human B-cell hybridoma technique, and the EBV-hybridoma technique (Kohler, et al. (1975) Nature 256:495-497; Kozbor, et al. (1985) J. Immunol. Methods 81:31-42; Cote, et al. (1983) Proc. Natl. Acad. Sci. 80:2026-2030; Cole, et al. (1984) Mol. Cell Biol. 62:109-120).

In particular embodiments, the instant antibodies are humanized. Humanized or chimeric antibodies can be produced by splicing of mouse antibody genes to human antibody genes to obtain a molecule with appropriate antigen specificity and biological activity (see Morrison, et al. (1984) Proc. Natl. Acad. Sci. 81, 6851-6855; Neuberger, et al. (1984) Nature 312:604-608; Takeda, et al. (1985) Nature 314:452-454; Queen, et al. (1989) Proc. Natl. Acad. Sci. USA 86:10029-10033; WO 90/07861). For example, a mouse antibody is expressed as the Fv or Fab fragment in a phage selection vector. The gene for the light chain (and in a parallel experiment, the gene for the heavy chain) is exchanged for a library of human antibody genes. Phage antibodies, which still bind the antigen, are then identified. This method, commonly known as chain shuffling, provided humanized antibodies that should bind the same epitope as the mouse antibody from which it descends (Jespers, et al. (1994) Biotechnology NY 12:899-903). As an alternative, chain shuffling can be performed at the protein level (see, Figini, et al. (1994) J. Mol. Biol. 239:68-78).

Human antibodies can also be obtained using phage-display methods. See, e.g., WO 91/17271 and WO 92/01047. In these methods, libraries of phage are produced in which members display different antibodies on their outer surfaces. Antibodies are usually displayed as Fv or Fab fragments. Phage displaying antibodies with a desired specificity are selected by affinity enrichment to ADDLs. Human antibodies against ADDLs can also be produced from non-human transgenic mammals having transgenes encoding at least a segment of the human immunoglobulin locus and an inactivated endogenous immunoglobulin locus. See, *e.g.,* WO 93/12227 and WO 91/10741. Human antibodies can be selected by competitive binding experiments, or otherwise, to have the same epitope specificity as a particular mouse antibody. Such antibodies generally retain the useful functional properties of the mouse antibodies. Human polyclonal antibodies can also be provided in the form of serum from humans immunized with an immunogenic agent. Optionally, such polyclonal antibodies can be concentrated by affinity purification using ADDLs as an affinity reagent.

As exemplified herein, humanized antibodies can also be produced by veneering or resurfacing of murine antibodies. Veneering involves replacing only the surface fixed region amino acids in the mouse heavy and light variable regions with those of a homologous human antibody sequence. Replacing mouse surface amino acids with human residues in the same position from a homologous human sequence has been shown to reduce the immunogenicity of the mouse antibody while preserving its ligand binding. The replacement of exterior residues generally has little, or no, effect on the interior domains, or on the interdomain contacts. (See, e.g., U.S. Patent No. 6,797,492).

Human or humanized antibodies can be designed to have IgG, IgD, IgA, IgM or IgE constant regions, and any isotype, including IgG1, IgG2, IgG3 and IgG4. In particular embodiments, an antibody of the invention is IgG or IgM, or a combination thereof. Other embodiments of the present invention embrace a constant region formed by selective incorporation of human IgG4 sequences into a standard human IgG2 constant region. An exemplary mutant IgG2 Fc is IgG2m4, set forth herein as SEQ ID NO:140. Antibodies can be expressed as tetramers containing two light and two heavy chains, as separate heavy chains and light chains or as single chain antibodies in which heavy and light chain variable domains are linked through a spacer. Techniques for the production of single chain antibodies are well-known in the art.

Exemplary humanized antibodies derivatives of murine 20C2 monoclonal antibody are provided herein by CDR grafting and veneering. Amino acid sequences for IgG2M4 heavy chain variable regions, as well as kappa light chain variable regions for humanized 20C2 (i.e., Hu20C2A3) generated by veneering are presented in Figures 7A and 7C and set forth herein as SEQ ID NO: 138 and SEQ ID NO: 140.

Diabodies are also contemplated. A diabody refers to an engineered antibody construct prepared by isolating the binding domains (both heavy and light chain) of a binding antibody, and supplying a linking moiety which joins or operably links the heavy and light chains on the same polypeptide chain thereby preserving the binding function (see, Holliger et al. (1993) Proc. Natl. Acad. Sci. USA 90:6444; Poljak (1994) Structure 2:1121-1123). This forms, in essence, a radically abbreviated antibody, having only the variable domain necessary for binding the antigen. By using a linker that is too short to allow pairing between the two domains on the same chain, the domains are forced to pair with the complementary domains of another chain and create two antigen-binding sites. These dimeric antibody fragments, or diabodies, are bivalent and bispecific. The skilled artisan will appreciate that any method to generate diabodies can be used. Suitable methods are described by Holliger, et al. (1993) *supra,* Poljak (1994) *supra,* Zhu, et al. (1996) Biotechnology 14:192-196, and U.S. Patent No. 6,492,123, incorporated herein by reference.

Fragments of an isolated antibody of the invention are also expressly encompassed by the instant invention. Fragments are intended to include Fab fragments, F(ab')₂ fragments, F(ab') fragments, bispecific scFv fragments, Fd fragments and fragments produced by a Fab expression library, as well as peptide aptamers. For example, F(ab')₂ fragments are produced by pepsin digestion of the antibody molecule of the invention, whereas Fab fragments are generated by reducing the disulfide bridges of the F(ab')₂ fragments. Alternatively, Fab expression libraries can be constructed to allow rapid and easy identification of monoclonal Fab fragments with the desired specificity (see Huse, et al. (1989) Science 254:1275-1281). In particular embodiments, antibody fragments of the present invention are fragments of neutralizing antibodies which retain the variable region binding site thereof. Exemplary are F(ab')₂ fragments, F(ab') fragments, and Fab fragments. See generally Immunology: Basic Processes (1985) 2nd edition, J. Bellanti (Ed.) pp. 95-97.

Peptide aptamers which differentially recognize multi dimensional conformations of ADDLs can be rationally designed or screened for in a library of aptamers (*e.g.,* provided by Aptanomics SA, Lyon, France). In general, peptide aptamers are synthetic recognition molecules whose design is based on the structure of antibodies. Peptide aptamers consist of a variable peptide loop attached at both ends to a protein scaffold. This double structural constraint greatly increases the binding affinity of the peptide aptamer to levels comparable to that of an antibody (nanomolar range).

Exemplary nucleic acid sequences encoding heavy and light chain variable regions for use in producing antibody and antibody fragments of the instant invention are respectively disclosed herein in Figures 7B and 7D (i.e., SEQ ID NOs:142 and 144). As will be appreciated by the skilled artisan, the heavy chain variable regions disclosed herein can be used in combination with any one of the light chain variable regions disclosed herein to generate antibodies with modified affinities, dissociate constants, epitopes and the like.

Antibodies or antibody fragments of the present invention can have additional moieties attached thereto. For example, a microsphere or microparticle can be attached to the antibody or antibody fragment, as described in U.S. Patent No. 4,493,825, the disclosure of which is incorporated herein by reference.

Moreover, particular embodiment embrace antibody or antibody fragments which are mutated and selected for increased antigen affinity, neutralizing activity (i.e., the ability to block binding of ADDLs to neuronal cells or the ability to block ADDL assembly), or a modified dissociation constant. Mutator strains of E. coli (Low, et al. (1996). J. Mol. Biol. 260:359-368) *;* chain shuffling (Figini, et al. (1994) *supra),* and PCR mutagenesis are established methods for mutating nucleic acid molecules encoding antibodies. By way of illustration, increased affinity can be selected for by contacting a large number of phage antibodies with a low amount of biotinylated antigen so that the antibodies compete for binding. In this case, the number of antigen molecules should exceed the number of phage antibodies, but the concentration of antigen should be somewhat below the dissociation constant. Thus, predominantly mutated phage antibodies with increased affinity bind to the biotinylated antigen, while the larger part of the weaker affinity phage antibodies remains unbound. Streptavidin can then assist in the enrichment of the higher affinity, mutated phage antibodies from the mixture (Schier, et al. (1996) J. Mol. Biol. 255:28-43). Exemplary affinity-maturated light chain CDR3 amino acid sequences are disclosed herein (see Tables 6 and 7), with particular embodiments embracing a light chain CDR3 amino acid sequence of Xaa₁-Gln-Xaa₂-Thr-Arg-Val-Pro-Leu-Thr (SEQ ID NO:2), wherein Xaa₁ is Phe or Leu, and Xaa₂ is Ala or Thr. Affinity-maturated heavy chain CDR3 amino acid sequences are also provided herein. An exemplary heavy chain CDR3 amino acid sequence is set forth herein as Arg-Gln-Leu-Gly-Thr-Arg-Gly-Thr-Asp-Ala-Met-Asp-Tyr (SEQ ID NO:3). The present invention also embraces derivatives of this CDR3, e.g., Arg-Ala-Leu-Ser-Pro-Arg-Ser-Ile-Asp-Ala-Met-Asp-Tyr (SEQ ID NO:4), Arg-Gln-Leu-Gly-Ala-Arg-Lys-Thr-Asp-Ala-Met-Asp-Tyr (SEQ ID NO:5), Arg-Gln-Leu-Gly-Pro-Arg-Lys-Arg-Asp-Ala-Met-Asp-Tyr (SEQ ID NO:6), Arg-Gln-Leu-Gly-Lys-Leu-Lys-Thr-Asp-Ala-Met-Asp-Tyr (SEQ ID NO:7), or Arg-Gln-Leu-Gly-Arg-Arg-Ser-Val-Asp-Ala-Met-Asp-Tyr (SEQ ID NO:8), wherein differences with the Hu20C2A3 heavy chain CDR3 are underlined. In this regard, the present invention specifically embraces an anti-ADDL antibody having a CDR3 amino acid sequence of Arg-Xaa₁-Leu-Xaa₂-Xaa₃-Xaa₄-Xaa₅-Xaa₆-Asp-Ala-Met-Asp-Tyr (SEQ ID NO:9), wherein Xaa₁ is Gln or Ala; Xaa₂ is Ser or Gly; Xaa₃ is Pro, Ala, Lys, Arg, or Thr; Xaa₄ is Lys or Arg; Xaa₅ is Gly, Ser, or Lys; Xaa₆ is Val, Thr, Ile or Arg

Other antibody derivatives encompassed within the scope of the present invention include any humanized antibody identical to Hu20C2A3's variable regions except with a one amino acid residue difference in the frame region of the light chain (e.g., Leu-Pro-Val-Thr-Pro-Gly-Glu-Pro-Ala-Ser, SEQ ID NO:10).

For some therapeutic applications it may be desirable to reduce the dissociation of the antibody from the antigen. To achieve this, phage antibodies are bound to biotinylated antigen and an excess of unbiotinylated antigen is added. After a period of time, predominantly the phage antibodies with the lower dissociation constant can be harvested with streptavidin (Hawkins, et al. (1992) J. Mol. Biol. 226:889-96).

Various immunoassays including those disclosed herein can be used for screening to identify antibodies, or fragments thereof, having the desired specificity for multi-dimensional conformations of ADDLs. Numerous protocols for competitive binding (e.g, ELISA), latex agglutination assays, immunoradiometric assays, kinetics *(e.g.,* BIACORE™ analysis) using either polyclonal or monoclonal antibodies, or fragments thereof, are well-know in the art. Such immunoassays typically involve the measurement of complex formation between a specific antibody and its cognate antigen. A two-site, monoclonal-based immunoassay utilizing monoclonal antibodies reactive to two non-interfering epitopes is suitable, but a competitive binding assay can also be employed. Such assays can also be used in the detection of multi-dimensional conformations of ADDLs in a sample.

An antibody or antibody fragment can also be subjected to other biological activity assays, e.g., displacement of ADDL binding to neurons or cultured hippocampal cells or blockade of ADDL assembly, in order to evaluate neutralizing or pharmacological activity and potential efficacy as a prophylactic or therapeutic agent. Such assays are described herein and are well-known in the art.

Antibodies and fragments of antibodies can be produced and maintained as hydridomas or alternatively recombinantly produced in any well-established expression system including, but not limited to, E. *coli,* yeast (e.g., *Saccharomyces* spp. and Pichia spp.), baculovirus, mammalian cells (e.g., myeloma, CHO, COS), plants, or transgenic animals (Breitling and Dübel (1999) In: Recombinant Antibodies, John Wiley & Sons, Inc., NY, pp. 119-132). Antibodies and fragments of antibodies can be isolated using any appropriate methods including, but not limited to, affinity chromatography, immunoglobulins-binding molecules (e.g., proteins A, L, G or H), tags operatively linked to the antibody or antibody fragment (e.g., His-tag, FLAG^{®}-tag, Strep tag, c-myc tag) and the like. See, Breitling and Dübel (1999) *supra.*

Antibodies and antibody fragments of the instant invention have a variety of uses including, diagnosis of diseases associated with accumulation of ADDLs, blocking or inhibiting binding of ADDLs to neuronal cells, blocking ADDL assembly, prophylactically or therapeutically treating a disease associated with ADDLs, identifying therapeutic agents that prevent binding of ADDLs to neurons, and preventing the phosphorylation of tau protein at Ser202/Thr205.

Antibody and antibody fragments of the instant invention are also useful in a method for blocking or inhibiting binding of ADDLs to neuronal cells. This method of the invention is carried out by contacting a neuron, in vitro or *in vivo,* with an antibody or antibody fragment of the present invention so that binding of ADDLs to the neuron is blocked. In particular embodiments, an antibody or antibody fragment of the instant invention achieves at least a 15%, 20%, 30%, 40%, 50%, 60%, 70%, 80%, 90%, 95%, or 97% decrease in the binding of ADDLs as compared to binding of ADDLs in the absence of the antibody or antibody fragment. The degree to which an antibody can block the binding of ADDLs to a neuron can be determined in accordance with the methods disclosed herein, i.e., immunocytochemistry or cell-based alkaline phosphatase assay or any other suitable assay. Antibodies particularly useful for decreasing binding of ADDLs to neuronal cells include anti-ADDL antibodies having a CDR3 amino acid sequence set forth in SEQ ID NO:9, as well as derivatives and fragments thereof.

Antibody and antibody fragments of the instant invention are further useful in a method for blocking or inhibiting assembly of ADDLs. This method involves contacting a sample containing amyloid β 1-42 peptides with an antibody or antibody fragment of the instant invention so that ADDL assembly is inhibited. The degree to which an antibody can block the assembly of ADDLs can be determined in accordance with the methods disclosed herein, i.e., FRET or fluorescence polarization or any other suitable assay. Antibodies particularly useful for blocking the assembly of ADDLs include anti-ADDL antibodies having a CDR3 amino acid sequence set forth in SEQ ID NO:9, as well as derivatives and fragments thereof.

Antibodies disclosed herein are also useful in methods for preventing the phosphorylation of tau protein at Ser202/Thr205. This method involves contacting a sample containing tau protein with an antibody or antibody fragment of the instant invention so that binding of ADDLs to neurons is blocked thereby preventing phosphorylation of tau protein. The degree to which an antibody can prevent the phosphorylation of tau protein at Ser202/Thr205 can be determined in accordance with the methods disclosed herein or any other suitable assay.

Blocking or decreasing binding of ADDLs to neurons, inhibiting assembly of ADDLs, and preventing the phosphorylation of tau protein at Ser202/Thr205 all find application in methods of prophylactically or therapeutically treating a disease associated with the accumulation of ADDLs. Accordingly, the present invention also embraces the use of an antibody or antibody fragment of the instant invention to prevent or treat a disease associated with the accumulation of ADDLs (e.g. Alzheimer's or similar memory-related disorders). Evidence in the art indicates that elevated levels of Aβ, but not necessarily aggregated plaque, are causative for Alzheimer's Disease-associated dementia and subsequent tau abnormalities. Aβ-derived diffusible ligands are directly implicated in neurotoxicity associated with Alzheimer's Disease. The art indicates that ADDLs are elevated in transgenic mice and Alzheimer's Disease patients and modulate functional activity associated with mnemonic processes in animal models. Thus, removing this form of Aβ could provide relief from the neurotoxicity associated with Alzheimer's Disease. As such, treatment with the instant antibody, which reduces central nervous system ADDL load, could prove efficacious for the treatment of Alzheimer's Disease. Patients amenable to treatment include individuals at risk of disease but not exhibiting symptoms, as well as patients presently exhibiting symptoms. In the case of Alzheimer's Disease, virtually anyone is at risk of suffering from Alzheimer's Disease if he or she lives long enough. Therefore, the antibody or antibody fragments of the present invention can be administered prophylactically to the general population without the need for any assessment of the risk of the subject patient. The present methods are especially useful for individuals who have a known genetic risk of Alzheimer's Disease. Such individuals include those having relatives who have been diagnosed with the disease, and those whose risk is determined by analysis of genetic or biochemical markers. Genetic markers of risk for Alzheimer's Disease include mutations in the APP gene, particularly mutations at position 717 and positions 670 and 671 referred to as the Hardy and Swedish mutations respectively. Other markers of risk are mutations in the presenilin genes, PS1 and PS2, and ApoE4, family history of Alzheimer's Disease, hypercholesterolemia or atherosclerosis. Individuals presently suffering from Alzheimer's Disease can be recognized from characteristic dementia, as well as the presence of risk factors described above. In addition, a number of diagnostic tests are available for identifying individuals who have Alzheimer's Disease. These include measurement of CSF tau and Aβ1-42 levels. Individuals suffering from Alzheimer's Disease can also be diagnosed by ADRDA criteria or the method disclosed herein.

In asymptomatic patients, treatment can begin at any age (e.g., 10, 20, 30 years of age). Usually, however, it is not necessary to begin treatment- until a patient reaches 40, 50, 60 or 70 years of age. Treatment typically entails multiple dosages over a period of time. Treatment can be monitored by assaying for the presence of ADDLs over time.

In therapeutic applications, a pharmaceutical composition or medicament containing an antibody or antibody fragment of the invention is administered to a patient suspected of, or already suffering from such a disease associated with the accumulation of ADDLs in an amount sufficient to cure, or at least partially arrest, the symptoms of the disease (biochemical, histologic and/or behavioral), including its complications and intermediate pathological phenotypes in development of the disease. In prophylactic applications, a pharmaceutical composition or medicament containing an antibody or antibody fragment of the invention is administered to a patient susceptible to, or otherwise at risk of, a disease associated with the accumulation of ADDLs in an amount sufficient to achieve passive immunity in the patient thereby eliminating or reducing the risk, lessening the severity, or delaying the outset of the disease, including biochemical, histologic and/or behavioral symptoms of the disease, its complications and intermediate pathological phenotypes presenting during development of the disease. In some methods, administration of agent reduces or eliminates myocognitive impairment in patients that have not yet developed characteristic Alzheimer's pathology. In particular embodiments, an effective amount of an antibody or antibody fragment of the invention is an amount which achieves at least a 15%, 20%, 30%, 40%, 50%, 60%, 70%, 80%, 90%, 95%, or 97% decrease in the binding of ADDLs to neurons in the patient as compared to binding of ADDLs in the absence of treatment. As such, impairment of long-term potentiation/memory formation is decreased.

Effective doses of the compositions of the present invention, for the treatment of the above described conditions vary depending upon many different factors, including means of administration, physiological state of the patient, whether the patient is human or an animal, other medications administered, and whether treatment is prophylactic or therapeutic. Usually, the patient is a human but nonhuman mammals such as dogs or transgenic mammals can also be treated.

Treatment dosages are generally titrated to optimize safety and efficacy. For passive immunization with an antibody or antibody fragment, dosage ranges from about 0.0001 to 100 mg/kg, and more usually 0.01 to 5 mg/kg, of the host body weight are suitable. For example, dosages can be 1 mg/kg body weight or 10 mg/kg body weight or within the range of 1-10 mg/kg. In some methods, two or more antibodies of the invention with different binding specificities are administered simultaneously, in which case the dosage of each antibody administered falls within the ranges indicated. Antibodies are usually administered on multiple occasions, wherein intervals between single dosages can be weekly, monthly or yearly. An exemplary treatment regime entails subcutaneous dosing, once biweekly or monthly. Advantageously, subcutaneous administration has been found to reduce the flu-like symptoms associated with intravenous infusions (Lundin, et al. (2002) Blood 100:768-773). Intervals can also be irregular as indicated by measuring blood levels of antibody to ADDLs in the patient. In some methods, dosage is adjusted to achieve a plasma antibody concentration of 1-1000 *µ*g/mL and in some methods 25-300 *µ*g/mL. Alternatively, the antibody or antibody fragment can be administered as a sustained-release formulation, in which case less frequent administration is required. Dosage and frequency vary depending on the half-life of the antibody in the patient. In general, human and humanized antibodies have longer half-lives than chimeric antibodies and nonhuman antibodies. As indicated above, dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Antibody and antibody fragments of the instant invention can be administered as a component of a pharmaceutical composition or medicament. Pharmaceutical compositions or medicaments generally contain the active therapeutic agent and a variety of other pharmaceutically acceptable components. See Remington: The Science and Practice of Pharmacy, Alfonso R. Gennaro, editor, 20th ed. Lippincott Williams & Wilkins: Philadelphia, PA, 2000. The preferred form depends on the intended mode of administration and therapeutic application. Pharmaceutical compositions can contain, depending on the formulation desired, pharmaceutically-acceptable, non-toxic carriers or diluents, which are defined as vehicles commonly used to formulate pharmaceutical compositions for animal or human administration. Diluents are selected so as not to affect the biological activity of the combination. Examples of such diluents are distilled water, physiological phosphate buffered saline, Ringer's solutions, dextrose solution, and Hank's solution.

Pharmaceutical compositions can also contain large, slowly metabolized macromolecules such as proteins, polysaccharides such as chitosan, polylactic acids, polyglycolic acids and copolymers (such as latex-functionalized SEPHAROSE™, agarose, cellulose, and the like), polymeric amino acids, amino acid copolymers, and lipid aggregates (such as oil droplets or liposomes).

Administration of a pharmaceutical composition or medicament of the invention can be carried out via a variety of routes including, but not limited to, oral, topical, pulmonary, rectal, subcutaneous, intradermal, intranasal, intracranial, intramuscular, intraocular, or intra-articular injection, and the like. The most typical route of administration is intravenous followed by subcutaneous, although other routes can be equally effective. Intramuscular injection can also be performed in the arm or leg muscles. In some methods, agents are injected directly into a particular tissue where deposits have accumulated, for example, intracranial injection. In some embodiments, an antibody or antibody fragment is injected directly into the cranium. In other embodiments, antibody or antibody fragment is administered as a sustained-release composition or device, such as a MEDIPAD™ device.

For parenteral administration, antibody or antibody fragments of the invention can be administered as injectable dosages of a solution or suspension of the substance in a physiologically acceptable diluent with a pharmaceutical carrier that can be a sterile liquid such as water, oils, saline, glycerol, or ethanol. Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are suitable liquid carriers, particularly for injectable solutions. Antibodies can be administered in the form of a depot injection or implant preparation which can be formulated in such a manner as to permit a sustained-release of the active ingredient.

An exemplary composition contains the instant antibody or antibody fragment formulated as a sterile, clear liquid at a concentration of at least 10 mg/ml in isotonic buffered saline (10 mM histidine, 150 mM sodium chloride, 0.01% (w/v) POLYSORBATE 80, pH 6.0). An exemplary antibody formulation is filled as a single dose, 0.6 ml glass vials filled with 3.3 ml of solution per vial. Each vial is stopped with a TEFLON-coated stopper and sealed with an aluminum cap.

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced delivery.

For suppositories, binders and carriers include, for example, polyalkylene glycols or triglycerides; such suppositories can be formed from mixtures containing the active ingredient in the range of 0.5% to 10%, or more desirably 1%-2%.

Oral formulations include excipients, such as pharmaceutical grades of mannitol, lactose, starch, magnesium stearate, sodium saccharine, cellulose, and magnesium carbonate. These compositions take the form of solutions, suspensions, tablets, pills, capsules, sustained-release formulations or powders and contain 10%-95% of active ingredient, or more suitably 25%-70%.

Topical application can result in transdermal or intradermal delivery. Topical administration can be facilitated by co-administration of the agent with cholera toxin or detoxified derivatives or subunits thereof or other similar bacterial toxins (see Glenn, et al. (1998) Nature 391:851). Co-administration can be achieved by using the components as a mixture or as linked molecules obtained by chemical crosslinking or expression as a fusion protein.

Alternatively, transdermal delivery can be achieved using a skin path or using transferosomes (Paul, et al. (1995) Eur. J. Immunol. 25:3521-24; Cevc, et al. (1998) Biochem. Biophys. Acta 1368:201-15).

An antibody or antibody fragment of the invention can optionally be administered in combination with other agents that are at least partly effective in treatment of amyloidogenic disease. For example, the instant antibody can be administered with existing palliative treatments for Alzheimer's Disease, such as acetylcholinesterase inhibitors such as ARICEPT™, EXELON™, and REMINYL™ and, the NMDA antagonist, NAMENDA™. In addition to these approved treatments, the instant antibody can be used to provide synergistic/additive benefit for any of several approaches currently in development for the treatment of Alzheimer's Disease, which include without limitation, inhibitors of Aβ production and aggregation.

Antibody and antibody fragments of the instant invention also find application in the identification of therapeutic agents that prevent the binding of ADDLs to neurons (e.g., a hippocampal cell) thereby preventing downstream events attributed to ADDLs. Such an assay is carried out by contacting a neuron with ADDLs in the presence of an agent and using an antibody of antibody fragment of the invention to determine binding of the ADDLs to the neuron in the presence of the agent. As will be appreciated by the skilled artisan, an agent that blocks binding of ADDLs to a neuron will decrease the amount of ADDLs bound to the neuron as compared to a neuron which has not been contacted with the agent; an amount which is detectable in an immunoassay employing an antibody or antibody fragment of the instant invention. Suitable immunoassays for detecting neuronal-bound ADDLs are disclosed herein.

Agents which can be screened using the method provided herein encompass numerous chemical classes, although typically they are organic molecules, preferably small organic compounds having a molecular weight of more than 100 and less than about 2,500 daltons. Agents encompass functional groups necessary for structural interaction with proteins, particularly hydrogen bonding, and typically include at least an amine, carbonyl, hydroxyl or carboxyl group, preferably at least two of the functional chemical groups. The agents often contain cyclical carbon or heterocyclic structures and/or aromatic or polyaromatic structures substituted with one or more of the above functional groups. Agents can also be found among biomolecules including peptides, antibodies, saccharides, fatty acids, steroids, purines, pyrimidines, derivatives, structural analogs or combinations thereof. Agents are obtained from a wide variety of sources including libraries of natural or synthetic compounds.

A variety of other reagents such as salts and neutral proteins can be included in the screening assays. Also, reagents that otherwise improve the efficiency of the assay, such as protease inhibitors, nuclease inhibitors, anti-microbial agents, and the like can be used. The mixture of components can be added in any order that provides for the requisite binding.

Agents identified by the screening assay of the present invention will be beneficial for the treatment of amyloidogenic diseases and/or tauopathies. In addition, it is contemplated that the experimental systems used to exemplify these concepts represent research tools for the evaluation, identification and screening of novel drug targets associated with amyloid beta induction of tau phosphorylation.

The present invention also provides methods for detecting ADDLs and diagnosing a disease associated with accumulation of ADDLs using an antibody or antibody fragment of the instant invention. A disease associated with accumulation of ADDLs is intended to include any disease wherein the accumulation of ADDLs results in physiological impairment of long-term potentiation/memory formation. Diseases of this type include, but are not limited to, Alzheimer's Disease and similar memory-related disorders.

In accordance with these methods, a sample from a patient is contacted with an antibody or antibody fragment of the invention and binding of the antibody or antibody fragment to the sample is indicative of the presence of ADDLs in the sample. As used in the context of the present invention, a sample is intended to mean any bodily fluid or tissue which is amenable to analysis using immunoassays. Suitable samples which can be analyzed in accordance with the methods of the invention include, but are not limited to, biopsy samples and fluid samples of the brain from a patient (e.g., a mammal such as a human). For in vitro purposes (e.g., in assays monitoring oligomer formation), a sample can be a neuronal cell line or tissue sample. For diagnostic purposes, it is contemplated that the sample can be from an individual suspected of having a disease associated with accumulation of ADDLs or from an individual at risk of having a disease associated with accumulation of ADDLs, e.g., an individual with a family history which predisposes the individual to a disease associated with accumulation of ADDLs.

Detection of binding of the antibody or antibody fragment to ADDLs in the sample can be carried out using any standard immunoassay (e.g., as disclosed herein), or alternatively when the antibody fragment is, e.g., a peptide aptamer, binding can be directly detected by, for example, a detectable marker protein (e.g., β-galactosidase, GFP or luciferase) fused to the aptamer. Subsequently, the presence or absence of the ADDL-antibody complex is correlated with the presence or absence, respectively, of ADDLs in the sample and therefore the presence or absence, respectively, of a disease associated with accumulation of ADDLs. It is contemplated that one or more antibodies or antibody fragments of the present invention can be used in conjunction with current non-invasive immuno-based imaging techniques to greatly enhance detection and early diagnosis of a disease associated with accumulation of ADDLs.

To facilitate diagnosis the present invention also pertains to a kit for containing an antibody or antibody fragment of the instant invention. The kit includes a container holding one or more antibody or antibody fragments which recognizes multi-dimensional conformation of ADDLs and instructions for using the antibody for the purpose of binding to ADDLs to form an antibody-antigen complex and detecting the formation of the antibody-antigen complex such that the presence or absence of the antibody-antigen complex correlates with presence or absence of ADDLs in the sample. Examples of containers include multiwell plates which allow simultaneous detection of ADDLs in multiple samples.

The invention is described in greater detail by the following non-limiting examples.

### Example 1: General Materials and Methods

*ADDL Preparation.* ADDLs in F12 medium (Biosource, Camarillo, CA) were prepared from Aβ1-42 in accordance with established methods (Lambert, et al. (2001) *supra).* Briefly, Aβ1-42 peptide (American Peptide Co., Sunnyvale, CA or California Peptide Research, Inc., Napa, CA) was weighed and placed in a glass vial capable of holding a sufficient quantity of HFIP (1,1,1,3,3,3-hexafluoro-2-propanol) to achieve a peptide concentration of 10 mg/mL. HFIP was added to the dry peptide, the vial was capped and gently swirl to mix, and the peptide/HFIP solution was stored at room temperature for at least one hour. Aliquots (50 or 100 *µ*L, 0.5 or 1.0 mg, respectively) of peptide solution was dispensed into a series of 1.5 mL conical centrifuge tubes. The tubes were placed in a SPEEDVAC^{®} overnight to remove the HFIP. Tubes containing the dried peptide film were capped and stored at -70°C in a sealed container with dessicant.

Prior to use, the Aβ1-42 peptide film was removed from -70°C storage and allowed to warm to room temperature. Fresh DMSO (44 µL/mg of peptide film; 5 mM) was added and the peptide/DMSO mixture was incubated on a vortex mixer at the lowest possible speed for ten minutes. F12 media (2 mL/mg peptide) was dispensed into each tube of DMSO/peptide and the tube was capped and mixed by inversion. The 100 *µ*M preparation was stored at 2-8°C for eighteen to twenty four hours. The samples were centrifuged at 14,000 x g for ten minutes at 2-8°C. The supernatant was transferred to a fresh tube and stored at 2-8°C until used.

Biotinylated ADDL preparations (bADDLs) were prepared in the same manner as described above for ADDL preparations using 100% N-terminal biotinylated amyloid beta peptide (American Peptide Company, Sunnyvale, CA).

*Monomer Preparation.* HFIP dry down preparations of amyloid beta (1-40) peptide (Aβ1-40) were prepared as outlined for Aβ(1-42) peptide. The peptide film was dissolved in 2 mL of 25 mM borate buffer (pH 8.5) per mg of peptide, divided into aliquots, and frozen at -70°C until used.

*Primary Neurons.* Primary hippocampal cultures were prepared from frozen, dissociated neonatal rat hippocampal cells (Cambrex, Corp., East Rutherford, NJ) that were thawed and plated in 96-well COSTAR^{®} plates at a concentration of 20,000 cells per well. The cells were maintained in NEUROBASAL™ media without L-glutamine (GIBCO-BRL™, Gaithersburg, MD) and supplemented with B27 (GIBCO-BRL™, Gaithersburg, MD) for a period of two weeks and then used for binding studies.

*Immunocytochemistry.* Immunocytochemistry was performed according to established methods (Lambert, et al. (2001) *supra*), except the secondary antibodies were conjugated to ALEXAFLUOR^{®} 588 (Molecular Probes, Eugene, OR). Antibodies and ADDLs were preincubated for 1 hour at room temperature, at a molar ratio of 1:4 antibody:ADDL before application to the 21-day hippocampal cell culture. For endogenous ADDLs, human brain protein (prepared as in Lambert, et al. (2001) supra) was incubated with cells for 1 hour before the cells were washed, fixed, and visualized as above.

Lightly fixed frozen sections (4% paraformaldehyde at 4°C for 30 hours and cryoprotected in 40 µm sucrose) from Alzheimer's Disease and control hippocampus were incubated with antibody (1:1000 in phosphate-buffered saline (PBS)) overnight at 4°C. After removal of antibody, sections were washed 3 times with PBS and incubated with secondary antibody at room temperature. Binding was then visualized with DAB (SIGMA™, St. Louis, MO). Sections were then counterstained with hematoxylin, mounted, and imaged on a NIKON^{®} ECLIPSE^{®} E600 light microscope with a SPOT™ INSIGHT™ digital video camera (v. 3.2).

*ELISA*. Polyclonal anti-ADDLs IgG (M90/1; Bethyl Laboratories, Inc., Montgomery, TX) was plated at 0.25 mg/well on IMMULON™ 3 REMOVAWELL™ strips (Dynatech Labs, Chantilly, VA) for 2 hours at room temperature and the wells blocked with 2% BSA in TBS. Samples diluted with 1% BSA in F12 were added to the wells, allowed to bind for 2 hours at 4°C, and washed 3X with BSA/TBS at room temperature. Monoclonal antibodies diluted in BSA/TBS were incubated for 90 minutes at room temperature and detected with a VECTASTAIN^{®} ABC kit to mouse IgG. The HRP label was visualized with BIO-RAD^{®} peroxidase substrate and read at 405 nm on a Dynex MRX-TC microplate reader.

### Example 2: Isolation of Mouse Antibody Variable Region Sequences

The cDNAs coding for the variable domains of the 20C2 mouse antibody were cloned and sequenced following a polymerase chain reaction (PCR) using specially designed primers that hybridize to the 5'-ends of the mouse constant regions and to the murine leader sequences upstream of the V regions. This ensured that the mouse variable region sequences obtained were complete and accurate. In short, mRNA was extracted from mouse hybridoma cell lines using the QIAGEN^{®} OLIGOTEX^{®} Direct mRNA Mini Kit and subsequently converted to cDNA using a first-strand cDNA synthesis kit. The cDNA was then used as template in PCR reactions to obtain the antibody variable region sequences.

To obtain the light chain variable region sequence, eleven independent PCR reactions were set up using each of the eleven light chain 5' PCR primers (MKV-1 to MKV-11) and the 3' PCR primer MKC-1 (Table 1).

**TABLE 1**

| 5' Primer | Sequence | SEQ ID NO: |
|---|---|---|
| MKV-1 | GAT CTC TAG **ATG AAG ATT GCC TGT TAG GCT GTT GGT GCT G** | 11 |
| MKV-2 | GAT CTC TAG **ATG GAG WCA GAC ACA CTC CTG YTA TGG GTG** | 12 |
| MKV-3 | GAT CTC TAG **ATG AGT GTG CTC ACT CAG GTC CTG GSG TTG** | 13 |
| MKV-4 | GAT CTC TAG **ATG AGG RCC CCT GCT CAG WTT YTT GGM WTC TTG** | 14 |
| MKV-5 | GAT CTC TAG **ATG GAT TTW CAG GTG CAG ATT WTC AGC TTC** | 15 |
| MKV-6 | GAT CTC TAG **ATG AGG TKC YYT GYT SAY CTY CTC TGR GG** | 16 |
| MKV-7 | GAT CTC TAG **ATG GGC WTC AAA GAT GGA GTC ACA KWY YCW GG** | 17 |
| MKV-8 | GAT CTC TAG **ATG TGG GGA YCT KTT TYC MMT TTT TCA ATG** | 18 |
| MKV-9 | GAT CTC TAG **ATG GTR TCC WCA SCT CAG TTC CTT G** | 19 |
| MKV-10 | GAT CTC TAG **ATG TAT ATA TGT TTG TTG TCT ATT TCT** | 20 |
| MKV-11 | GAT CTC TAG **ATG GAA GCC CCA GCT CAG CTT CTC TTC C** | 21 |

| 3' Primer | Sequence | SEQ ID NO: |
|---|---|---|
| MKC-1 | GAT CGA GCT **CAC TGG ATG GTG GGA AGA TGG** | 22 |

| | | |
|---|---|---|
| Underlined and italic sequences denote *Xba*I and *Sac*I restriction sites, respectively. W = A or T, M = A or C, K = G or T, Y = C or T, and R = A or G. | | |

To obtain the heavy chain variable region sequences twelve independent PCR reactions were set up using each of the twelve heavy chain 5' PCR primers (MHV-1 to MHV-12) and the appropriate isotype specific 3' primer (MHCG-1, MHCG-2A, MHCG-2B, MHCG-3) (Table 2).

**TABLE 2**

| 5' Primer | Sequence | SEQ ID NO: |
|---|---|---|
| MHV-1 | GAT CTC TAG **ATG AAA TGC AGC TGG GGC ATS TTC TTC** | 23 |
| MHV-2 | GAT CTC TAG **ATG GGA TGG AGC TRT ATC ATS YTC TT** | 24 |
| MHV-3 | GAT CTC TAG **ATG AAG WTG TGG TTA AAC TGG GTT TTT** | 25 |
| MHV-4 | GAT CTC TAG **ATG RAC TTT GGG YTC AGC TTG RTT T** | 26 |
| MHV-5 | GAT CTC TAG **ATG GGA CTC CAG GCT TCA ATT TAG TTT TCC TT** | 27 |
| MHV-6 | GAT CTC TAG **ATG GCT TGT CYT TRG SGC TRC TCT TCT GC** | 28 |
| MHV-7 | GAT CTC TAG **ATG GRA TGG AGC KGG RGT CTT TMT CTT** | 29 |
| MHV-8 | GAT CTC TAG **ATG AGA GTG CTG ATT CTT TTG TG** | 30 |
| MHV-9 | GAT CTC TAG **ATG GMT TGG GTG TGG AMC TTG CTT ATT CCT G** | 31 |
| MHV-10 | GAT CTC TAG **ATG GGC AGA CTT ACC ATT CTC ATT CCT G** | 32 |
| MHV-11 | GAT CTC TAG **ATG GAT TTT GGG CTG ATT TTT TTT ATT G** | 33 |
| MHV-12 | GAT CTC TAG **ATG ATG GTG TTA AGT CTT CTG TAC CTG** | 34 |

| 3' Primer | Sequence | SEQ ID NO: |
|---|---|---|
| MHCG - 1 | GCATC *GAG CTC* **CAG TGG ATA GAC AGA TGG GGG** | 35 |
| MHCG-2A | GCATC *GAG CTC* **CAG TGG ATA GAC CGA TGG GGG** | 36 |
| MHCG-2B | GCATC GAG CTC **CAG TGG ATG AGC TGA TGG GGG** | 37 |
| MHCG-3 | GCATC *GAG CTC* **CAA GGG ATA GAC AGA TGG GGC** | 38 |

| | | |
|---|---|---|
| Underlined and italic sequences denote *Xba*I and *Sac*I restriction sites, respectively. W = A or T, M = A or C, K = G or T, Y = C or T, and R = A or G. | | |

Each of the light chain PCR reactions contained 46 µL INVITROGEN™ PLATINUM^{®} PCR Super Mix, 1.0 µL of one of the 100 µM 5' primers (MKV-1 to MKV-11), 1.0 µL of the 100 µM 3' primer (MKC-1), and 2.0 µL of hybridoma cDNA. Similar PCR reactions were employed to clone the mouse heavy chain variable region sequences. Reactions were placed in a DNA thermal cycler and, after an initial denaturation step at 97°C for 2.0 minutes, subjected to 30 cycles of: 95°C for 30 seconds, 55°C for 45 seconds, and 72°C for 90 seconds. Following the last cycle, a final extension step at 72°C for 10 minutes was employed. To determine which PCR reactions yielded product, 5 µL aliquots from each reaction were separated on 1.5% (w/v) agarose/1X TAE buffer gels, containing 0.5 µg/mL ethidium bromide. PCR products from reactions that produced fragments of the expected size (420 to 500 bp) were then gel purified, digested with *Xba*I and *Sac*I and ligated into the *Xba*I and *Sac*I sites in the multicloning region of plasmid pNEB193 (New England Biolabs, Beverly, MA). Alternatively, PCR products were ligated directly into plasmid pCR^{®}2.1 using the INVITROGEN™ TA CLONING^{®} kit. Ligation products were then transformed into XL-1 cells and aliquots of the transformed *E*. *coli* were plated onto LB agar plates containing 50 *µ*g/mL ampicillin and overlaid with 40 *µ*L of X-Gal stock (50 mg/mL) and 40 *µ*L IPTG (100 mM) solution for blue/white selection. Plates were incubated overnight at 37°C and potential clones were identified as white colonies. DNA from at least 24 independent clones for each PCR product were sequenced on both strands using universal forward and reverse primers for pNEB193 and pCR^{®}2.1. The resulting sequences were then assembled into a contig to generate a consensus sequence for each antibody light and heavy chain variable region. Using this approach the sequences were determined for the light and heavy antibody variable regions of hybridoma 20C2 (Figures 1A-1B). The six complementarity-determining regions (CDRs), which form the structure complementary to the antigen, are underlined in Figures 1A-1B.

### Example 3: Humanization of Mouse Anti-ADDL Antibody Variable Region Sequences

Mouse antibody heavy and light variable domain nucleic acids obtained from mouse hybridoma cell line 20C2 were humanized using a CDR grafting approach. It will be appreciated by those skilled in the art that humanization of mouse antibody sequences can maximize the therapeutic potential of an antibody by improving its serum half-life and effector functions thereby reducing the anti-globulin response.

Humanization by CDR grafting was carried out by selecting the human light and heavy chain variable regions from the NCBI protein database with the highest homology to the mouse variable domains. The mouse variable region sequences were compared to all human variable region sequences in the database using the protein-protein Basic Local Alignment Search Tool (BLAST). Subsequently, mouse CDRs were joined to the human framework regions and the preliminary amino acid sequence was analyzed. All differences between the mouse and human sequences in the framework regions were evaluated particularly if they were part of the canonical sequences for loop structure or were residues located at the VL/VH interface (O'Brien and Jones (2001) In: Antibody Engineering, Kontermann and Dubel (Eds.), Springer Laboratory Manuals). Framework regions were also scanned for unusual or rare amino acids in comparison to the consensus sequences for the human subgroup and for potential glycosylation sites. Wherein amino acid sequence differences existed between the mouse and human framework region sequences that were not found to be involved in canonical sequences, or located at the VL/VH interface, the human residue was selected at that position. Wherein a difference in a key residue existed, two versions of the variable region sequence were generated for evaluation. The CDR grafting strategy made the minimum number of changes to the human framework region so that good antigen binding was achieved while maintaining human framework regions that closely matched the sequence from a natural human antibody. The heavy chain and light chain variable region amino acid sequences of the resulting humanized antibody generated by CDR grafting of murine 20C2 are shown in Figures 2A and 2B, respectively. This antibody is designated herein as Hu20C2.

Humanized sequences for 20C2 were also designed using a veneering strategy (See, e.g., U.S. Patent No. 6,797,492). Humanization was carried out by selecting the human light and heavy chain variable regions from the NCBI protein database with the highest homology to the mouse variable domains, as well as to the closest human antibody germline family or families (see, Kabat, eta 1. (1991) Sequences of proteins of immunological interest, 5^{th} ed., U.S. Dept. Health and Human Services, NIH, Washington DC). The mouse variable region sequences were compared to all human variable region sequences in the database using protein-protein BLAST. The murine variable sequences and their closest human homologues were modeled to the closest crystallized human antibody as determined by computer modeling as practiced in the art. From the model of the murine VH and VL sequences, a surface area map was constructed, which dictated the solvent accessibility of the amino acids in the mouse heavy and light variable regions. To confirm the modeling, these exposed residues were compared position-by-position with known surface accessible residues (see, e.g., Padlan (1994) Mol. Immunol. 31(3):169-217). A score was assigned for each residue in the sequence designating it as exposed, mostly exposed, partly buried, mostly buried and buried according to established methods (see, U.S. Patent No. 6,797,492, incorporated herein by reference in its entirety). Mouse framework residues that scored as exposed or mostly exposed and differed from the homologous human sequence were changed to the human residue at that position. The designed veneered sequences retained the mouse CDRs, residues neighboring the CDRs, residues known be involved in canonical sequences, residues locates at the VL/VH interface, and residues at the N-terminal sequences of the mouse heavy and light chain. The N-terminal sequences are known to be contiguous with the CDR surface and are potentially involved in ligand binding. Once the veneered sequences were finalized they were remodeled to look for are any potential obvious structural issues. A total of 12 and 9 amino acid residues were changed in the heavy chain and light chain frames, respectively. The heavy chain and light chain variable region amino acid sequences of the resulting humanized antibody generated by veneering of murine 20C2 are shown in Figures 2A and 2B, respectively. This antibody is designated herein as Hu20C2A3.

In comparison to 20C2, it is noted that the light chain substitutions resulting in Hu20C2A3, but not the heavy chain substitutions, are in common with Hu20C2 (Figure 2). In particular, heavy chain variable region CDR3 is unique to Hu20C2A3.

Once the humanized amino acid sequences were selected the sequences were reverse-translated to obtain the corresponding DNA sequence. The DNA sequences were codon-optimized using art-established methods (Lathe (1985) J. Mol. Biol. 183 (1) :1-12) and designed with flanking restriction enzyme sites for cloning into human antibody expression vectors. The nucleotide sequences encoding the light chain variable region and two versions of the heavy chain variable region for Hu20C2 are presented in Figures 3A-3C. The two heavy chain variable region versions differ by a single amino acid substitution at position 24; heavy chain variable region for version A of Hu20C2 is Phe at position 24 and heavy chain variable region of version B of Hu20C2 is Leu at position 24.

### Example 4: Affinity Maturation

Affinity maturation was carried out on the Hu20C2 antibody. Nucleic acid molecules encoding humanized Hu20C2 versions A and B variable heavy chain only, light chain only or heavy chain version A and light chain together were cloned in the Fab phage-display vector pFab4. Nucleic acid sequence analysis confirmed sequence and orientation in pFab4. The annotated Hu20C2 Fab sequences in pFab4 are presented in Figures 4A-4C and set forth herein as SEQ ID NO:116 for heavy chain version A, SEQ ID NO:117 for heavy chain version B, and SEQ ID NO:118 for the light chain. The nucleotide sequence for heavy chain version A and light chain together in the pFab4 vector is presented in Figures 4D-4E. These constructs were used in the Hu20C2 maturation program using art-established phage-displayed Fab library methods.

*Light Chain Maturation.* Two libraries were designed to mutate the nine wild-type amino acids of CDR3 of the light (kappa) chain of Hu20C2 (i.e., Phe-Gln-Gly-Ser-Leu-Val-Pro-Leu-Thr; SEQ ID NO:39). These libraries were designated LC3-1 and LC3-2 representing light chain CDR3 sequences of Xaa-Xaa-Xaa-Xaa-Xaa-Val-Pro-Leu-Thr (SEQ ID NO:40) and Phe-Gln-Gly-Ser-Xaa-Xaa-Xaa-Xaa-Xaa (SEQ ID NO:41), respectively. Biotinylated reverse primers, 20C2LC3-1 (SEQ ID NO:123) and 20C2LC3-2 (SEQ ID NO:126), were used in combination with forward primer 20C2LC3F (SEQ ID NO:120) to generate the LC3-1 and LC3-2 libraries (see Figure 5A). Primers were purified by polyacrylamide gel electrophoresis, whereas the vector DNA was purified by gel electrophoresis and electroelution. The two light chain libraries were designed to be randomly mutated. The final diversities of the three 10G5H6 LC₃ libraries were 4.76 x 10⁸ and 7.45 x 10⁸, respectively (Table 3). Sequence analysis of approximately 100 clones from the libraries showed 100% diversity of mutant clones at the designed amino acid positions.

**TABLE 3**

| Characteristic | Light Chain Library | |
|---|---|---|
| | LC3-1 | LC3-2 |
| Vector | pFab3d20C2HS | pFab3d20C2HS |
| Number of Transformants | 4.76 x 10⁸ | 7.45 x 10⁸ |
| Library Diversity | 4.76 x 10⁸ x 0.89 = 4.24 10⁸ | 7.45 x 10⁸ × 0.90 = 6.71 10⁸ |
| Primary Library Volume | 2 mL | 2 mL |
| Primary Library Titer | 2.13 x 10¹¹ | *9.3 x 10¹⁰ |

| | | |
|---|---|---|
| *Higher titers are achieved by concentration or phage rescue. | | |

Soluble panning of the two light chain libraries against high molecular weight bADDL was completed. Briefly, four rounds of panning were carried out using biotinylated high molecular weight ADDL (bADDL). The first three rounds were carried out using approximately 1.5 µM antigen concentration (input = 1 x 10¹⁰ to 1 x 10¹¹). Upon completion of the third round, the outputs of the two libraries were combined and divided into three groups for analysis with 10 nM, 100 nM and approximately 1.5 µM antigen to increase panning stringency. As such, a total of 58 output plates were tested in phage ELISA assays, i.e., two plates per library in the first round (a total of four plates), six plates per library in the second round (a total of 12 plates), eight plates for LC3-1 and 10 plates for LC3-2 libraries in the third round (a total of 18 plates) and eight plates for each antigen concentration in the fourth round (a total of 24 plates).

Panning resulted in 1000 hits, 436 of which were sequenced (Table 4).

**TABLE 4**

| Round | Antigen | Input | Output | % Recovery | ELISA Screen* | Sequenced |
|---|---|---|---|---|---|---|
| 1^{a} | 1.6 µM | 2.13x010¹⁰ | 7.3x10⁴ | 3.42x10⁻⁶ | 0% (0/176) | 0 |
| 2^{a} | 2.0 µM | 1.55x10¹¹ | 1.88x10⁵ | 1.21x10⁻⁶ | 1.5% (8/528) | 8 |
| 3^{a} | 1.1 µM | 1.80x10¹⁰ | 7.8x10⁴ | 4.3x10⁻⁶ | 5.8% (41/704) | 41 |
| 1^{b} | 1.6 µM | 9.30x10⁹ | 5.7x10⁴ | 6.13x10⁻⁶ | 2.3% (7/176) | 4 |
| 2^{b} | 2.0 µM | 1.23x10¹¹ | 1.07x10⁵ | 8.7x10⁻⁷ | 4.5% (24/528) | 24 |
| 3^{b} | 1.1 µM | 1.37x10¹⁰ | 3.32x10⁵ | 2.42x10⁻⁵ | 15% (134/880) | 134 |
| 4^{c} | 1.1 µM | 3.0x10¹¹ | 1.37x10⁵ | 4.6x10⁻⁷ | 39% (274/704) | - |
| 4^{C} | 100 nM | 3.0x10¹¹ | 3.88x10⁵ | 1.29x10⁻⁶ | 41% (290/704) | - |
| 4^{c} | 10 nM | 3.0x10¹¹ | 1.6x10⁵ | 5.3x10⁻⁷ | 32% (225/704) | 225 |
| | | | | Total | 1000/5104 | 436 |

| | | | | | | |
|---|---|---|---|---|---|---|
| ^{a}20C2 LC3-1 versus high molecular weight 10% bADDL. ^{b}20C2 LC3-2 versus high molecular weight 10% bADDL. ^{c}20C2 LC3-1 + 20C2 LC3-2 versus high molecular weight 10% bADDL. *Hits per total number of colonies. | | | | | | |

Sequence and frequency of highly enriched clones are presented in Table 5.

**TABLE 5**

| Clone Designation | LC CDR3 | (SEQ ID NO: | Round 2 | Round 3 | Round 4 | Total |
|---|---|---|---|---|---|---|
| Hu20C2LC | FQGSLVPLT | 39 | 6 | 15 | 14 | 35 |
| SJ-p1-31 | ADTTHVPLT | 42 | | 1 | 2 | 3 |
| SJ-p1-14 | AHSTFVPLT | 43 | 1 | 1 | 2 | 4 |
| 4P2-12-E3 | AQASFVPLT | 44 | | | 2 | 2 |
| SJ-p1-38 | AQATKVPLT | 45 | | 1 | 1 | 2 |
| 4P3-59 | AQSSKVPLT | 46 | | | 2 | 2 |
| SJ-p2-14 | AQSTLVPLT | 47 | | 1 | 2 | 3 |
| 4P3-11 | FAASSVPLT | 48 | | | 2 | 2 |
| 4P3-1 | FESTYVPLT | 49 | | | 2 | 2 |
| SJ-p2-10 | FESSRVPLT | 50 | | 1 | 1 | 2 |
| SJ-p2-11 | FNATWVPLT | 51 | | 2 | | 2 |
| SJ-p2-60 | FQASRVPLT | 52 | | 1 | 5 | 6 |
| SJ-p1-18 | FQATRVPLT | 53 | | 1 | 5 | 6 |
| SJ-p3-51 | FQGSFIGLS | 54 | 1 | | 1 | 2 |
| SJ-p3-16 | FQGSFIPGT | 55 | | 2 | 3 | 5 |
| SJ-p8-8F | FQGSFLPPS | 56 | | 1 | 1 | 2 |
| SJ-p3-26 | FQGSFLPQL | 57 | 1 | 2 | | 3 |
| SJ-p3-15 | FQGSLFPPV | 58 | 1 | 2 | | 3 |
| SJ-p2-70 | FQGSLFSPS | 59 | 1 | 5 | | 6 |
| SJ-p3-24 | FQGSRIPIS | 60 | | 1 | 1 | 2 |
| SJ-p3-33 | FQGSRLPVS | 61 | | 2 | 3 | 5 |
| SJ-p3-14 | FQGSRVPLV | 62 | | 2 | 1 | 3 |
| SJ-p2-1F | FQSSFVPLT | 63 | | 6 | 8 | 14 |
| 4P1-22 | FQSSRVPLT | 64 | | | 15 | 15 |
| SJ-p2-44 | GQTTLVPLT | 65 | | 1 | 3 | 4 |
| SJ-p1-56 | HESTLVPLT | 66 | | 2 | 1 | 3 |
| 4P1-40 | HQSSKVPLT | 67 | | | 4 | 4 |
| SJ-p2-20 | IQTSLVPLT | 68 | | 2 | | 2 |
| SJ-p1-41 | IQAALVPLT | 69 | | 1 | 1 | 2 |
| SJ-p2-13 | LQSSFVPLT | 70 | 1 | 4 | | 5 |
| 4P1-26 | LETSRVPLT | 71 | | | 3 | 3 |
| SJ-p1-33 | LASSHVPLT | 72 | | 2 | 1 | 3 |
| SJ-p2-27 | LNSTTVPLT | 73 | | 2 | 4 | 6 |
| SJ-p2-62 | LQSKSVPLT | 74 | | 2 | | 2 |
| 4P2-26-E5 | LQSVRVPLT | 75 | | | 3 | 3 |
| 4P1-32 | LQSSLVPLT | 76 | | | 5 | 5 |
| SJ-p2-37 | LQTTRVPLT | 77 | | 2 | 2 | 4 |
| SJ-p2-64 | LQTSFVPLT | 78 | | 3 | | 3 |
| 4P1-20 | LQTSNVPLT | 79 | | | 5 | 5 |
| SJ-p2-39 | LQTTRVPLT | 80 | | 2 | 6 | 8 |
| SJ-p2-52 | LSSTFVPLT | 81 | | 3 | 1 | 4 |
| SJ-p2-6L | LSSTHVPLT | 82 | | 2 | 1 | 3 |
| 4P1-77 | LTSSAVPLT | 83 | | | 2 | 2 |
| SJ-p1-59 | LVSSLVPLT | 84 | | 2 | | 2 |
| SJ-p2-23 | METANVPLT | 85 | | 2 | | 2 |
| SJ-p1-9M | MQSSFVPLT | 86 | | 1 | 3 | 4 |
| SJ-p2-28 | MQSSLVPLT | 87 | | 1 | 2 | 3 |
| SJ-p1-21 | MQTSKVPLT | 88 | | 1 | 1 | 2 |
| 4P1-17 | SQARMVPLT | 89 | | | 3 | 3 |
| SJ-p2-66 | SQASRVPLT | 90 | | 1 | 2 | 3 |
| SJ-p1-49 | TQSTQVPLT | 91 | | 2 | 1 | 3 |
| SJ-p2-24 | VCATFVPLT | 92 | | 1 | 1 | 2 |
| 4P1-41 | VQSSAVPLT | 93 | | | 2 | 2 |
| SJ-p2-51 | VQTSLVPLT | 94 | | 12 | 31 | 43 |
| 4P1-64 | VQTSVVPLT | 95 | | | 3 | 3 |
| SJ-p2-55 | VQTTAVPLT | 96 | | 2 | | 2 |
| SJ-p1-25 | LQTARVPLT | 97 | | 1 | 3 | 4 |

Fab fragments from the 10 top clones based on enrichment frequency were prepared and a total of 15 clones were converted into IgG1 humanized A version and two clones, 20C2-6 and 20C2-8, were converted to IgG1 humanized B version. K_{D} values for these clones were measured by BIACORE™ using biotin-Aβ1-20 (Table 6) and bADDL (Table 7) as antigens. Dramatic improvements in affinity were observed as compared to parental humanized 20C2A and 20C2B, as well as mouse 20C2 antibodies. In particular, low nanomolar to sub-picomolar K_{D}S were achieved with a light chain CDR3 of the sequence Xaa₁-Gln-Xaa₂-Thr-Arg-Val-Pro-Leu-Thr (SEQ ID NO:2), wherein Xaa₁ is Phe or Leu, and Xaa₁ is Ala or Thr. Moreover, a comparison between K_{D} values obtained with BIACORE^{™} using biotin-Aβ1-20 and bADDL further demonstrates that anti-ADDL antibodies such as Hu20C2 preferentially bind multi-dimensional conformations of ADDLs over monomeric Aβ peptides.

**TABLE 6**

| Name | Clone | LC-CDR3 | SEQ ID NO: | K_{D} (Biotin-Aβ1-20) | | |
|---|---|---|---|---|---|---|
| | | | | Fab | IgG1#1 | IgG1#2 |
| 20C2-1A | SJ-p2-60 | FQASRVPLT | 52 | 91 nM | 1.2 nM | -- |
| 20C2-2A | SJ-p1-18 | FQATRVPLT | 53 | 28 nM | 686 pM | 2 nM |
| 20C2-3A | SJ-p3-16 | FQGSFIPGT | 55 | -- | 1.7 nM | -- |
| 20C2-5A | SJ-p2-1F | FQSSFVPLT | 63 | 41 nM | 912 pM | 1.5 nM |
| 20C2-6A | 4P1-22 | FQSSRVPLT | 64 | 18 nM | 544 pM | 714 pM |
| 20C2-6B | 4P1-22 | FQSSRVPLT | 64 | -- | 53 pM | -- |
| 20C2-7A | SJ-p2-27 | LNSTTVPLT | 73 | 128 nM | -- | -- |
| 20C2-8A | SJ-p2-39 | LQTTRVPLT | 80 | 14 nM | 140 pM | 376 pM |
| 20C2-8B | SJ-p2-39 | LQTTRVPLT | 80 | -- | 46 pM | 64 pM |
| 20C2-9A | SJ-p2-51 | VQTSLVPLT | 94 | 36 nM | 241 pM | 420 pM |
| 20C2-10A | SJ-p3-33 | FQGSRLPVS | 61 | -- | 84 nM | -- |
| 20C2-11A | SJ-p3-6 | FQGSLLPLS | 98 | -- | -- | -- |
| 20C2-12A | 4P1-32 | LQSSLVPLT | 76 | 617 nM | 1.5 nM | -- |
| 20C2-13A | 4p1-20 | LQTSNVPLT | 79 | 94 nM | 3 nM | -- |
| 20C2-18A | SJ-p1-9M | MQSSFVPLT | 86 | 126 nM | 1.8 nM | -- |
| 20C2-20A | SJ-p3-15 | FQGSLFPPV | 58 | | 21 nM | |
| 20C2-22A | SJ-p2-66 | SQASRVPLT | 90 | | 2.3 nM | |
| 20C2-23A | 4P1-40 | HQSSKVPLT | 67 | | 649 pM | 1.5 nM |
| 20C2-24A | SJ-p2-44 | GQTTLVPLT | 65 | | 1.9 nM | |
| 20C2A | | FQGSLVPLT | 39 | | 27 nM | |
| 20C2B | | FQGSLVPLT | 39 | | 5.4 nM | |
| Mouse-20C2 | | FQGSLVPLT | 39 | 83 nM | 3.4 nM | |

**TABLE 7**

| Name | Clone | LC-CDR3 | SEQ ID NO : | K_{D} (bADDL) | | |
|---|---|---|---|---|---|---|
| | | | | Fab | IgG1#1 | IgG1#2 |
| 20C2-1A | SJ-p2-60 | FQASRVPLT | 52 | 85 nM | 75 pM | -- |
| 20C2-2A | SJ-p1-18 | FQATRVPLT | 53 | 28 nM | 15 pM | 0.3 pM |
| 20C2-3A | SJ-p3-16 | FQGSFIPGT | 55 | -- | 3.7 nM | -- |
| 20C2-5A | SJ-p2-1F | FQSSFVPLT | 63 | 41 nM | 317 pM | 68 pM |
| 20C2-6A | 4P1-22 | FQSSRVPLT | 64 | 42 nM | 4.3 pM | 24 pM |
| 20C2-6B | 4P1-22 | FQSSRVPLT | 64 | -- | 53 pM | -- |
| 20C2-7A | SJ-p2-27 | LNSTTVPLT | 73 | 435 nM | -- | -- |
| 20C2-8A | SJ-p2-39 | LQTTRVPLT | 80 | 13 nM | 3 pM | 0.7 pM |
| 20C2-8B | SJ-p2-39 | LQTTRVPLT | 80 | -- | 13 pM | 0.8 pM |
| 20C2-9A | SJ-p2-51 | VQTSLVPLT | 94 | 40 nM | -- | 2 pM |
| 20C2-10A | SJ-p3-33 | FQGSRLPVS | 61 | -- | 7.7 nM | |
| 20C2-11A | SJ-p3-6 | FQGSLLPLS | 98 | -- | -- | -- |
| 20C2-12A | 4P1-32 | LQSSLVPLT | 76 | 238 nM | 15 pM | -- |
| 20C2-13A | 4p1-20 | LQTSNVPLT | 79 | 567 nM | 764 pM | |
| 20C2-18A | SJ-p1-9M | MQSSFVPLT | 86 | 85 nM | 149 pM | |
| 20C2-20A | SJ-p3-15 | FQGSLFPPV | 58 | | 6.9 nM | |
| 20C2-22A | SJ-p2-66 | SQASRVPLT | 90 | | 198 pM | |
| 20C2-23A | 4P1-40 | HQSSKVPLT | 67 | | 85 pM | 66 pM |
| 20C2-24A | SJ-p2-44 | GQTTLVPLT | 65 | | 114 pM | |
| 20C2A | | FQGSLVPLT | 39 | | | |
| 20C2B | | FQGSLVPLT | 39 | | | |
| Mouse-20C2 | | FQGSLVPLT | 39 | 62 nM | 4.1 nM | |

*Heavy Chain Maturation*. The heavy chain of Hu20C2 was also subjected to optimization by generation of 3 libraries covering the heavy chain-CDR3 (RQLGLRSIDAMDY; SEQ ID N0:99). These libraries were designated 20C2B-39HC₃-1, 20C2B-39HC₃-2, and 20C2B-39HC₃-3 representing heavy chain CDR3 sequences of XXXXXRSIDAMDY (SEQ ID NO:100) and RQLGLRSIXXXXX (SEQ ID NO:101) and RQLGXXXXXAMDY (SEQ ID NO:102), respectively. Biotinylated reverse primers, 20C2HC3-1 (SEQ ID NO:130), 20C2HC3-2 (SEQ ID NO:133), and 20C2HC3-3 (SEQ ID NO:136) were used in combination with forward primer 20C2HC3F (SEQ ID NO:127) to generate the 3 libraries (see Figure 5B). The libraries contained >10⁸ functional diversity and covered all combinations of amino acids at every position randomized in each set (see Table 8).

**TABLE 8**

| Monoclonal Antibody | Diversity | Sequence | SEQ ID NO: |
|---|---|---|---|
| Hu20C2 | | RQLGLRSIDAMDY | 99 |
| 20C2B-39HC₃-1 | 5.78 x 10⁸ | XXXXXRSIDAMDY | 100 |
| 20C2B-39HC₃-2 | 6.16 x 10⁸ | RQLGLRSIXXXXX | 101 |
| 20C2B-39HC₃-3 | 3.99 x 10⁸ | RQLCXXXXXAMDY | 102 |

A total of 18 output plates from 4 rounds of panning were tested in a phage ELISA assay. A total of 1235 hits were found, of which 704 were sequenced. Based on BIACORE™ K_{D} values of Fab fragments against biotinylated-Aβ1-20 and biotinylated ADDL (bADDL) antigens, as well as single-point BIACORE™3000 analysis (Table 9), a total of 6 Fab clones were converted into IgG1 and IgG2m4 using either CDR grafting or veneering humanization techniques. One of these 6 Fabs, designated 4a-A3, was isolated from library 20C2B-39HC₃-3 and carried 3 amino acid substitutions (RQLGTRGTDAMDY; SEQ ID NO:3) in the middle section of the heavy chain. As is evident from Figure 2B, this heavy chain CDR3 sequence is that of Hu20C2A3.

**TABLE 9**

| Clone | bADDL Binding K_{D} (M) | Aβ 1-20 Binding K_{D} (M) | BIACORE™ 3000 Off-rate | Sequence | SEQ ID NO: |
|---|---|---|---|---|---|
| 4a-A3 | 8.23E-11 | 8.49E-10 | 6.65E-05 | RQLGTRGTDAMDY | 3 |
| 4b-A7 | 3.76E-10 | 1.72E-09 | 1.23E-04 | RQLGKLALDAMDY | 142 |
| 4b-H11 | 1.05E-09 | 1.33E-09 | 1.32E-04 | RQLGRRSVDAMDY | 8 |
| 20C28B | 1.10E-09 | 1.10E-09 | 8.76E-05 | RQLGLRSIDAMDY | 143 |
| 4a-F5 | 1.39E-09 | 1.33E-09 | 1.17E-04 | RQLGKLKTDAMDY | 7 |
| 4a-B2 | 1.92E-09 | 1.29E-09 | 2.13E-04 | RQLGARKTDAMDY | 5 |
| 4b-D8 | 2.23E-09 | 1.69E-09 | 1.45E-04 | RALSPRSIDAMDY | 4 |
| 4a-A4 | 2.67E-09 | 1.58E-09 | 1.20E-04 | RALSPRSIDAMDY | 4 |
| 4b-A1 | 2.87E-09 | 2.85E-09 | 1.23E-04 | RQLGPRKRDAMDY | 6 |
| 4a-A7 | 3.24E-09 | 2.21E-09 | 1.55E-04 | RQLGQRQTDAMDY | 144 |
| 4a-B9 | 3.44E-09 | 3.54E-09 | 1.94E-04 | RAIQPRSIDAMDY | 145 |
| 4a-B3 | 4.17E-09 | 3.64E-09 | 1.59E-04 | RQLGLRSIDAHTR | 146 |
| 4a-G10 | 4.52E-09 | 2.72E-09 | 1.78E-04 | RQLGQPSVDAMDY | 147 |
| 4a-E11 | 4.93E-09 | 3.48E-09 | 1.65E-04 | RQLGFQSTDAMDY | 148 |
| 4a-C9 | 8.43E-09 | 2.46E-09 | 1.75E-04 | RQLGQAGHDAMDY | 149 |
| 4a-D5 | 1.17E-09 | 3.91E-09 | 1.74E-04 | RQLGDNVADAMDY | 150 |
| 4a-E10 | 1.85E-08 | 3.60E-09 | 1.39E-04 | RQLGFQSTDAMDY | 148 |
| 4b-D4 | 1.86E-08 | 4.87E-09 | 1.89E-04 | RQLGMATPDAMDY | 151 |
| 4b-B10 | 6.28E-08 | 7.43E-09 | 1.81E-04 | RQLGAHWLDAMDY | 152 |
| 4b-A12 | 1.54E-07 | 1.01E-08 | 1.69E-04 | RQLGPEPQDAMDY | 153 |

### Example 5: Generation of IgG2m4 Antibodies

IgG2m4 antibody derivatives were prepared to decrease Fc receptor engagement, C1q binding, unwanted cytotoxicity or immunocomplex formation while maintaining both the long half-life and pharmacokinetic properties of a typical human antibody. The basic antibody format of IgG2m4 is that of IgG2, which has been shown to possess a superior half-life in experimental models (Zuckier, et al. (1994) Cancer Suppl. 73:794-799). The structure of IgG2 was modified to eliminate C1q binding, through selective incorporation of IgG4 sequences, while maintaining the typical low level of FcyR binding (Canfield and Morrison (1991) J. Exp. Med. 173:1483-1491). This was achieved by using cross-over points wherein sequences of IgG2 and IgG4 were identical, thereby producing an antibody containing natural Fc sequences rather than any artificial mutational sequences. The advantages of using the instant IgG2m4 antibody which exhibits minimal effector-related activity is comparable to the deglycosylated antibody disclosed by Wilcock et al. ((2006) J. Neurosci. 26:5340-6).

The IgG2m4 form of the human antibody constant region was formed by selective incorporation of human IgG4 sequences into a standard human IgG2 constant region, as shown in Figure 6. Conceptually, IgG2m4 resulted from a pair of chain-swaps within the CH2 domain as shown in Figure 6. Four single mutations were made corresponding to sequences from IgG4. The Fc residues mutated in IgG2 included His268Gln, Val309Leu, Ala330Ser, and Pro331Ser, which minimized the potential for neoepitopes. The specific IgG4 amino acid residues placed into the IgG2 constant region are shown in Table 10, along with other alternative from the basic structure.

**TABLE 10**

| **Residue (Kabat numbering)** | **Residue in IgG2** | **Residue in IgG4** | **Residue in IgG2m4** | **Alternative residue in IgG2m4** | **Comment** |
|---|---|---|---|---|---|
| 189 | Pro or Thr* | Pro | Thr | Pro | Key polymorphism of IgG2; Pro residue present in *IGHG*01* allotype and Thr residue present in *IGHG2*02* allotype^{a,b}. |
| 268 | His | Gln | Gln | -- | Change in the B/C loop known to be involved in FcγRII binding^{c}. |
| 309 | Val | Leu or Val | Leu | Val | FcRn binding domain |
| 330 | Ala | Ser | Ser | -- | Key residue for C1q binding^{d}; also potentially involved in binding FcγRII and FcγRIII^{e}. |
| 331 | Pro | Ser | Ser | -- | Key residue for C1q binding^{d,f} and FcγRI binding^{g}; also potentially involved in binding FcγRII and FcγRIII^{e}. |
| 397 | Met or Val* | Val | Met | Val | Val residue present in *IGHG*01* allotype and Met residue present in *IGHG2*02* allotype^{a}. |

| | | | | | |
|---|---|---|---|---|---|
| *Positions marked with an asterisk are subject to allelic variations. ^{a}Hougs, et al. (2001) Immunogenetics 52 (3-4) :242-8. ^{b}WO 97/11971. ^{c}Medgyesi, et al. (2004) Eur. J. Immunol. 34:1127-1135. ^{d}Tao, et al. (1991) J. Exp. Med. 173:1025-1028. ^{e}Armour, et al. (1999) Eur. J. Immunol. 29:2613. ^{f}Xu, et al. (1994) J. Biol. Chem. 269:3469-3474. ^{g}Canfield and Morrison (1991) J. Exp. Med. 173:1483. | | | | | |

Human IgG1/kappa and IgG2m4/kappa versions of humanized Hu20C2 and Hu20C2A3 antibodies were constructed. The complete amino acid sequence of the light and heavy chain Hu20C2A3 IgG2m4 antibody is shown in Figures 7A and 7C.

### Example 6: Binding Affinity and Specificity of Humanized Anti-ADDL Antibodies

Affinity maturation was carried out to improve affinity and improve preferential binding to ADDL. To evaluate ADDL binding affinity of the humanized antibodies, BIACORE™ and titration ELISAs were conducted as disclosed herein. Briefly, Streptavidin-coated, 96-well microtiter plates (Sigma, St. Louis, MO) were coated with 10% biotinylated ADDL antigen (1 *µ*M). A series of 2-fold dilutions of purified antibody, starting at 500 ng/mL was added to the ADDL captured plates and the plates were incubated for 2 hours at 25°C. After washing five times with PBS solution using a plate washer (Bio-Tek, Winooski, VA), polyclonal goat anti-human kappa light chain antibody (Biomeda, Foster City, CA) was added at a 1/2000 dilution in 3% non-fat milk blocker and incubated at room temperature for 1 hour. A rabbit anti-goat IgG (H+L) HRP-conjugated (Bethyl Laboratories, Inc., Montgomery, TX) detection antibody was then added at a 1/2000 dilution in blocking solution and incubated for 1 hour at room temperature. After washing with PBS, HRP substrate, 3,3',5'5-tetramethylbenzidine (ready-to-use TMB; Sigma, St. Louis, MO) was added and the reaction was stopped after 10 minutes with 0.5 N H₂SO₄. Absorbance at wavelength of 450 nm was read in a plate reader (model VICTOR V; Perkin Elmer, Boston, MA) and data were processed using EXCEL^{®} work sheet. Assay variations between plates were estimated within 20%.

The K_{D} of Fab clone A3, as measured by BIACORE™, was 849 pM against biotinylated-Aβ1-20. The K_{D} of the same Fab clone was 82 pM against bADDLs, indicating that the A3 Fab demonstrated preferential binding to ADDLs. When the clone was humanized by veneering and converted to a full IgG1 molecule or full IgG2m4 molecule (i.e., Hu20C2A3), the K_{D} values against Aβ1-20 and ADDL were below the reliable detection limit of the BIACORE™ instrument, indicating a significant improvement of Hu20C2A3's binding equilibrium constant against Aβ1-20 and ADDL as compared to Hu20C2.

Hu20C2A3, which is the veneered version of clone A3 with an IgG2m4 isotype, was expressed in both CHO and *Pichia.* The two sources of Hu20C2A3 were evaluated for their ability to interact with Aβ monomer and ADDLs by ELISA. As shown in Figure 8, Hu20C2A3 produced in either CHO (Figure 8A) or Pichia (Figure 8B) showed preferential ADDL binding versus Aβ40 monomer binding (6-fold). Binding constants (IgGk₅₀ values) as determined from these curves yielded values of 64 pM and 376 pM for ADDLs and Aβ monomer, respectively for Hu20C2A3 produced in *Pichia* and 58 pM and 361 pM for ADDLs and Aβ monomer, respectively for Hu20C2A3 produced in CHO cells.

### Example 7: Inhibition of ADDL Binding to Neurons Using Humanized Anti-ADDL Antibodies

The humanized anti-ADDL antibodies were further evaluated for their ability to block ADDL binding to primary hippocampal neurons using the methods disclosed herein. Hu20C2A3 antibody, or PBS as a control, was mixed at various molar ratios with bADDLs and incubated for one hour at 37°C on a slow rotator. After the preincubation, the antibody/bADDL preparations were added to primary neuron cultures and incubated for an additional hour at 37°C. At the end of the incubation period, the bADDLs/antibody mixture was removed and the plates washed six times with media. The cells were then fixed in 4% paraformaldehyde for ten minutes at room temperature, the solution removed, fresh fixative added, and the cells fixed for an additional ten minutes. The cells were permeabilized with 4% paraformaldehyde containing 0.1% TRITON™ X-100 (2 times, each for ten minutes at room temperature), washed six times in PBS and then treated with 10% BSA in PBS for one hour at 37°C. Alkaline phosphatase-conjugated streptavidin (1:1,500 in 1% BSA; Molecular Probes, Eugene, OR) was then added to the cells for one hour at room temperature. The cells were rinsed six times with PBS, the alkaline phosphatase substrate (CDP-STAR^{®} with SAPPHIRE-II™; Applied Biosystems, Foster City, CA) added to the cells and incubated for thirty minutes prior to determining the luminescence on a LJL Luminometer (Analyst AD; LJL Biosystems, Sunnyvale, CA). In this analysis, Hu20C2A3 was found to effectively inhibit bADDL binding to neurons at a sub-stoichiometric antibody to peptide ratio (EC₅₀ = 0.16; Figure 9).

The inhibition of bADDL binding indicated that Hu20C2A3 interacts with ADDLs in a biologically relevant manner. To demonstrate that Hu20C2A3 interacts with ADDLs relevant to the human Alzheimer's Disease condition, the ability of biotinylated Hu20C2A3 to immuno-label Aβ containing plaques in human Alzheimer's Disease brain tissue was evaluated. Immunohistochemical localization showed avid labeling of Aβ in both dense core and diffuse plaques in human Alzheimer's Disease brain tissue. The specificity of this binding was demonstrated by a loss of immunoreactivity following pre-incubation with increasing ADDL:antibody amounts. Similar to Hu20C2, Hu20C2A3, efficiently labels both dense core and diffuse Aβ deposits.

### Example 8: Thermal Stability of Hu20C2A3

An evaluation of the protein stability of Hu20C2A3 was assessed using SEC HPLC, fluorescent thermal melt analysis, and particle size analysis. Fluorescent thermal melt analysis indicated that Fc and Fab unfolding transitions occurred at approximately 70°C and 80°C, respectively, consistent with acceptable inherent protein stability (Figure 10).

### Example 9: In Vivo Pharmacodynamic and Efficacy Analysis

The prior art indicates that systemic injection of monoclonal anti-Aβ antibodies can increase plasma levels of Aβ acutely, whereas measurable lowering of brain Aβ requires chronic administration. It has been suggested that passive immunization in species with measurable Aβ results in an elevation of plasma Aβ due to a change in the equilibrium of Aβ between brain and peripheral compartments. This "peripheral sink" ultimately leads to lowering of brain Aβ. However, cognitive improvement has been observed in animals following acute antibody administration prior to notable changes in brain Aβ, indicating that changes in brain Aβ may occur in some form prior to a time point where these changes can be measured using known techniques. Alternately, elevations of plasma Aβ could be explained by a stabilization of peripheral Aβ following administration of antibody. Regardless of the interpretation, it has been established that early plasma elevations are a prerequisite for subsequent lowering of brain Aβ in animal models. Thus, the effect of Hu20C2A3 antibodies on plasma Aβ elevations were utilized as an indicator of target engagement. Following infusion of Hu20C2A3 at doses of 30, 100 and 300 µg/mouse IV, significant and robust increases in plasma Aβx-40 were observed relative to the non-relevant antibody (8B4) control group 4 hours post-injection (Figure 11). The observed increases in plasma Aβx-40-for CHO-derived material were 491% (30 µg, p>0.001), 826% (100 µg, p<0.001), and 755% (300 µg, p<0.001) of the 8B4 levels. Similarly, the increases in plasma Aβx-40 for Pichia-derived material were 395% (30 µg, p>0.001), 729% (100 µg, p<0.001), and 838% (300 µg, p<0.001) of the 8B4 levels.

## Claims

1. An isolated antibody capable of differentially recognizing a multi-dimensional conformation of one or more Aβ-derived diffusible ligands,
wherein the light chain of said antibody comprises Arg-Ser-Ser-Gln-Ser-Ile-Leu-His-Ser-Asn-Gly-Asn-Thr-Tyr-Leu-Glu;
Lys-Val-Ser-Asn-Arg-Phe-Ser; and Xaa₁-Gln-Xaa₂-Thr-Arg-Val-Pro-Leu-Thr (SEQ ID NO:2), wherein Xaa₁ is Phe or Leu, and Xaa₂ is Ala or Thr; and
wherein the heavy chain of said antibody comprises Thr-Ser-Gly-Met-Gly-Val-Gly; His-Ile-Trp-Trp-Asp-Asp-Asp-Lys-Ser-Tyr-Asn-Pro-Ser-Leu-Lys-Ser; and
Arg-Gln-Leu-Gly-Thr-Arg-Gly-Thr-Asp-Ala-Met-Asp-Tyr.

2. The isolated antibody of claim 1, wherein the heavy and light chain variable regions of said antibody respectively comprise the amino acid sequence set forth in SEQ ID NO:108 and SEQ ID NO:112.

3. The isolated antibody of claim 1, wherein the heavy and light chain of said antibody respectively comprise the amino acid sequence set forth in SEQ ID NO:138 and SEQ ID NO:140.

4. The isolated antibody of claim 1, wherein the light chain of said antibody comprises
Arg-Ser-Ser-Gln-Ser-Ile-Leu-His-Ser-Asn-Gly-Asn-Thr-Tyr-Leu-Glu;
Lys-Val-Ser-Asn-Arg-Phe-Ser; and Leu-Gln-Thr-Thr-Arg-Val-Pro-Leu-Thr; and
wherein the heavy chain of said antibody comprises Thr-Ser-Gly-Met-Gly-Val-Gly; His-Ile-Trp-Trp-Asp-Asp-Asp-Lys-Ser-Tyr-Asn-Pro-Ser-Leu-Lys-Ser; and
Arg-Gln-Leu-Gly-Thr-Arg-Gly-Thr-Asp-Ala-Met-Asp-Tyr.

5. A pharmaceutical composition comprising the isolated antibody of any one of claims 1 to 4 in admixture with a pharmaceutically acceptable carrier.

6. An *ex vivo* method for preventing binding of Aβ-derived diffusible ligands to a neuron comprising contacting the neuron with the antibody of any one of claims 1 to 4 so that binding of Aβ-derived diffusible ligands to the neuron is prevented.

7. An ex *vivo* method for inhibiting assembly of Aβ-derived diffusible ligands comprising contacting a sample containing amyloid β 1-42 peptides with the antibody of any one of claims 1 to 4 thereby inhibiting assembly of Aβ-derived diffusible ligands.

8. The antibody of any one of claims 1 to 4 for use in treating amyloidogenic disease.

9. An ex *vivo* method for blocking the phosphorylation of tau protein at Ser202/Thr205 comprising contacting a sample containing a tau protein with the antibody of any one of claims 1 to 4 thereby blocking the phosphorylation of tau protein at Ser202/Thr205.

10. The antibody of any one of claims 1 to 4 for use in treating tauopathy.

11. The isolated antibody of any one of claims 1 to 4 for use in prophylactically or therapeutically treating any disease wherein the accumulation of ADDLs results in physiological impairment of long-term potentiation/memory formation, the use comprising administering an effective amount of the antibody.

12. An ex *vivo* method for identifying a therapeutic agent that prevents the binding of Aβ-derived diffusible ligands to a neuron comprising contacting a neuron with Aβ-derived diffusible ligands in the presence of an agent and using the antibody of any one of claims 1 to 4 to determine binding of Aβ-derived diffusible ligands to the neuron in the presence of the agent.

13. An ex *vivo* method for detecting Aβ-derived diffusible ligands in a sample comprising contacting a sample with the antibody of any one of claims 1 to 4 so that Aβ-derived diffusible ligands are detected.

14. An *ex vivo* method for diagnosing a disease associated with Aβ-derived diffusible ligands comprising contacting a sample with the antibody of any one of claims 1 to 4 so that a disease associated with Aβ-derived diffusible ligands is diagnosed.

15. A kit for detecting Aβ-derived diffusible ligands comprising the isolated antibody of any one of claims 1 to 4.

16. The isolated antibody for use of claim 11, wherein the disease wherein the accumulation of ADDLs results in physiological impairment of long-term potentiation/memory formation comprises Alzheimer's disease.

## Patentansprüche

1. Isolierter Antikörper, der eine mehrdimensionale Konformation von einem oder mehreren Aβ-Derived Diffusible Ligands differentiell erkennen kann,
worin die leichte Kette des genannten Antikörpers Folgendes umfasst:
Arg-Ser-Ser-Gln-Ser-Ile-Leu-His-Ser-Asn-Gly-Asn-Thr-Tyr-Leu-Glu;
Lys-Val-Ser-Asn-Arg-Phe-Ser; und
Xaa₁-Gln-Xaa₂-Thr-Arg-Val-Pro-Leu-Thr (Seq.-ID Nr. 2),
worin Xaa₁ Phe oder Leu ist und Xaa₂ Ala oder Thr ist; und
worin die schwere Kette des genannten Antikörpers Folgendes umfasst:
Thr-Ser-Gly-Met-Gly-Val-Gly;
His-Ile-Trp-Trp-Asp-Asp-Asp-Lys-Ser-Tyr-Asn-Pro-Ser-Leu-Lys-Ser; und
Arg-Gln-Leu-Gly-Thr-Arg-Gly-Thr-Asp-Ala-Met-Asp-Tyr.

2. Isolierter Antikörper nach Anspruch 1, worin die variablen Regionen der schweren und leichten Kette des genannten Antikörpers entsprechend die Aminosäuresequenz umfassen, die in SEQ.-ID Nr. 108 und SEQ.-ID Nr. 112 dargelegt ist.

3. Isolierter Antikörper nach Anspruch 1, worin die schwere und leichte Kette des genannten Antikörpers entsprechend die Aminosäuresequenz umfassen, die in SEQ.-ID Nr. 138 und SEQ.-ID Nr. 140 dargelegt ist.

4. Isolierter Antikörper nach Anspruch 1, worin die leichte Kette des genannten Antikörpers Folgendes umfasst:
Arg-Ser-Ser-Gln-Ser-Ile-Leu-His-Ser-Asn-Gly-Asn-Thr-Tyr-Leu-Glu;
Lys-Val-Ser-Asn-Arg-Phe-Ser; und
Leu-Gln-Thr-Thr-Arg-Val-Pro-Leu-Thr; und
worin die schwere Kette des genannten Antikörpers Folgendes umfasst:
Thr-Ser-Gly-Met-Gly-Val-Gly;
His-Ile-Trp-Trp-Asp-Asp-Asp-Lys-Ser-Tyr-Asn-Pro-Ser-Leu-Lys-Ser; und
Arg-Gln-Leu-Gly-Thr-Arg-Gly-Thr-Asp-Ala-Met-Asp-Tyr.

5. Pharmazeutische Zusammensetzung, die den isolierten Antikörper nach einem der Ansprüche 1 bis 4 in Beimischung mit einem pharmazeutisch verträglichen Träger umfasst.

6. Ex-vivo-Verfahren zur Prävention der Bindung von Aβ-Derived Diffusible Ligands zu einem Neuron, das das Inkontaktbringen des Neurons mit dem Antikörper nach einem der Ansprüche 1 bis 4 umfasst, sodass die Bindung von Aβ-Derived Diffusible Ligands zu dem Neuron verhindert wird.

7. Ex-vivo-Verfahren zur Hemmung der Assemblierung von Aβ-Derived Diffusible Ligands, das das Inkontaktbringen einer Probe, die Amyloid-β (1-42)-Peptide enthält, mit dem Antikörper nach einem der Ansprüche 1 bis 4 umfasst, wodurch die Assemblierung von Aβ-Derived Diffusible Ligands gehemmt wird.

8. Antikörper nach einem der Ansprüche 1 bis 4 zur Verwendung bei der Behandlung von amyloidogener Erkrankung.

9. Ex-vivo-Verfahren zur Blockierung der Phosphorylierung von Tau-Protein am Ser202/Thr205, das das Inkontaktbringen einer Probe, die ein Tau-Protein enthält, mit dem Antikörper nach einem der Ansprüche 1 bis 4 umfasst, wodurch die Phosphorylierung des Tau-Proteins am Ser202/Thr205 blockiert wird.

10. Antikörper nach einem der Ansprüche 1 bis 4 zur Verwendung bei der Behandlung von Tauopathie.

11. Isolierter Antikörper nach einem der Ansprüche 1 bis 4 zur Verwendung bei der prophylaktischen oder therapeutischen Behandlung einer jedweden Erkrankung, worin die Ansammlung von ADDLs zur physiologischen Beeinträchtigung von Langzeitpotenzierung/Gedächtnisbildung führt, wobei die Verwendung das Verabreichen einer wirksamen Menge des Antikörpers umfasst.

12. *Ex-vivo*-Verfahren zur Identifizierung eines therapeutischen Mittels, das die Bindung von Aβ-Derived Diffusible Ligands zu einem Neuron verhindert, das das Inkontaktbringen eines Neurons mit Aβ-Derived Diffusible Ligands in Gegenwart eines Mittels und unter Verwendung des Antikörpers nach einem der Ansprüche 1 bis 4 umfasst, um die Bindung von Aβ-Derived Diffusible Ligands zu dem Neuron in Gegenwart des Mittels zu bestimmen.

13. *Ex-vivo*-Verfahren zum Nachweis von Aβ-Derived Diffusible Ligands in einer Probe, das das Inkontaktbringen einer Probe mit dem Antikörper nach einem der Ansprüche 1 bis 4 umfasst, sodass Aβ-Derived Diffusible Ligands nachgewiesen werden.

14. Ex-vivo-Verfahren zur Diagnose einer Erkrankung, die mit Aβ-Derived Diffusible Ligands im Zusammenhang steht, das das Inkontaktbringen einer Probe mit dem Antikörper nach einem der Ansprüche 1 bis 4 umfasst, sodass eine Erkrankung, die mit Aβ-Derived Diffusible Ligands im Zusammenhang steht, diagnostiziert wird.

15. Kit zum Nachweis von Aβ-Derived Diffusible Ligands, der den isolierten Antikörper nach einem der Ansprüche 1 bis 4 umfasst.

16. Isolierter Antikörper zur Verwendung nach Anspruch 11, worin die Erkrankung, worin die Ansammlung von ADDLs zur physiologischen Beeinträchtigung von Langzeitpotenzierung/Gedächtnisbildung führt, die Alzheimer-Krankheit umfasst.

## Revendications

1. Anticorps isolé capable de reconnaître de manière différentielle une conformation multidimensionnelle d'un ou plusieurs ligands diffusibles dérivés d'Aβ,
où la chaîne légère dudit anticorps comprend
Arg-Ser-Ser-Gln-Ser-Ile-Leu-His-Ser-Asn-Gly-Asn-Thr-Tyr-Leu-Glu ;
Lys-Val-Ser-Asn-Arg-Phe-Ser ; et
Xaa₁-Gln-Xaa₂-Thr-Arg-Val-Pro-Leu-Thr (SEQ ID n°2),
où Xaa₁ est Phe ou Leu et Xaa₂ est Ala ou Thr ; et
où la chaîne lourde dudit anticorps comprend
Thr-Ser-Gly-Met-Gly-Val-Gly ;
His-Ile-Trp-Trp-Asp-Asp-Asp-Lys-Ser-Tyr-Asn-Pro-Ser-Leu-Lys-Ser ; et
Arg-Gln-Leu-Gly-Thr-Arg-Gly-Thr-Asp-Ala-Met-Asp-Tyr.

2. Anticorps isolé selon la revendication 1, où les régions variables des chaînes lourde et légère dudit anticorps comprennent respectivement la séquence d'acides aminés exposée dans SEQ ID n°108 et SEQ ID n°112.

3. Anticorps isolé selon la revendication 1, où les chaînes lourde et légère dudit anticorps comprennent respectivement la séquence d'acides aminés exposée dans SEQ ID n°138 et SEQ ID n°140.

4. Anticorps isolé selon la revendication 1, où la chaîne légère dudit anticorps comprend
Arg-Ser-Ser-Gln-Ser-Ile-Leu-His-Ser-Asn-Gly-Asn-Thr-Tyr-Leu-Glu ;
Lys-Val-Ser-Asn-Arg-Phe-Ser ; et
Leu-Gln-Thr-Thr-Arg-Val-Pro-Leu-Thr ; et
où la chaîne lourde dudit anticorps comprend
Thr-Ser-Gly-Met-Gly-Val-Gly ;
His-Ile-Trp-Trp-Asp-Asp-Asp-Lys-Ser-Tyr-Asn-Pro-Ser-Leu-Lys-Ser ; et
Arg-Gln-Leu-Gly-Thr-Arg-Gly-Thr-Asp-Ala-Met-Asp-Tyr.

5. Composition pharmaceutique comprenant l'anticorps isolé selon l'une quelconque des revendications 1 à 4, en mélange avec un véhicule pharmaceutiquement acceptable.

6. Méthode *ex vivo* de prévention de la fixation de ligands diffusibles dérivés d'Aβ à un neurone, comprenant la mise en contact du neurone avec l'anticorps selon l'une quelconque des revendications 1 à 4, de sorte que la fixation de ligands diffusibles dérivés d'Aβ au neurone soit empêchée.

7. Méthode *ex vivo* d'inhibition de l'assemblage de ligands diffusibles dérivés d'Aβ, comprenant la mise en contact d'un échantillon contenant les peptides 1-42 de l'amyloïde β avec l'anticorps selon l'une quelconque des revendications 1 à 4, afin d'inhiber ainsi l'assemblage de ligands diffusibles dérivés d'Aβ.

8. Anticorps selon l'une quelconque des revendications 1 à 4, pour une utilisation dans le traitement d'une maladie amyloïdogène.

9. Méthode *ex vivo* de blocage de la phosphorylation de la protéine tau au niveau de Ser202/Thr205, comprenant la mise en contact d'un échantillon contenant une protéine tau avec l'anticorps selon l'une quelconque des revendications 1 à 4, afin de bloquer ainsi la phosphorylation de la protéine tau au niveau de Ser202/Thr205.

10. Anticorps selon l'une quelconque des revendications 1 à 4, pour une utilisation dans le traitement de la tauopathie.

11. Anticorps isolé selon l'une quelconque des revendications 1 à 4, pour une utilisation dans le traitement prophylactique ou thérapeutique d'une maladie quelconque dans laquelle l'accumulation d'ADDL entraîne une déficience physiologique de la potentialisation à long terme/formation de la mémoire, l'utilisation comprenant l'administration d'une quantité efficace de l'anticorps.

12. Méthode *ex vivo* d'identification d'un agent thérapeutique qui empêche la fixation de ligands diffusibles dérivés d'Aβ à un neurone, comprenant la mise en contact du neurone avec des ligands diffusibles dérivés d'Aβ en présence d'un agent et l'utilisation de l'anticorps selon l'une quelconque des revendications 1 à 4, afin de déterminer la fixation de ligands diffusibles dérivés d'Aβ au neurone en présence de l'agent.

13. Méthode *ex vivo* de détection de ligands diffusibles dérivés d'Aβ dans un échantillon, comprenant la mise en contact de l'échantillon avec l'anticorps selon l'une quelconque des revendications 1 à 4, afin de détecter les ligands diffusibles dérivés d'Aβ.

14. Méthode *ex vivo* de diagnostic d'une maladie associée aux ligands diffusibles dérivés d'Aβ, comprenant la mise en contact d'un l'échantillon avec l'anticorps selon l'une quelconque des revendications 1 à 4, afin de diagnostiquer une maladie associée aux ligands diffusibles dérivés d'Aβ.

15. Kit de détection de ligands diffusibles dérivés d'Aβ, comprenant l'anticorps isolé selon l'une quelconque des revendications 1 à 4.

16. Anticorps isolé pour une utilisation selon la revendication 11, où la maladie dans laquelle l'accumulation d'ADDL entraîne une déficience physiologique de la potentialisation à long terme/formation de la mémoire comprend la maladie d'Alzheimer.
